# EUROPEAN PATENT APPLICATION

(11) **EP 3 604 528 A1**
(43) Date of publication of application: **05.02.2020**
(21) Application number: 18776038.4
(22) Date of filing: 30.03.2018
(51) Int. Cl.: C12N 15/11, A61K 31/711, A61K 31/7115, A61K 31/713, A61K 47/54, A61K 47/65, A61K 48/00, A61P 43/00

(54) **CYCLIC NUCLEIC ACID MOLECULE HAVING GENE EXPRESSION CONTROL FUNCTION**

(30) Priority: 31.03.2017 JP 2017070695
(71) Applicant: Bonac Corporation, Kurume-shi, Fukuoka 839-0861 (JP)
(72) Inventor: OHGI, Tadaaki, Kurume-shi Fukuoka 839-0861 (JP); EMURA, Chisato, Kurume-shi Fukuoka 839-0861 (JP); YASUOKA, Junichi, Kurume-shi Fukuoka 839-0861 (JP); EGUCHI, Yutaka, Kurume-shi Fukuoka 839-0861 (JP)
(74) Representative: J A Kemp LLP
(86) International application number: PCT/JP2018/013960
(87) International publication number: WO 2018/182008

(57) **Abstract**

The present invention aims to provide a nucleic acid molecule having improved stability in serum and capable of exhibiting a gene expression regulation function after transfer into the cell. A cyclic nucleic acid molecule represented by the following formula (A): 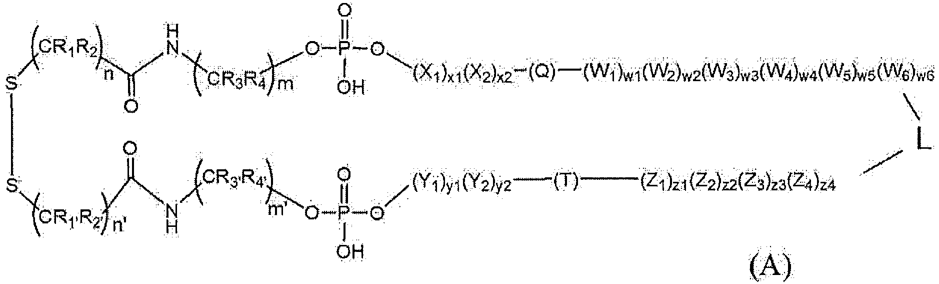 wherein each symbol is as described in the DESCRIPTION can be synthesized easily at a low cost, and can inhibit the translation of a protein encoded by the gene. Since the cyclic nucleic acid molecule of the present invention can inhibit the expression of a target gene as described above, for example, it is useful as a pharmaceutical product, a diagnostic agent, an agricultural chemical, and a tool for conducting research on agriculture, medical science, life science, and the like.

SEQUENCE LISTING

<110>  BONAC CORPORATION

<120>  Cyclic nucleic acid molecule for controlling gene expression

<130>  092715

<150>  JP2017-070695
<151>  2017-03-31

<160>  52

<170>  PatentIn version 3.5

<210>  1
<211>  19
<212>  RNA
<213>  Artificial Sequence

<220>
<223>  expression inhibitory sequence

<400>  1
uaugcugugu guacucugc                                                    19


<210>  2
<211>  17
<212>  RNA
<213>  Artificial Sequence

<220>
<223>  complementary sequence to expression inhibitory sequence

<400>  2
gcagaguaca cacagca                                                      17


<210>  3
<211>  21
<212>  RNA
<213>  Artificial Sequence

<220>
<223>  expression inhibitory sequence

<400>  3
uagcaccauu ugaaaucagu g                                                 21


<210>  4
<211>  23
<212>  RNA
<213>  Artificial Sequence

<220>
<223>  complementary sequence to expression inhibitory sequence

<400>  4
cacugauuuc aaauggugcu aga                                               23

<210>  5
<211>  48
<212>  RNA
<213>  Artificial Sequence

<220>
<223>  single-staranded nucleic acid molecule_PA-0001

<400>  5
gcagaguaca cacagcauau accgguauau gcugugugua cucugcuu                48


<210>  6
<211>  50
<212>  RNA
<213>  Artificial Sequence

<220>
<223>  single-staranded nucleic acid molecule_PA-0002

<400>  6
aagcagagua cacacagcau auaccgguau augcugugug uacucugcuu              50


<210>  7
<211>  50
<212>  DNA
<213>  Artificial Sequence

<220>
<223>  single-staranded nucleic acid molecule_PA-0003

<400>  7
aagcagagua cacacagcat ataccggtau augcugugug uacucugcuu              50


<210>  8
<211>  50
<212>  DNA
<213>  Artificial Sequence

<220>
<223>  single-staranded nucleic acid molecule_PA-0004

<400>  8
aagcagagua cacacagcat ataccggtau augcugugug uacucugcuu              50


<210>  9
<211>  50
<212>  DNA
<213>  Artificial Sequence

<220>
<223>  single-staranded nucleic acid molecule_PA-0005

<400>  9
aagcagagua cacacagcat ataccgguau augcugugug uacucugcuu              50


<210>  10
<211>  50
<212>  DNA

<213>  Artificial Sequence

<220>
<223>  single-staranded nucleic acid molecule_PA-0006

<400>  10
aagcagagua cacacagcau ataccgguau augcugugug uacucugcuu                50


<210>  11
<211>  50
<212>  DNA
<213>  Artificial Sequence

<220>
<223>  single-staranded nucleic acid molecule_PA-0007

<400>  11
aagcagagua cacacagcau ataccggtat augcugugug uacucugcuu                50


<210>  12
<211>  50
<212>  RNA
<213>  Artificial Sequence

<220>
<223>  single-staranded nucleic acid molecule_PA-0008


<220>
<221>  modified_base
<222>  (20)..(20)
<223>  um

<220>
<221>  modified_base
<222>  (21)..(21)
<223>  2'-O-methyladenosine

<220>
<221>  modified_base
<222>  (22)..(22)
<223>  um

<220>
<221>  modified_base
<222>  (23)..(23)
<223>  2'-O--methyladenosine

<220>
<221>  modified_base
<222>  (24)..(24)
<223>  cm

<220>
<221>  modified_base
<222>  (25)..(25)
<223>  cm

<220>
<221>  modified_base
<222>  (26)..(26)

<223>  gm

<220>
<221>  modified_base
<222>  (27)..(27)
<223>  gm

<220>
<221>  modified_base
<222>  (28)..(28)
<223>  um

<400>  12
aagcagagua cacacagcau auaccgguau augcugugug uacucugcuu          50


<210>  13
<211>  50
<212>  RNA
<213>  Artificial Sequence

<220>
<223>  single-staranded nucleic acid molecule_PA-0009


<220>
<221>  modified_base
<222>  (20)..(20)
<223>  um

<220>
<221>  modified_base
<222>  (21)..(21)
<223>  2'-O--methyladenosine

<220>
<221>  modified_base
<222>  (22)..(22)
<223>  um

<220>
<221>  modified_base
<222>  (23)..(23)
<223>  2'-O--methyladenosine

<220>
<221>  modified_base
<222>  (24)..(24)
<223>  cm

<220>
<221>  modified_base
<222>  (25)..(25)
<223>  cm

<400>  13
aagcagagua cacacagcau auaccgguau augcugugug uacucugcuu          50


<210>  14
<211>  50
<212>  RNA
<213>  Artificial Sequence

<220>
<223>  single-staranded nucleic acid molecule_PA-0010

<220>
<221>  modified_base
<222>  (26)..(26)
<223>  gm

<220>
<221>  modified_base
<222>  (27)..(27)
<223>  gm

<220>
<221>  modified_base
<222>  (28)..(28)
<223>  um

<400>  14
aagcagagua cacacagcau auaccgguau augcugugug uacucugcuu          50

<210>  15
<211>  50
<212>  RNA
<213>  Artificial Sequence

<220>
<223>  single-staranded nucleic acid molecule_PA-0011

<400>  15
aaccaugaga aguaugacaa cagccggcug uugucauacu ucucaugguu          50

<210>  16
<211>  50
<212>  DNA
<213>  Artificial Sequence

<220>
<223>  single-staranded nucleic acid molecule_PA-0012

<400>  16
aaccaugaga aguaugacaa cagccggcug uugucauacu ucucaugguu          50

<210>  17
<211>  50
<212>  RNA
<213>  Artificial Sequence

<220>
<223>  single-staranded nucleic acid molecule_PA-0013

<220>
<221>  modified_base
<222>  (20)..(20)
<223>  um

<220>

<221>  modified_base
<222>  (21)..(21)
<223>  2'-O--methyladenosine

<220>
<221>  modified_base
<222>  (22)..(22)
<223>  um

<220>
<221>  modified_base
<222>  (23)..(23)
<223>  2'-O--methyladenosine

<220>
<221>  modified_base
<222>  (24)..(24)
<223>  cm

<220>
<221>  modified_base
<222>  (25)..(25)
<223>  cm

<400>  17
aagcagagua cacacagcau auaccgguau augcugugug uacucugcuu                50


<210>  18
<211>  50
<212>  RNA
<213>  Artificial Sequence

<220>
<223>  single-staranded nucleic acid molecule_PA-0014


<220>
<221>  modified_base
<222>  (20)..(20)
<223>  um

<220>
<221>  modified_base
<222>  (21)..(21)
<223>  2'-O--methyladenosine

<220>
<221>  modified_base
<222>  (22)..(22)
<223>  um

<220>
<221>  modified_base
<222>  (23)..(23)
<223>  2'-O--methyladenosine

<220>
<221>  modified_base
<222>  (24)..(24)
<223>  cm

<220>

<221>  modified_base
<222>  (25)..(25)
<223>  cm

<220>
<221>  modified_base
<222>  (26)..(26)
<223>  gm

<220>
<221>  modified_base
<222>  (27)..(27)
<223>  gm

<220>
<221>  modified_base
<222>  (28)..(28)
<223>  um

<220>
<221>  modified_base
<222>  (49)..(49)
<223>  um

<400>  18
aagcagagua cacacagcau auaccgguau augcugugug uacucugcuu                50


<210>  19
<211>  54
<212>  RNA
<213>  Artificial Sequence

<220>
<223>  single-staranded nucleic acid molecule_PA-0015

<400>  19
aauagcacca uuugaaauca guguuccgga acacugauuu caaauggugc uauu          54


<210>  20
<211>  54
<212>  DNA
<213>  Artificial Sequence

<220>
<223>  single-staranded nucleic acid molecule_PA-0016

<400>  20
aauagcacca uuugaaauca gtgttccgga acacugauuu caaauggugc uauu          54


<210>  21
<211>  50
<212>  DNA
<213>  Artificial Sequence

<220>
<223>  single-staranded nucleic acid molecule_PA-0017

<400>  21
aagcagagua cacacagcau auaccggtau augcugugug uacucugcuu                50

<210>  22
<211>  50
<212>  DNA
<213>  Artificial Sequence

<220>
<223>  single-staranded nucleic acid molecule_PA-0018

<400>  22
aagcagagua cacacagcat ataccggtau augcugugug uacucugctt                50


<210>  23
<211>  50
<212>  DNA
<213>  Artificial Sequence

<220>
<223>  single-staranded nucleic acid molecule_PA-0019

<400>  23
ggtauaugcu guguguacuc ugcuuaagca gaguacacac agcatatacc                50


<210>  24
<211>  50
<212>  DNA
<213>  Artificial Sequence

<220>
<223>  single-staranded nucleic acid molecule_PA-0020

<400>  24
aagcagagua cacacagcat ataccggtau augcugugug uacucugcuu                50


<210>  25
<211>  50
<212>  DNA
<213>  Artificial Sequence

<220>
<223>  single-staranded nucleic acid molecule_PA-0021

<400>  25
aagcagagua cacacagcat ataccggtau augcugugug uacucugcuu                50


<210>  26
<211>  50
<212>  RNA
<213>  Artificial Sequence

<220>
<223>  single-staranded nucleic acid molecule_PA-0024

<400>  26
aagcagagua cacacagcau auaccgguau augcugugug uacucugcuu                50


<210>  27
<211>  50

<212>  DNA
<213>  Artificial Sequence

<220>
<223>  single-staranded nucleic acid molecule_PA-0025

<400>  27
aagcagagua cacacagcat ataccggtau augcugugug uacucugctt          50


<210>  28
<211>  50
<212>  RNA
<213>  Artificial Sequence

<220>
<223>  single-staranded nucleic acid molecule_PA-0026


<220>
<221>  modified_base
<222>  (1)..(1)
<223>  2'-O--methyladenosine

<220>
<221>  modified_base
<222>  (2)..(2)
<223>  2'-O--methyladenosine

<400>  28
aagcagagua cacacagcau auaccgguau augcugugug uacucugcuu          50


<210>  29
<211>  50
<212>  DNA
<213>  Artificial Sequence

<220>
<223>  single-staranded nucleic acid molecule_PA-0027

<400>  29
aagcagagua cacacagcat ataccggtau augcugugug uacucugctt          50


<210>  30
<211>  50
<212>  DNA
<213>  Artificial Sequence

<220>
<223>  single-staranded nucleic acid molecule_PA-0028


<220>
<221>  modified_base
<222>  (4)..(4)
<223>  cm

<220>
<221>  modified_base
<222>  (11)..(11)
<223>  cm

<220>
<221>  modified_base
<222>  (13)..(13)
<223>  cm

<220>
<221>  modified_base
<222>  (15)..(15)
<223>  cm

<220>
<221>  modified_base
<222>  (18)..(18)
<223>  cm

<220>
<221>  modified_base
<222>  (34)..(34)
<223>  cm

<220>
<221>  modified_base
<222>  (43)..(43)
<223>  cm

<220>
<221>  modified_base
<222>  (45)..(45)
<223>  cm

<220>
<221>  modified_base
<222>  (48)..(48)
<223>  cm

<400>  30
aagcagagua cacacagcat ataccggtau augcugugug uacucugcuu                50


<210>  31
<211>  50
<212>  DNA
<213>  Artificial Sequence

<220>
<223>  single-staranded nucleic acid molecule_PA-0029


<220>
<221>  modified_base
<222>  (9)..(9)
<223>  um

<220>
<221>  modified_base
<222>  (32)..(32)
<223>  um

<220>
<221>  modified_base
<222>  (35)..(35)
<223>  um

<220>
<221>  modified_base
<222>  (37)..(37)
<223>  um

<220>
<221>  modified_base
<222>  (39)..(39)
<223>  um

<220>
<221>  modified_base
<222>  (41)..(41)
<223>  um

<220>
<221>  modified_base
<222>  (44)..(44)
<223>  um

<220>
<221>  modified_base
<222>  (46)..(46)
<223>  um

<220>
<221>  modified_base
<222>  (49)..(49)
<223>  um

<220>
<221>  modified_base
<222>  (50)..(50)
<223>  um

<400>  31
aagcagagua cacacagcat ataccggtau augcugugug uacucugcuu                50


<210>  32
<211>  50
<212>  DNA
<213>  Artificial Sequence

<220>
<223>  single-staranded nucleic acid molecule_PA-0030


<220>
<221>  modified_base
<222>  (20)..(20)
<223>  um

<220>
<221>  modified_base
<222>  (21)..(21)
<223>  2'-O--methyladenosine

<220>
<221>  modified_base
<222>  (22)..(22)
<223>  um

<220>
<221>  modified_base
<222>  (23)..(23)
<223>  2'-O--methyladenosine

<220>
<221>  modified_base
<222>  (24)..(24)
<223>  cm

<220>
<221>  modified_base
<222>  (25)..(25)
<223>  cm

<400>  32
aagcagagua cacacagcau auaccggtau augcugugug uacucugctt          50


<210>  33
<211>  50
<212>  RNA
<213>  Artificial Sequence

<220>
<223>  single-staranded nucleic acid molecule_GA-0001

<400>  33
aagcagagua cacacagcau auaccgguau augcugugug uacucugcuu          50


<210>  34
<211>  50
<212>  RNA
<213>  Artificial Sequence

<220>
<223>  single-staranded nucleic acid molecule_KA-0001

<400>  34
aagcagagua cacacagcau auaccgguau augcugugug uacucugcuu          50


<210>  35
<211>  54
<212>  DNA
<213>  Artificial Sequence

<220>
<223>  single-staranded nucleic acid molecule_PA-0031

<400>  35
aauagcacca uuugaaauca gugutccgga acacugauuu caaauggugc uauu     54


<210>  36
<211>  54
<212>  RNA
<213>  Artificial Sequence

<220>
<223>  single-staranded nucleic acid molecule_PA-0032

<400>  36
aacacugauu ucaaauggug cuagaccggu cuagcaccau uugaaaucag uguu          54


<210>  37
<211>  54
<212>  DNA
<213>  Artificial Sequence

<220>
<223>  single-staranded nucleic acid molecule_PA-0033

<400>  37
aacacugauu ucaaauggug ctagaccggt cuagcaccau uugaaaucag uguu          54


<210>  38
<211>  21
<212>  RNA
<213>  Artificial Sequence

<220>
<223>  siRNA_NI-0053 (sense)

<400>  38
gcagaguaca cacagcauau a                                              21


<210>  39
<211>  21
<212>  RNA
<213>  Artificial Sequence

<220>
<223>  siRNA_NI-0053 (antisense)

<400>  39
uaugcugugu guacucugcu u                                              21


<210>  40
<211>  21
<212>  RNA
<213>  Artificial Sequence

<220>
<223>  siRNA_NI-0011(sense)

<400>  40
ccaugagaag uaugacaaca g                                              21


<210>  41
<211>  21
<212>  RNA
<213>  Artificial Sequence

<220>
<223>  siRNA_NI-0011 (antisense)

<400>  41
guugucauac uucucauggu u                                              21

<210>  42
<211>  23
<212>  RNA
<213>  Artificial Sequence

<220>
<223>  siRNA_NI-0211(sense)

<400>  42
cacugauuuc aaauggugcu aga                                                23


<210>  43
<211>  23
<212>  RNA
<213>  Artificial Sequence

<220>
<223>  siRNA_NI-0211(antisense)

<400>  43
uagcaccauu ugaaaucagu guu                                                23


<210>  44
<211>  48
<212>  RNA
<213>  Artificial Sequence

<220>
<223>  single-staranded nucleic acid molecule_PH-0009

<400>  44
gcagaguaca cacagcauau accgguauau gcugugugua cucugcuu                     48


<210>  45
<211>  22
<212>  DNA
<213>  Artificial Sequence

<220>
<223>  PCR primer for TGF-beta 1

<400>  45
ttgtgcggca gtggttgagc cg                                                 22


<210>  46
<211>  24
<212>  DNA
<213>  Artificial Sequence

<220>
<223>  PCR primer for TGF-beta 1

<400>  46
gaagcaggaa aggccggttc atgc                                               24


<210>  47

<211>  20
<212>  DNA
<213>  Artificial Sequence

<220>
<223>  PCR primer for COL1A1

<400>  47
cccaaggaca agaggcatgt                                                   20


<210>  48
<211>  20
<212>  DNA
<213>  Artificial Sequence

<220>
<223>  PCR primer for COL1A1

<400>  48
ccgccatact cgaactggaa                                                   20


<210>  49
<211>  23
<212>  DNA
<213>  Artificial Sequence

<220>
<223>  PCR primer for beta actin

<400>  49
gccacggctg cttccagctc ctc                                               23


<210>  50
<211>  25
<212>  DNA
<213>  Artificial Sequence

<220>
<223>  PCR primer for beta actin

<400>  50
aggtctttgc ggatgtccac gtcac                                             25


<210>  51
<211>  19
<212>  DNA
<213>  Artificial Sequence

<220>
<223>  PCR primer for GAPDH

<400>  51
atggggaagg tgaaggtcg                                                    19


<210>  52
<211>  23
<212>  DNA
<213>  Artificial Sequence

<220>
<223>  PCR primer for beta actin

<400>  52
gggtcattga tggcaacaat atc                                              23

## Description

### [Technical Field]

The present invention relates to a cyclic nucleic acid molecule having a gene expression regulation function, a composition containing same and use thereof.

### [Background Art]

As a technique for inhibiting gene expression, for example, RNA interference (RNAi) is known (Non-Patent Document 1). Inhibition of gene expression by RNA interference is generally carried out, for example, by administering a short double-stranded RNA molecule to a cell or the like. The aforementioned double-stranded RNA molecule is generally called siRNA (small interfering RNA). It has been reported that gene expression can also be inhibited by a circular RNA molecule having a double strand partially formed therein by intermolecular annealing (Patent Document 1).

MicroRNA (miRNA) is known as a nucleic acid molecule that inhibits gene expression and has been reported to inhibit transcription of a protein encoded by a gene via, for example, the following production process. That is, an miRNA transcript (Pri-miRNA) having a cap structure on the 5'-terminal and poly(A) on the 3'-terminal is produced in the nucleus. The aforementioned Pri-miRNA is cleaved by RNase (Drosha) to produce a miRNA precursor (Pre-miRNA). The aforementioned Pre-miRNA forms a hairpin structure having a loop region and a stem region. The Pre-miRNA moves out from the nucleus and is degraded by RNase (Dicer) in the cytoplasm, and a double-stranded miRNA having 1 - 4 bases of overhang on the 3'-terminal is cleaved out. One of the strands of the double-stranded miRNA is called a guide strand and the other strand is called a passenger strand, and the aforementioned guide strand is bonded to a complex similar to RNA-induced Silencing Complex (RISC). This miRNA/RISC complex binds to a particular mRNA to induce a cleavage of the aforementioned mRNA or an inhibition of translation of the aforementioned mRNA into a protein.

It has been clarified that miRNA is deeply involved in life phenomena such as differentiation, cell proliferation, apoptosis and the like and many diseases such as viral infections, cancer and the like (patent document 2, non-patent document 2, non-patent document 3). Therefrom its application in, particularly, the medical field has been expected.

For the purpose of improving the stability in vivo and reducing the side effects due to the enhanced innate immune response, self-annealing single-stranded nucleic acid molecules possibly having one or two hairpin-type partial secondary structures and having terminals of double-stranded nucleic acids such as siRNA, miRNA and the like linked by various linkers have been developed (patent documents 2 - 5).

### [Document List]

### [Patent documents]

patent document 1: JP-A-2008-278784
patent document 2: WO 2012/017919
patent document 3: WO 2013/103146
patent document 4: WO 2012/005368
patent document 5: WO 2013/180038

### [non-patent document]

non-patent document 1: Fire, et al., 1998, Nature, 391, 806-811
non-patent document 2: Deiters, 2009, The AAPS Journal, 12, 51-60
non-patent document 3: Takeshita et al., 2010, Mol. Ther., 18, 181-187

### [SUMMARY OF THE INVENTION]

### [Problems to be Solved by the Invention]

Generally, nucleic acid is easily degraded and unstable in solution, and particularly inferior in the stability in serum. For example, when a nucleic acid molecule is administered to a living body for the purpose of regulating gene expression, a problem occurs that a desired effect cannot be obtained sufficiently. In addition, problems of intracellular transferability and pharmacokinetics also exist.

The present invention aims to provide a nucleic acid molecule having improved stability in serum and capable of exhibiting a gene expression regulation function after transfer into the cell.

### [Means of Solving the Problems]

The present inventors have conducted intensive studies in an attempt to solve the aforementioned problems and succeeded in improving the stability in the serum by cyclizing a single-stranded nucleic acid molecule with a gene expression regulation function by a disulfide bond and further enhancing the stability in the serum by revising the structures of the 3'-side and the loop side, which resulted in the completion of the present invention. That is, the present invention provides the following.
[1] A cyclic nucleic acid molecule represented by the following formula (A): 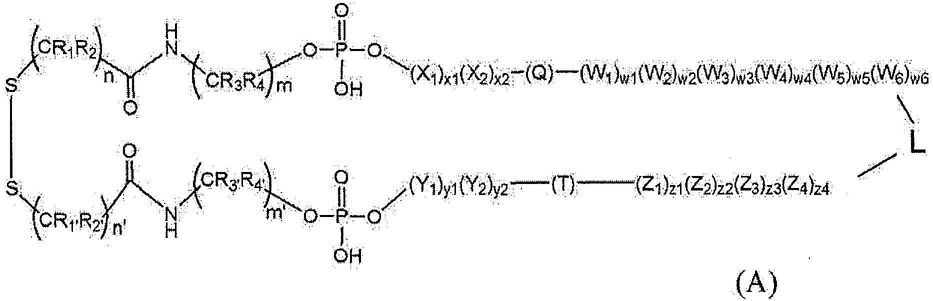 wherein
   R₁, R₂, R_{1'} and R_{2'} are each independently a hydrogen atom, an alkyl group, an alkoxy group or hydroxy;
   R₃, R₄, R_{3'} and R_{4'} are each independently a hydrogen atom, an alkyl group, an alkoxy group or hydroxy;
   X₁ and X₂ are each independently an optionally modified ribonucleotide residue or deoxyribonucleotide residue;
   Y₁ and Y₂ are each independently an optionally modified ribonucleotide residue or deoxyribonucleotide residue;
   Z₁ - Z₄ are each independently an optionally modified ribonucleotide residue or deoxyribonucleotide residue;
   W₁ - W₆ are each independently an optionally modified ribonucleotide residue or deoxyribonucleotide residue;
   Q and T are each a guide strand or a passenger strand (when one is a guide strand, the other is a passenger strand);
   L is a linker;
   m and m' are each independently an integer of 1 - 12;
   n and n' are each independently an integer of 1 - 10;
   x1 and x2 are each independently 0 or 1;
   y1 and y2 are each independently 0 or 1;
   z1 - z4 are each independently 0, 1 or 2; and
   w1 - w6 are each independently 0, 1 or 2.
[2] The nucleic acid molecule of the above-mentioned [1] wherein X₁, X₂, Y₁, Y₂, Z₁ - Z₄ and W₁ - W₆ are each independently a ribonucleotide residue or deoxyribonucleotide residue optionally modified by a methyl group.
[3] The nucleic acid molecule of the above-mentioned [1] or [2]
   wherein X₁ and X₂ are each independently a ribonucleotide residue optionally modified by a methyl group;
   Y₁ and Y₂ are each independently a ribonucleotide residue optionally modified by a methyl group;
   Z₁ - Z₄ are each independently a ribonucleotide residue or deoxyribonucleotide residue optionally modified by a methyl group; and
   W₁ - W₆ are each independently a ribonucleotide residue or deoxyribonucleotide optionally modified by a methyl group.
[4] The nucleic acid molecule of any of the above-mentioned [1] to [3] wherein the linker is represented by any of the following formula: 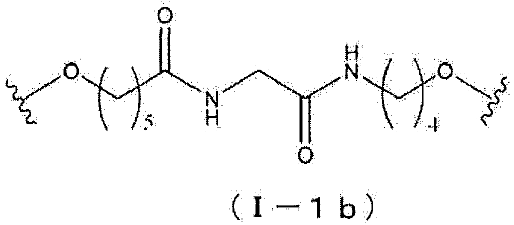 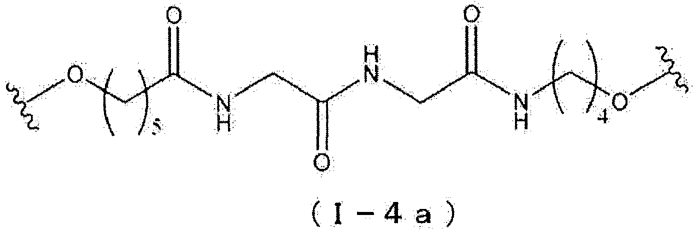 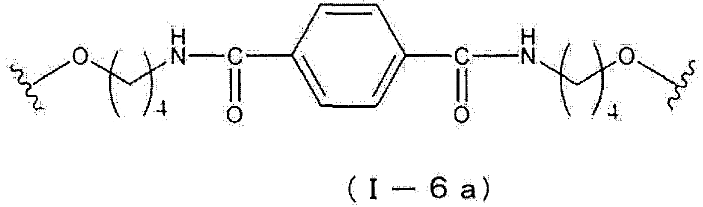 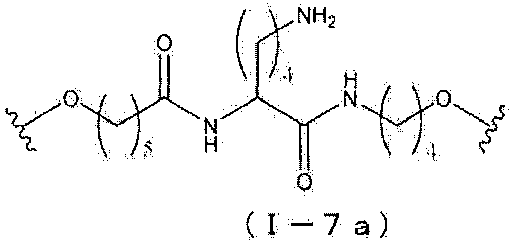 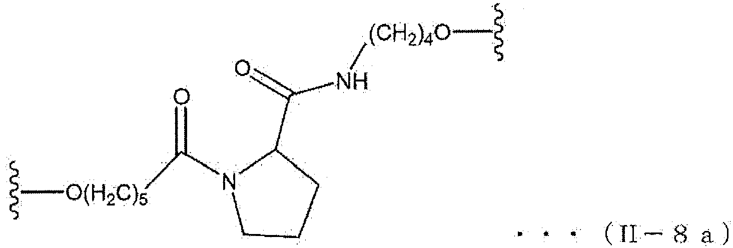 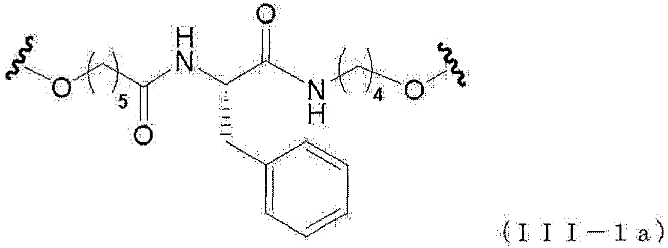 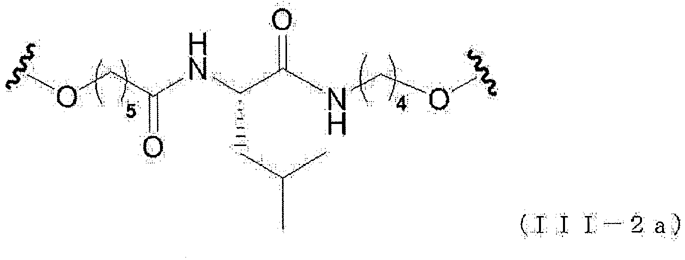 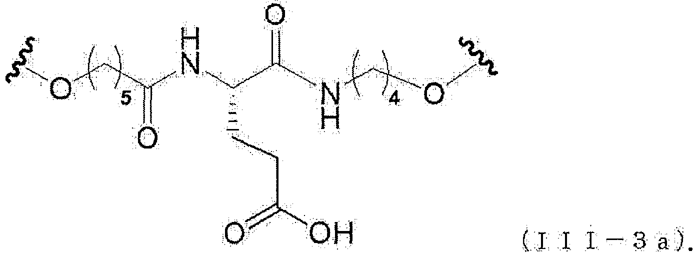
[5] The nucleic acid molecule of any of the above-mentioned [1] to [4] wherein at least one of X₁, X₂, Y₁, Y₂, Z₁ - Z₄ and W₁ - W₆ is independently a ribonucleotide residue or deoxyribonucleotide residue optionally modified by a methyl group.
[6] The nucleic acid molecule of any of the above-mentioned [1] to [4] wherein at least five of X₁, X₂, Y₁, Y₂, Z₁ - Z₄ and W₁ - W₆ are each independently a ribonucleotide residue or deoxyribonucleotide residue optionally modified by a methyl group.
[7] The nucleic acid molecule of any of the above-mentioned [1] to [6] comprising a polynucleotide strand comprising a guide strand, a passenger strand and a linker, and a double stranded structure formed in a part thereof, wherein both ends of the double stranded structure are blunt ends.
[8] A composition for inhibiting expression of a target gene, comprising the nucleic acid molecule of any of the above-mentioned [1] to [7].
[9] A pharmaceutical composition comprising the nucleic acid molecule of any of the above-mentioned [1] to [7].
[10] A method for inhibiting expression of a target gene comprising using the nucleic acid molecule of any of the above-mentioned [1] to [7].
[11] The method for inhibiting expression of the above-mentioned [10] comprising a step of administering the aforementioned nucleic acid molecule to a cell, tissue or organ.
[12] The method for inhibiting expression of the above-mentioned [10] or [11] wherein the aforementioned nucleic acid molecule is administered in vivo or in vitro.
[13] The method for inhibiting expression of the above-mentioned [10] or [11] wherein the aforementioned nucleic acid molecule is administered to a non-human animal.

### [Effect of the Invention]

The cyclic nucleic acid molecule of the present invention shows improved stability in serum and activity on cleavage after intracellular transfer, and therefore, sustention of the effect can be expected.

### [Brief Description of the Drawings]

Fig. 1 is an RP-HPLC chart of PA-0001-DSP.
Fig. 2 is an RP-HPLC chart of PA-0001-DSO.
Fig. 3 is an RP-HPLC chart of PA-0002-DSP.
Fig. 4 is an RP-HPLC chart of PA-0003-DSP.
Fig. 5 is an RP-HPLC chart of PA-0004-DSP.
Fig. 6 is an RP-HPLC chart of PA-0005-DSP.
Fig. 7 is an RP-HPLC chart of PA-0006-DSP.
Fig. 8 is an RP-HPLC chart of PA-0007-DSP.
Fig. 9 is an RP-HPLC chart of PA-0008-DSP.
Fig. 10 is an RP-HPLC chart of PA-0009-DSP.
Fig. 11 is an RP-HPLC chart of PA-0010-DSP.
Fig. 12 is an RP-HPLC chart of PA-0011-DSP.
Fig. 13 is an RP-HPLC chart of PA-0012-DSP.
Fig. 14 is an RP-HPLC chart of PA-0013-DSP.
Fig. 15 is an RP-HPLC chart of PA-0014-DSP.
Fig. 16 is an RP-HPLC chart of PA-0015-DSP.
Fig. 17 is an RP-HPLC chart of PA-0016-DSP.
Fig. 18 is an RP-HPLC chart of PA-0017-DSP.
Fig. 19 is an RP-HPLC chart of PA-0018-DSP.
Fig. 20 is an RP-HPLC chart of PA-0019-DSP.
Fig. 21 is an RP-HPLC chart of PA-0020-DSP.
Fig. 22 is an RP-HPLC chart of PA-0021-DSP.
Fig. 23 is an RP-HPLC chart of PA-0024-DSP.
Fig. 24 is an RP-HPLC chart of PA-0025-DSP.
Fig. 25 is an RP-HPLC chart of PA-0026-DSP.
Fig. 26 is an RP-HPLC chart of PA-0027-DSP.
Fig. 27 is an RP-HPLC chart of PA-0028-DSP.
Fig. 28 is an RP-HPLC chart of PA-0029-DSP.
Fig. 29 is an RP-HPLC chart of PA-0030-DSP.
Fig. 30 is an RP-HPLC chart of GA-0001-DSP.
Fig. 31 is an RP-HPLC chart of KA-0001-DSP.
Fig. 32 is an RP-HPLC chart of PA-0031-DSP.
Fig. 33 is an RP-HPLC chart of PA-0032-DSP.
Fig. 34 is an RP-HPLC chart of PA-0033-DSP.
Fig. 35 is an electrophoresis image showing the evaluation results of the stability of each cyclic nucleic acid molecule in serum.
Fig. 36 is an electrophoresis image showing the evaluation results of the stability of each cyclic nucleic acid molecule in serum.
Fig. 37 is an electrophoresis image showing the evaluation results of the stability of each cyclic nucleic acid molecule in serum.
Fig. 38 is an electrophoresis image showing the evaluation results of the stability of each cyclic nucleic acid molecule in serum.
Fig. 39 is an electrophoresis image showing the evaluation results of the stability of each cyclic nucleic acid molecule in serum.
Fig. 40 is an electrophoresis image showing the evaluation results of the stability of each cyclic nucleic acid molecule in serum.
Fig. 41 is an electrophoresis image showing the evaluation results of the stability of each cyclic nucleic acid molecule in serum.
Fig. 42 is an electrophoresis image showing the evaluation results of the stability of each cyclic nucleic acid molecule in serum.
Fig. 43 is an electrophoresis image showing the evaluation results of the stability of each cyclic nucleic acid molecule in serum.
Fig. 44-1 is a graph showing the gene expression inhibitory effect of each cyclic nucleic acid molecule.
Fig. 44-2 is a graph showing the gene expression inhibitory effect of each cyclic nucleic acid molecule.

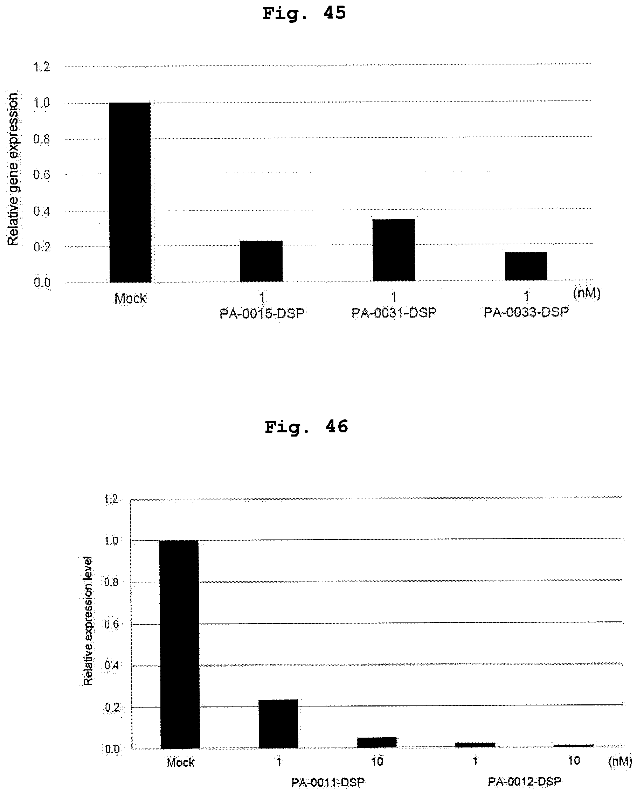

Fig. 45 is a graph showing the gene expression inhibitory effect of each cyclic nucleic acid molecule.
Fig. 46 is a graph showing the gene expression inhibitory effect of each cyclic nucleic acid molecule.
Fig. 47 is a graph showing the evaluation results of the stability of each nucleic acid molecule in the body (blood) of mouse.

### [Description of Embodiments]

Unless otherwise specified, the terms used in the present specification mean what is generally meant by them in the art.

### (1) cyclic nucleic acid molecule

As mentioned above, the cyclic nucleic acid molecule of the present invention is characteristically represented by the following formula (A).

The formula (A): 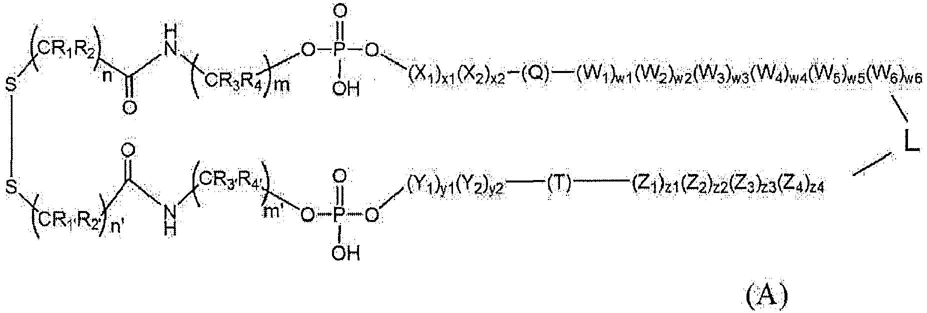 wherein
R₁, R₂, R_{1'} and R_{2'} are each independently a hydrogen atom, an alkyl group, an alkoxy group or hydroxy;
R₄, R_{3'} and R_{4'} are each independently a hydrogen atom, an alkyl group, an alkoxy group or hydroxy;
X₁ and X₂ are each independently an optionally modified ribonucleotide residue or deoxyribonucleotide residue;
Y₁ and Y₂ are each independently an optionally modified ribonucleotide residue or deoxyribonucleotide residue;
Z₁ - Z₄ are each independently optionally modified ribonucleotide residue or deoxyribonucleotide;
W₁ - W₆ are each independently an optionally modified ribonucleotide residue or deoxyribonucleotide residue;
Q and T are each a guide strand or passenger strand (when one is a guide strand, the other is a passenger strand);
L is a linker;
m and m' are each independently an integer of 1 - 12;
n and n' are each independently an integer of 1 - 10;
x1 and x2 are each independently 0 or 1;
y1 and y2 are each independently 0 or 1;
z1 - z4 are each independently 0, 1 or 2; and
w1 - w6 are each independently 0, 1 or 2.

The cyclic nucleic acid molecule of the present invention can inhibit, for example, expression of the target gene. Inhibition of expression means, for example, inhibition of the translation of the aforementioned target gene, that is, inhibition of the translation of a protein encoded by the aforementioned target gene, more particularly, inhibition of the translation of the aforementioned protein from mRNA of the aforementioned target gene. The aforementioned inhibition of the expression of the target gene can be verified by, for example, a decrease in the amount of a transcript derived from the target gene; a decrease in the activity of the aforementioned transcript; a decrease in the amount of a translation product generated from the aforementioned target gene; a decrease in the activity of the aforementioned translation product; or the like. The aforementioned proteins may be, for example, mature proteins, precursor proteins before being subjected to processing or post-translational modification.

In the cyclic nucleic acid molecule of the present invention, the 5'-terminal and the 3'-terminal of the single stranded nucleic acid molecule are linked by a disulfide bond. Therefore, annealing of two single strands is not necessary unlike, for example, a cyclic nucleic acid molecule, and the cyclic nucleic acid molecule can be produced at a low cost. Furthermore, the stability in serum is improved as shown in the below-mentioned Examples.

In the cyclic nucleic acid molecule of the present invention, a guide strand sequence and a passenger strand sequence are linked via a linker molecule of a non-nucleotide structure. Therefore, the cyclic nucleic acid molecule can be synthesized easily and provided at a low cost, and is also superior in the pharmacokinetics and intracellular transferability, as compared to a single-stranded nucleic acid molecule comprising a pre-miRNA having a comparatively long nucleotide loop.

In the cyclic nucleic acid molecule of the present invention, the aforementioned Q region contains a guide strand sequence or a passenger strand sequence of any nucleic acid molecule having a gene expression regulation function. When the aforementioned Q region contains the aforementioned guide strand sequence, the aforementioned T region contains a passenger strand sequence of the aforementioned nucleic acid molecule having a gene expression regulation function, and when the aforementioned Q region contains the aforementioned passenger strand sequence, the aforementioned T region contains the aforementioned guide strand sequence.

In the cyclic nucleic acid molecule of the present invention, the aforementioned Q region contains, as mentioned above, a guide strand sequence (a passenger strand sequence). On the other hand, the aforementioned T region contains a passenger strand sequence (a guide strand sequence). The guide strand sequence and a passenger strand sequence of a nucleic acid molecule having a gene expression regulation function are, for example, registered in various databases (e.g., http://www.mirbase.org/ etc.). Therefore, the aforementioned Q region and the aforementioned T region can be set based on, for example, the information.

In the aforementioned Q(T) region, the length of the aforementioned guide strand sequence (passenger strand sequence) is not particularly limited and may be, for example, the length of a guide strand sequence (passenger strand sequence). Specific examples thereof include a lower limit of 10 - 15 base length and an upper limit of 25 - 30 base length, and ranges of 10 - 30 base length, 15 - 20 base length.

In the cyclic nucleic acid molecule of the present invention, the sequence of the aforementioned nucleic acid molecule having a gene expression regulation function is, for example, a sequence that exhibits an activity of inhibiting the expression of the aforementioned target gene when the cyclic nucleic acid molecule of the present invention is introduced into a cell in vivo or in vitro. The aforementioned expression inhibitory sequence is not particularly limited, and can be set as appropriate depending on the kind of a target gene. As the aforementioned expression inhibitory sequence, for example, a sequence involved in RNA interference caused by siRNA can be used as appropriate. Generally, RNA interference is a phenomenon in which a long double-stranded RNA (dsRNA) is cleaved in a cell by Dicer to produce a double-stranded RNA (siRNA: small interfering RNA) composed of about 19 to 21 base pairs and having a protruding 3' end, and one of the single-stranded RNAs binds to a target mRNA to degrade the aforementioned mRNA, whereby the translation of the mRNA is inhibited. As the sequence of the single-stranded RNA of the aforementioned siRNA binding to the aforementioned target mRNA, for example, various kinds of sequences for various kinds of target genes have been reported. In the present invention, for example, the sequence of the single-stranded RNA of the aforementioned siRNA can be used as the aforementioned expression inhibitory sequence. In the present invention, not only the sequences of the single-stranded RNA of the siRNA known at the time of the filing of the present application but also sequences that would be identified in the future can be used, for example, as the aforementioned expression inhibitory sequence.

Specifically, as a nucleic acid molecule having the aforementioned gene expression regulation function, for example, a nucleic acid molecule for inhibiting expression of TGF-β1 gene can be mentioned. The following nucleotide sequence is characteristically contained as a sequence for inhibiting expression of TGF-β1 gene.
3'-CGUCUCAUGUGUGUCGUAU-5' (SEQ ID NO: 1)

When the sequence is selected as a guide strand sequence, a sequence complementary thereto can be used as a passenger strand sequence. The aforementioned complementary sequence may be, for example, a sequence showing 100% complementarity to the aforementioned guide strand sequence or a sequence showing less than 100% complementarity within an annealable range. The aforementioned complementarity is not particularly limited and it is, for example, 90% - 100%, 93% - 100%, 95% - 100%, 98% - 100%, 99% - 100% and the like.

As the aforementioned complementary sequence, for example, one having the following nucleotide sequence can be recited. 5'-GCAGAGUACACACAGCA-3' (SEQ ID NO: 2)

For example, when a nucleic acid molecule for inhibiting expression TGF-β1 gene is used as a nucleic acid molecule having a gene expression regulation function, diseases caused by the expression of TGF-β1 gene, such as lung fibrosis, acute lung injury and the like, can be prevented or treated since expression of the TGF-β1 gene can be inhibited.

As the aforementioned nucleic acid molecule having a gene expression regulation function, for example, a nucleic acid molecule for inhibiting expression of COL1A gene can be mentioned. As the nucleic acid molecule for inhibiting expression of COL1A gene, miR-29b1 can be mentioned, and it characteristically contains the following nucleotide sequence. 3'-GUGACUAAAGUUUACCACGAU-5' (SEQ ID NO: 3)

When the sequence is selected as a guide strand sequence, a sequence complementary thereto can be used as a passenger strand sequence. The aforementioned complementary sequence may be, for example, a sequence showing 100% complementarity to the aforementioned guide strand sequence or a sequence showing less than 100% complementarity within an annealable range. The aforementioned complementarity is not particularly limited and it is, for example, 90% - 100%, 93% - 100%, 95% - 100%, 98% - 100%, 99% - 100% and the like.

As the aforementioned complementary sequence, for example, one having the following nucleotide sequence can be recited. 5'-CACUGAUUUCAAAUGGUGCUAGA-3' (SEQ ID NO: 4)

For example, when a nucleic acid molecule for inhibiting expression COL1A1 gene is used as a nucleic acid molecule having a gene expression regulation function, diseases caused by the expression of COL1A1 gene, such as osteogenesis imperfecta and the like, can be prevented or treated since expression of the COL1A1 gene can be inhibited.

In the formula (A),
R₁ and R₂ in the number of n and R_{1'} and R_{2'} in the number of n' are each independently a hydrogen atom, an alkyl group (described later), an alkoxy group (described later) or hydroxy. R₁ and R₂ in the number of n and R_{1'} and R_{2'} in the number of n' are preferably the same, more preferably the same and hydrogen atoms.

In the formula (A),
R₃ and R₄ in the number of m and and R_{4'} in the number of m' are each independently a hydrogen atom, an alkyl group (described later), an alkoxy group (described later) or hydroxy. R₃ and R₄ in the number of m and R_{3'} and R_{4'} in the number of m' are preferably the same, more preferably the same and hydrogen atoms.

X₁ and X₂ are each independently an optionally modified ribonucleotide residue or deoxyribonucleotide residue.

Y₁ and Y₂ are each independently an optionally modified ribonucleotide residue or deoxyribonucleotide residue.

Z₁ - Z₄ are each independently an optionally modified ribonucleotide residue or deoxyribonucleotide residue.

W₁ - W₆ are each independently an optionally modified ribonucleotide residue or deoxyribonucleotide residue.

Preferably, X₁, X₂, Y₁, Y₂, Z₁ - Z₄ and W₁ - W₆ are each independently a ribonucleotide residue or deoxyribonucleotide residue optionally modified by a methyl group. Preferably, any one, more preferably any two, further preferably three, particularly preferably four, particularly more preferably five, most preferably six, of X₁, X₂, Y₁, Y₂, Z₁ - Z₄ and W₁ - W₆ are ribonucleotide residues or deoxyribonucleotide residues optionally modified by methyl group.

Preferably, any one, more preferably any two, particularly preferably any three, of Z₁ - Z₄ are ribonucleotide residues or deoxyribonucleotide residues optionally modified by methyl group.

Preferably, any one, more preferably any two, further preferably three, particularly preferably four, particularly more preferably five, most preferably six, of W₁ - W₆ are ribonucleotide residues or deoxyribonucleotide residues optionally modified by methyl group.

In the present specification, the ribonucleotide residue and the deoxyribonucleotide residue are also collectively referred to as nucleotide residue. The aforementioned nucleotide residue is preferably a ribonucleotide residue. The aforementioned nucleotide residue may be, for example, the one that is not modified (unmodified nucleotide residue) or the one that has been modified (modified nucleotide residue). By configuring the nucleic acid molecule of the present invention to include the aforementioned modified nucleotide residue, for example, the resistance of the nucleic acid molecule to nuclease can be improved, thereby allowing the stability of the nucleic acid molecule to be improved. The aforementioned nucleotide residue is optionally substituted by a methoxy group.

The aforementioned components of the aforementioned unmodified nucleotide residue are the same or substantially the same as, for example, the components of a naturally-occurring nucleotide residue. Specifically, for example, the components are the same or substantially the same as the components of a nucleotide residue occurring naturally in a human body.

For example, the aforementioned modified nucleotide residue may be such that any of the components of the aforementioned unmodified nucleotide residue is modified. Examples of the aforementioned modified nucleotide residue include naturally-occurring nucleotide residues and artificially-modified nucleotide residues.

The optionally modified ribonucleotide residue or deoxyribonucleotide residue may be, for example, a residue of an alternative of fluorine atom, a methyl group or the aforementioned unmodified nucleotide. Examples of the aforementioned alternative include artificial nucleic acid monomer residues. Specific examples thereof include PNA (Peptide Nucleic Acid), LNA (Locked Nucleic Acid), and ENA (2'-O,4'-C-Ethylenebridged Nucleic Acids).

The aforementioned nucleotide residue contains, for example, sugar, base and phosphoric acid as constituent elements. In the aforementioned nucleotide residue, the aforementioned base is not particularly limited. The aforementioned base may be, for example, a natural base or a non-natural base. The aforementioned base may be, for example, a naturally-derived base or a synthetic base. As the aforementioned base, for example, a common base, a modified analog thereof, and the like can be used.

Examples of the aforementioned base include purine bases such as adenine and guanine and the like, and pyrimidine bases such as cytosine, uracil, thymine and the like. Examples of the aforementioned base include, besides these, inosine, thymine, xanthine, hypoxanthine, purine, isoguanine, 7-deaza-adenine and the like. Examples of the aforementioned base include alkyl derivatives such as 2-aminoadenine, 6-methylated purine and the like; alkyl derivatives such as 2-propylated purine and the like; 5-halouracil and 5-halocytosine; 5-propynyluracil and 5-propynylcytosine; 6-azouracil, 6-azocytosine and 6-azothymine; 5-uracil (pseudouracil), 4-thiouracil, 5-halouracil, 5-(2-aminopropyl)uracil, 5-aminoallyluracil; 8-halogenated, aminated, thiolated, thioalkylated, hydroxylated and other 8-substituted purines; 5-trifluoromethylated and other 5-substituted pyrimidines; 7-methylguanine; 5-substituted pyrimidine; 6-aza pyrimidine; N-2, N-6, and O-6-substituted purines (including 2-aminopropyladenine); dihydrouracil; 3-deaza-5-aza cytosine; 2-aminopurine; 5-alkyluracil; 7-alkylguanine; 5-alkylcytosine; N6,N6-dimethyladenine; 5-amino-allyl-uracil; N3-methyluracil; substituted 1,2,4-triazole; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N4-acetylcytosine; 2-thiocytosine; N6-methyladenine; N6-isopentyladenine; 2-methylthio-N6-isopentenyladenine; N-methylguanine; O-alkylated base and the like. Purine and pyrimidine include, for example, those disclosed in US Patent No. 3,687,808, "Concise Encyclopedia Of Polymer Science And Engineering", pages 858 - 859, ed. Kroschwitz J.I., John Wiley & Sons, 1990, and Englisch et al., Angewandte Chemie, International Edition, 1991, vol. 30, p. 613.

In the formula (A),
m and m' are each independently an integer of 1 - 12, preferably the same and integers of 4 - 8, particularly preferably 6.

n and n' are each independently an integer of 1 - 10, preferably the same and integers of 2 - 7, particularly preferably 2 or 7.

x1 and x2 are each independently 0 or 1, preferably the same and 1.

y1 and y2 are each independently 0 or 1, preferably the same and 1.

z1 - z4 are each independently 0, 1 or 2, preferably 0 or 1, more preferably the same and 1.

w1 - w6 are each independently 0, 1 or 2, preferably 0 or 1, more preferably the same and 1.

In the formula (A), L is a linker. Specifically, the linker has a structure shown by L region in the following formula (I).

The following formula (I): 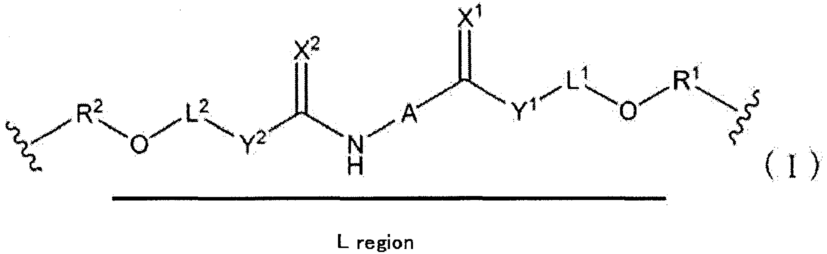 in formula (I),
X¹ and X² are each independently H₂, O, S or NH;
Y¹ and Y² are each independently a single bond, CH₂, NH, O or S;
L¹ is an alkylene chain having n_{L} carbon atoms, and a hydrogen atom on an alkylene carbon atom may or may not be substituted by OH, OR^{a}, NH₂, NHR^{a}, NR^{a}R^{b}, SH or SR^{a}, or,
L¹ is a polyether chain obtained by substituting at least one carbon atom on the aforementioned alkylene chain with an oxygen atom,
provided that when Y¹ is NH, O or S, then an atom bound to Y¹ in L¹ is carbon, an atom bound to OR¹ in L¹ is carbon, and oxygen atoms are not adjacent to each other;
L² is an alkylene chain having m_{L} carbon atoms, and a hydrogen atom on an alkylene carbon atom may or may not be substituted by OH, OR^{c}, NH₂, NHR^{c}, NR^{c}R^{d}, SH or SR^{c}, or
L² is a polyether chain obtained by substituting at least one carbon atom on the aforementioned alkylene chain with an oxygen atom,
provided that when Y² is NH, O or S, then an atom bound to Y² in L² is carbon, an atom bound to OR² in L² is carbon, and oxygen atoms are not adjacent to each other;
R^{a}, R^{b}, R^{c}, and R^{d} are each independently a substituent or a protecting group;
m_{L} is an integer of 0 - 30;
n_{L} is an integer of 0 - 30;
R¹ and R² may or may not be present, and when they are present, R¹ and R² are each independently a nucleotide residue or the aforementioned structure (I); and
A is any atomic group, provided that the following chemical formula (Ia) is an amino acid or peptide:

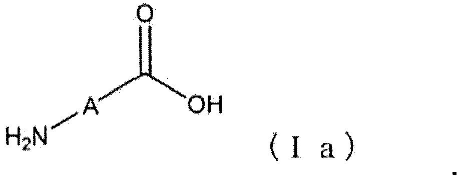

L in the aforementioned formula (A) binds to the adjacent nucleotide residue via -OR¹- or -OR²- in the aforementioned formula (I).

In the aforementioned formula (I), for example, X¹ and X² are each independently H₂, O, S or NH. In the aforementioned formula (I), "X¹ is H₂" means that X¹ forms CH₂ (a methylene group) together with a carbon atom to which X¹ binds. The same applies to X².

In the aforementioned formula (I), Y¹ and Y² are each independently a single bond, CH₂, NH, O or S.

In the aforementioned formula (I), L¹ is an alkylene chain having n_{L} carbon atoms. A hydrogen atom(s) on the aforementioned alkylene carbon atom(s) may or may not be substituted by, for example, OH, OR^{a}, NH₂, NHR^{a}, NR^{a}R^{b}, SH or SR^{a}. Alternatively, L¹ may be a polyether chain obtained by substituting at least one carbon atom on the aforementioned alkylene chain with an oxygen atom. The aforementioned polyether chain is, for example, polyethylene glycol. When Y¹ is NH, O or S, an atom bound to Y¹ in L¹ is carbon, an atom bound to OR¹ in L¹ is carbon, and oxygen atoms are not adjacent to each other. That is, for example, when Y¹ is O, this oxygen atom and the oxygen atom in L¹ are not adjacent to each other, and the oxygen atom in OR¹ and the oxygen atom in L¹ are not adjacent to each other.

In the aforementioned formula (I), L² is an alkylene chain having m_{L} carbon atoms. A hydrogen atom(s) on the aforementioned alkylene carbon atom(s) may or may not be substituted by, for example, OH, OR^{c}, NH₂, NHR^{c}, NR^{c}R^{d}, SH or SR^{c}. Alternatively, L² may be a polyether chain obtained by substituting at least one carbon atom on the aforementioned alkylene chain with an oxygen atom. When Y² is NH, O or S, an atom bound to Y² in L² is carbon, an atom bound to OR² in L² is carbon, and oxygen atoms are not adjacent to each other. That is, for example, when Y² is O, this oxygen atom and the oxygen atom in L² are not adjacent to each other, and the oxygen atom in OR² and the oxygen atom in L² are not adjacent to each other.

n_{L} of L¹ and m_{L} of L² are not particularly limited, and the lower limit of each of them may be 0, for example, and the upper limit of the same is not particularly limited. For example, n_{L} and m_{L} can be set as appropriate depending on a desired length of the aforementioned linker region L. For example, from the view point of manufacturing cost, yield, and the like, n_{L} and m_{L} are each preferably 0 - 30, more preferably 0 - 20, and still more preferably 0 - 15. n_{L} and m_{L} may be the same (n_{L} = m_{L}) or different. n_{L} + m_{L} is, for example, 0 - 30, preferably 0 - 20, and more preferably 0 - 15.

For example, R^{a}, R^{b}, R^{c} and R^{d} are each independently a substituent or a protecting group, and may be the same or different. Examples of the aforementioned substituent include hydroxy, carboxy, sulfo, halogen, alkyl halide (haloalkyl, e.g., CF₃, CH₂CF₃, CH₂CCl₃), nitro, nitroso, cyano, alkyl (e.g., methyl, ethyl, isopropyl, tert-butyl), alkenyl (e.g., vinyl), alkynyl (e.g., ethynyl), cycloalkyl (e.g., cyclopropyl, adamantyl), cycloalkylalkyl (e.g., cyclohexylmethyl, adamantylmethyl), cycloalkenyl (e.g., cyclopropenyl), cyclylalkyl, hydroxyalkyl (e.g., hydroxymethyl, hydroxyethyl), alkoxyalkyl (e.g., methoxymethyl, ethoxymethyl, ethoxyethyl), aryl (e.g., phenyl, naphthyl), arylalkyl (e.g., benzyl, phenethyl), alkylaryl (e.g., p-methylphenyl), heteroaryl (e.g., pyridyl, furyl), heteroarylalkyl (e.g., pyridylmethyl), heterocyclyl (e.g., piperidyl), heterocyclylalkenyl, heterocyclylalkyl (e.g., morpholylmethyl), alkoxy (e.g., methoxy, ethoxy, propoxy, butoxy), halogenated alkoxy (e.g., OCF₃), alkenyloxy (e.g., vinyloxy, allyloxy), aryloxy (e.g., phenyloxy), alkyloxycarbonyl (e.g., methoxycarbonyl, ethoxycarbonyl, tert-butoxycarbonyl), arylalkyloxy (e.g., benzyloxy), amino [alkylamino (e.g., methylamino, ethylamino, dimethylamino), acylamino (e.g., acetylamino, benzoylamino), arylalkylamino (e.g., benzylamino, tritylamino), hydroxyamino], aminoalkyl (e.g., aminomethyl), alkylaminoalkyl (e.g., diethylaminomethyl), carbamoyl, sulfamoyl, oxo, silyl, silyloxyalkyl and the like. These substituents are optionally substituted by one or more further substituents or further protecting groups. While the aforementioned further substituent is not particularly limited, for example, it may be a substituent exemplified above. The aforementioned further protecting group is not particularly limited and, for example, it may be a protecting group exemplified below. Hereinafter the same.

The aforementioned protecting group (or the aforementioned further protecting group) is a functional group that inactivates, for example, a highly-reactive functional group. Examples of the protecting group include known protecting groups. Regarding the aforementioned protecting group, for example, the description in the literature (J. F. W. McOmie, "Protecting Groups in Organic Chemistry", Plenum Press, London and New York, 1973) can be incorporated herein. The aforementioned protecting group is not particularly limited, and examples thereof include a tert-butyldimethylsilyl group (TBDMS), a bis(2-acetoxyethyloxy)methyl group (ACE), a triisopropylsilyloxymethyl group (TOM), a 1-(2-cyanoethoxy)ethyl group (CEE), a 2-cyanoethoxymethyl group (CEM), a tolylsulfonylethoxymethyl group (TEM), and a dimethoxytrityl group (DMTr). When R³ is OR⁴, the aforementioned protecting group is not particularly limited, and examples thereof include a TBDMS group, an ACE group, a TOM group, a CEE group, a CEM group, and a TEM group. Other examples of the protecting group include silyl-containing groups of the following formulas (P1) and (P2). Among these, it is preferably either a DMTr group or the aforementioned silyl-containing group.

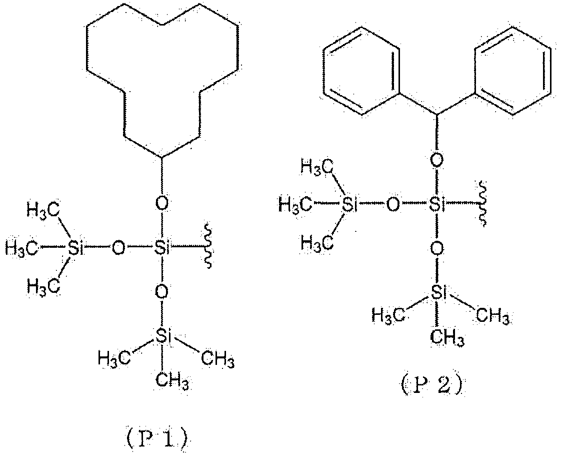

In the aforementioned formula (I), hydrogen atoms each independently may be substituted by, for example, a halogen such as Cl, Br, F or I.

L in the aforementioned formula (A) binds to a phosphoric acid group of the adjacent nucleotide residue via -OR¹- or-OR²- in the aforementioned formula (I). R¹ and R² may or may not be present. When R¹ and R² are present, R¹ and R² are each independently a nucleotide residue or the structure of the aforementioned formula (I). When R¹ and/or R² are/is the structure of the aforementioned formula (I), the structure of the aforementioned linker region (L) is such that, for example, two or more of the residues having the structure of the aforementioned formula (I) are linked to each other. The number of the structures of the aforementioned formula (I) may be, for example, 1, 2, 3 or 4. When plural structures mentioned above are contained, the structures of the aforementioned (I) may be for example, directly linked or bonded via the aforementioned nucleotide residue(s).

In the aforementioned formula (I), atomic group A is not particularly limited as long as the following formula (Ia): 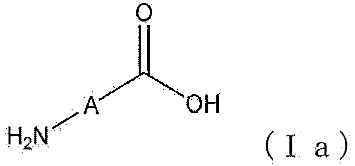 is an amino acid or peptide.

For example, the atomic group A in the aforementioned (I) or (Ia) may or may not contain, for example, at least one selected from the group consisting of chain atomic group, alicyclic atomic group and aromatic atomic group. While the aforementioned chain atomic group is not particularly limited, for example, alkyl, alkenyl, alkynyl, haloalkyl, hydroxyalkyl, alkoxyalkyl, aminoalkyl, silyl, silyloxyalkyl and the like can be mentioned. While the aforementioned alicyclic atomic group is not particularly limited, for example, cycloalkyl, cycloalkenyl, cycloalkylalkyl, cyclylalkyl and the like can be mentioned. While the aforementioned aromatic atomic group is not particularly limited, for example, aryl, arylalkyl, alkylaryl, condensed aryl, condensed arylalkyl, condensed alkylaryl and the like can be mentioned. In the atomic group A in the aforementioned formula (I) or (Ia), each of the aforementioned atomic groups may or may not further have a substituent or a protecting group. When the aforementioned substituent or protecting group is in plurality, they may be the same or different. The aforementioned substituents are, for example, those exemplified for the aforementioned R^{a}, R^{b}, R^{c} and R^{d}, more specifically, for example, halogen, hydroxy, alkoxy, amino, carboxy, sulfo, nitro, carbamoyl, sulfamoyl, alkyl, alkenyl, alkynyl, haloalkyl, aryl, arylalkyl, alkylaryl, cycloalkyl, cycloalkenyl, cycloalkylalkyl, cyclylalkyl, hydroxyalkyl, alkoxyalkyl, aminoalkyl, silyl, silyloxyalkyl, pyrrolyl, imidazolyl, and the like. The aforementioned protecting groups are, for example, the same as those exemplified for the aforementioned R^{a}, R^{b}, R^{c} and R^{d}.

In the present invention, the "amino acid" refers to any organic compound containing at least one amino group and at least one carboxy group in a molecule. The "peptide" in the present invention refers to an organic compound having a structure wherein not less than 2 molecules of amino acid are bonded via a peptide bond. The aforementioned peptide bond may be an acid amide structure or an acid imide structure. When plural amino groups are present in the amino acid molecule represented by the aforementioned formula (Ia), the amino group clearly shown in the aforementioned formula (Ia) may be any amino group. In addition, when plural carboxy groups are present in the amino acid molecule represented by the aforementioned formula (Ia), the carboxy group clearly shown in the aforementioned formula (Ia) may be any carboxy group.

In the aforementioned linker region (L) of the cyclic nucleic acid molecule of the present invention, the aforementioned amino acid may be natural amino acid or artificial amino acid. In the present invention, the "natural amino acid" refers to an amino acid having a naturally-occurring structure or an optical isomer thereof. The production method of the aforementioned natural amino acid is not particularly limited and, for example, it may be extracted from the nature or may be synthesized. In the present invention, moreover, the "artificial amino acid" refers to an amino acid having a structure not occurring naturally. That is, the aforementioned artificial amino acid is an amino acid, i.e., a carboxylic acid derivative containing an amino group (organic compound containing at least one amino group and at least one carboxy group in a molecule) and having a structure not occurring naturally. The aforementioned amino acid may be an amino acid constituting, for example, a protein. The aforementioned amino acid may be, for example, at least one kind selected from the group consisting of glycine, α-alanine, arginine, asparagine, aspartic acid, cysteine, cystine, glutamine, glutamic acid, histidine, isoleucine, leucine, lysine, hydroxylysine, methionine, phenylalanine, serine, threonine, tyrosine, valine, proline, 4-hydroxyproline, tryptophan, β-alanine, 1-amino-2-carboxycyclopentane, aminobenzoic acid, aminopyridinecarboxylic acid and amino acid represented by the following chemical formula (Ia2), and may or may not further have a substituent or a protecting group. Examples of the aforementioned substituent include the substituents exemplified for the aforementioned R^{a}, R^{b}, R^{c} and R^{d}. More specifically, for example, halogen, hydroxy, alkoxy, amino, carboxy, sulfo, nitro, carbamoyl, sulfamoyl, alkyl, alkenyl, alkynyl, haloalkyl, aryl, arylalkyl, alkylaryl, cycloalkyl, cycloalkenyl, cycloalkylalkyl, cyclylalkyl, hydroxyalkyl, alkoxyalkyl, aminoalkyl, silyl, silyloxyalkyl, pyrrolyl, imidazolyl, and the like can be mentioned. The aforementioned protecting group is the same as, for example, the protecting groups exemplified for the aforementioned R^{a}, R^{b}, R^{c} and R^{d}. When the amino acid or peptide of the aforementioned chemical formula (Ia) contains isomers such as optical isomer, geometric isomer, stereoisomer and the like, any isomer can be used.

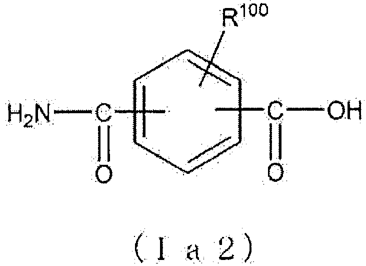

In the aforementioned chemical formula (Ia2), R¹⁰⁰ is an optional substituent and may or may not be present. When it is present, the number thereof may be one or more and, when it is present in plurality, they may be the same or different. Examples of the aforementioned optional substituent for R¹⁰⁰ include the substituents exemplified for the aforementioned R^{a}, R^{b}, R^{c} and R^{d}, more specifically, for example, halogen, hydroxy, alkoxy, amino, carboxy, sulfo, nitro, carbamoyl, sulfamoyl, alkyl, alkenyl, alkynyl, haloalkyl, aryl, arylalkyl, alkylaryl, cycloalkyl, cycloalkenyl, cycloalkylalkyl, cyclylalkyl, hydroxyalkyl, alkoxyalkyl, aminoalkyl, silyl, silyloxyalkyl, pyrrolyl, imidazolyl, and the like. The structure of the aforementioned chemical formula (Ia2) may be, for example, the following chemical formula (Ia3).

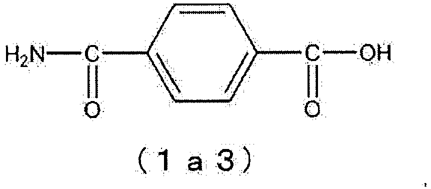

When the structure of the aforementioned chemical formula (Ia) is the aforementioned chemical formula (Ia2), the structure of the atomic group A in the aforementioned chemical formula (I) is represented by the following chemical formula (A2). R¹⁰⁰ in the following chemical formula (A2) is the same as that in the aforementioned chemical formula (Ia2). When the structure of the aforementioned chemical formula (Ia) is the aforementioned chemical formula (Ia3), the structure of the atomic group A in the aforementioned chemical formula (I) is represented by the following chemical formula (A2a).

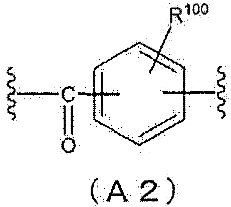

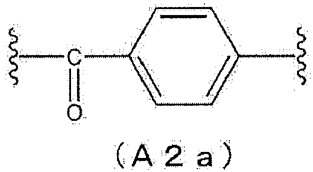

The structure of the aforementioned chemical formula (I) is, for example, the following chemical formulas (I-1) - (I-7), wherein n_{L} and m_{L} are the same as those in the aforementioned chemical formula (I).

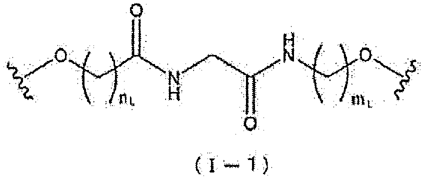

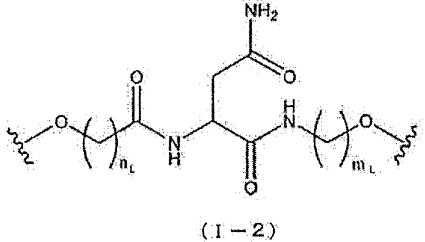

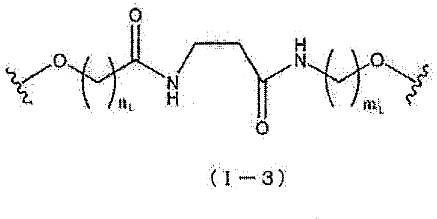

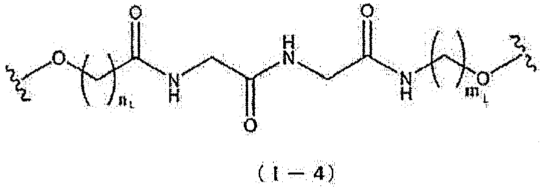

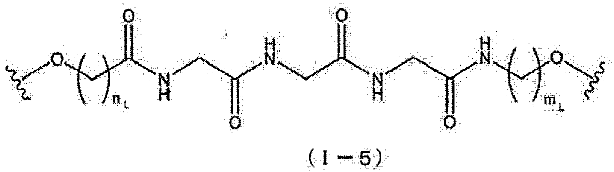

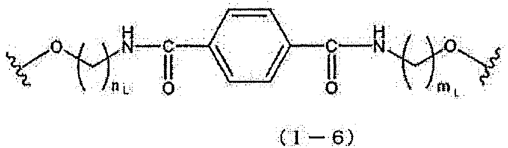

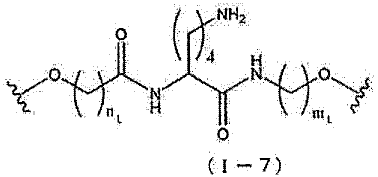

In the aforementioned chemical formulas (I-1) - (I-7), n_{L} and m_{L} are not particularly limited, and are as described above. Specific examples thereof include n_{L}=11 and m_{L}=12 or n_{L}=5 and m_{L}=4 in the aforementioned chemical formula (I-1), n_{L}=5 and m_{L}=4 in the aforementioned chemical formula (I-4), n_{L}=4 and m_{L}=4 in the aforementioned chemical formula (I-6), and n_{L}=5 and m_{L}=4 in the aforementioned chemical formula (1-7). The structures thereof are shown in the following chemical formulas (I-1a), (I-1b), (I-4a), (I-6a) and (I-7a). Among these, the formulas (I-1b), (I-4a), (I-6a) and (I-7a) are preferable.

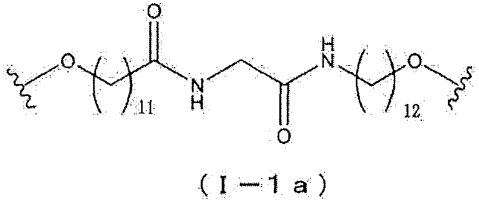

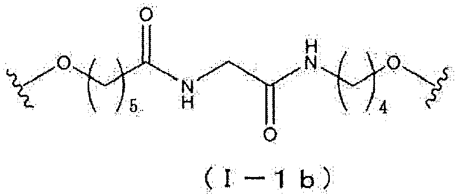

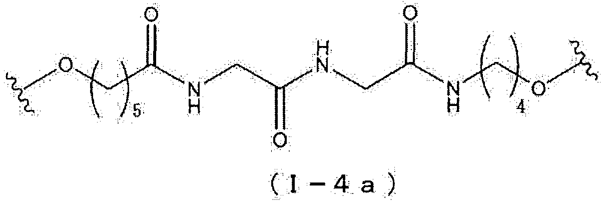

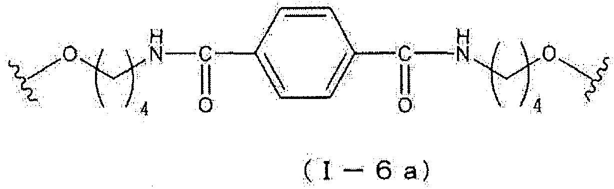

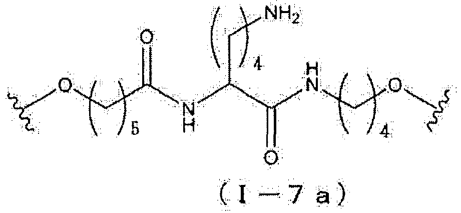

Alternatively, in the cyclic nucleic acid molecule of the present invention, the aforementioned linker region L is represented by the following formula (II):

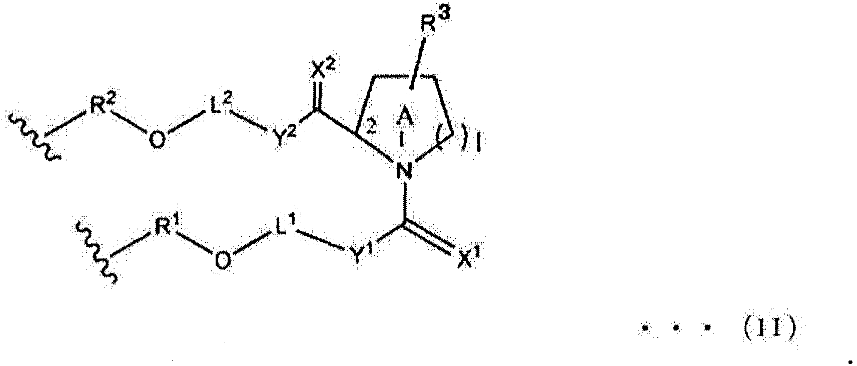

In the aforementioned formula (II),
X¹ and X² are each independently H₂, O, S or NH;
Y¹ and Y² are each independently a single bond, CH₂, NH, O or S;
R³ is a hydrogen atom or a substituent which is bonded to C-3, C-4, C-5 or C-6 on ring A,
L¹ is an alkylene chain having n_{L} atoms, and a hydrogen atom on an alkylene carbon atom may or may not be substituted by OH, OR^{a}, NH₂, NHR^{a}, NR^{a}R^{b}, SH or SR^{a}, or,
L¹ is a polyether chain obtained by substituting at least one carbon atom on the aforementioned alkylene chain with an oxygen atom,
provided that when Y¹ is NH, O or S, then an atom bound to Y¹ in L¹ is carbon, an atom bound to OR¹ in L¹ is carbon, and oxygen atoms are not adjacent to each other;
L² is an alkylene chain having m_{L} atoms, and a hydrogen atom on an alkylene carbon atom may or may not be substituted by OH, OR^{c}, NH₂, NHR^{c}, NR^{c}R^{d}, SH or SR^{c}, or
L² is a polyether chain obtained by substituting at least one carbon atom on the aforementioned alkylene chain with an oxygen atom,
provided that when Y² is NH, O or S, then an atom bound to Y² in L² is carbon, an atom bound to OR² in L² is carbon, and oxygen atoms are not adjacent to each other;
R^{a}, R^{b}, R^{c}, and R^{d} are each independently a substituent or a protecting group;
l is 1 or 2;
m_{L} is an integer of 0 - 30;
n_{L} is an integer of 0 - 30; and
in ring A, one carbon atom other than C-2 on the aforementioned ring A may be substituted by nitrogen, oxygen or sulfur, and may contain, in the aforementioned ring A, a carbon-carbon double bond or a carbon-nitrogen double bond,
R¹ and R² may or may not be present, and when they are present, R¹ and R² are each independently a nucleotide residue or the aforementioned structure (II), and
L in the aforementioned formula (A) is linked to the adjacent nucleotide residue via -OR¹- or -OR²- in the aforementioned formula (II).

In the aforementioned formula (II), for example, X¹ and X² are each independently H₂, O, S or NH. In the aforementioned formula (II), "X¹ is H₂" means that X¹ forms CH₂ (a methylene group) together with a carbon atom to which X¹ binds. The same applies to X².

In the aforementioned formula (II), Y¹ and Y² are each independently a single bond, CH₂, NH, O or S.

In the aforementioned formula (II), L¹ is an alkylene chain composed of atoms in the number of n_{L}. The hydrogen atom on the aforementioned alkylene carbon atom is optionally substituted or not substituted by, for example, OH, OR^{a}, NH₂, NHR^{a}, NR^{a}R^{b}, SH or SR^{a}. Alternatively, L¹ may be a polyether chain in which one or more carbon atoms of the aforementioned alkylene chain are substituted by an oxygen atom. The aforementioned polyether chain is, for example, polyethylene glycol. When Y¹ is NH, O or S, the atom of L¹ that binds to Y¹ is carbon, the atom of L¹ that binds to OR¹ is carbon, and the oxygen atoms are not adjacent to each other. That is, for example, when Y¹ is O, the oxygen atom is not adjacent to the oxygen atom for L¹, and the oxygen atom for OR¹ is not adjacent to the oxygen atom for L¹.

In the aforementioned formula (II), L² is an alkylene chain composed of atoms in the number of m_{L}. The hydrogen atom on the aforementioned alkylene carbon atom is optionally substituted or not substituted by, for example, OH, OR^{c}, NH₂, NHR^{c}, NR^{c}R^{d}, SH or SR^{c}. Alternatively, L² may be a polyether chain in which one or more carbon atoms of the aforementioned alkylene chain are substituted by an oxygen atom. When Y² is NH, O or S, the atom of L² that binds to Y² is carbon, the atom of L² that binds to OR² is carbon, and the oxygen atoms are not adjacent to each other. That is, for example, when Y² is O, the oxygen atom is not adjacent to the oxygen atom for L², and the oxygen atom for OR² is not adjacent to the oxygen atom for L².

The n_{L} for L¹ and m_{L} for L² are not particularly limited and the lower limit is, for example, 0 for each and the upper limit is not particularly limited, either. The n_{L} and m_{L} can be appropriately set, for example, according to the desired length of the aforementioned linker region (L). For example, n_{L} and m_{L} are each preferably 0 to 30, more preferably 0 to 20, further preferably 0 to 15, in view of production cost, yield and the like. The n_{L} and m_{L} may be the same (n_{L}=m_{L}) or different. The n_{L}+m_{L} is, for example, 0 to 30, preferably 0 to 20, more preferably 0 to 15.

For example, R^{a}, R^{b}, R^{c} and R^{d} are each independently a substituent or a protecting group. The aforementioned substituent and the aforementioned protecting group are, for example, the same as those mentioned above.

In the aforementioned formula (II), the hydrogen atoms are each independently optionally substituted by, for example, halogen such as Cl, Br, F, I and the like.

The aforementioned L in the aforementioned formula (A) binds to a phosphoric acid group of the adjacent nucleotide residue via -OR¹- or -OR²- in the aforementioned formula (II). Here, R¹ and R² may or may not be present. When R¹ and R² are present, R¹ and R² are each independently a nucleotide residue or the structure of the aforementioned formula (II). When R¹ and/or R² have/has a structure of the aforementioned formula (II), the aforementioned linker region (L) has a structure in which not less than two residues with the structure of the aforementioned formula (II) are linked. The structure of the aforementioned formula (II) may be contained in the number of, for example, 1, 2, 3 or 4. When plural structures mentioned above are contained, the structure of the aforementioned (II) may be, for example, directly linked or bonded via the aforementioned nucleotide residue(s).

In the aforementioned formula (II), 1 in ring A is 1 or 2. When l = 1, ring A is a 5-membered ring, for example, the aforementioned pyrrolidine skeleton. The aforementioned pyrrolidine skeleton is, for example, proline skeleton, prolinol skeleton or the like, and exemplified by the divalent structures thereof. When l = 2, ring A is a 6-membered ring, for example, the aforementioned piperidine skeleton. In ring A, one carbon atom other than C-2 on ring A may be substituted by nitrogen, oxygen or sulfur. Ring A may contain, in ring A, a carbon-carbon double bond or a carbon-nitrogen double bond. Ring A is, for example, L type or D type.

In the aforementioned formula (II), R³ is a hydrogen atom or substituent bonded to C-3, C-4, C-5 or C-6 on ring A. When R³ is the aforementioned substituent, substituent R³ may be one or more or may be absent. When R³ is present in plurality, they may be the same or different.

The substituent R³ is, for example, halogen, OH, OR⁴, NH₂, NHR⁴, NR⁴R⁵, SH, SR⁴, oxo group (=O) and the like.

R⁴ and R⁵ are, for example, each independently a substituent or a protecting group, and may be the same or different. Examples of the aforementioned substituent include halogen, alkyl, alkenyl, alkynyl, haloalkyl, aryl, heteroaryl, arylalkyl, cycloalkyl, cycloalkenyl, cycloalkylalkyl, cyclylalkyl, hydroxyalkyl, alkoxyalkyl, aminoalkyl, heterocyclylalkenyl, heterocyclylalkyl, heteroarylalkyl, silyl, silyloxyalkyl and the like. The same applies hereinafter. The substituent R³ may be selected from the substituents recited above.

The aforementioned protecting group is a functional group that inactivates, for example, a highly-reactive functional group. Examples of the protecting group include known protecting groups and the like. Regarding the aforementioned protecting group, for example, the description in the literature (J.F.W. McOmie, "Protecting Groups in Organic Chemistry", Prenum Press, London and New York, 1973) can be incorporated herein. The aforementioned protecting group is not particularly limited, and examples thereof include a tert-butyldimethylsilyl group (TBDMS), a bis(2-acetoxyethyloxy)methyl group (ACE), a triisopropylsilyloxymethyl group (TOM), a 1-(2-cyanoethoxy)ethyl group (CEE), a 2-cyanoethoxymethyl group (CEM), a tolylsulfonylethoxymethyl group (TEM), a dimethoxytrityl group (DMTr) and the like. When R³ is OR⁴, the aforementioned protecting group is not particularly limited, and examples thereof include a TBDMS group, an ACE group, a TOM group, a CEE group, a CEM group, a TEM group and the like. Other examples of the protecting group include silyl-containing groups. The same applies hereinafter.

Examples of the structure of the aforementioned formula (II) include the structures of the following formulas (II-1) to (II-9). In the following formulae, n_{L} and m_{L} are the same as in the aforementioned formula (II). In the following formulae, q_{L} is an integer of 0 - 10.

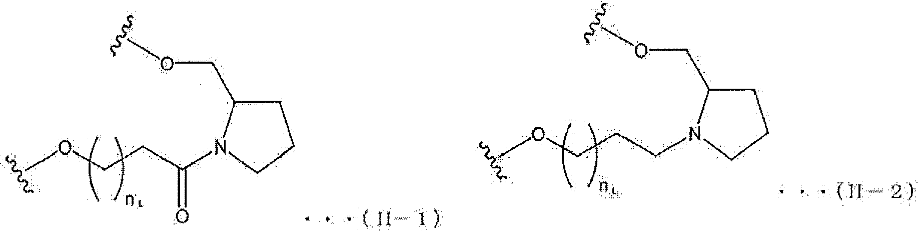

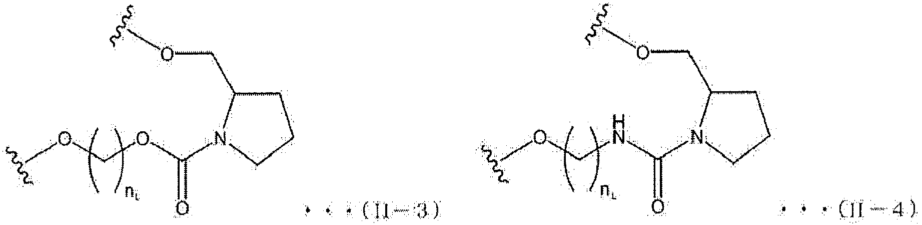

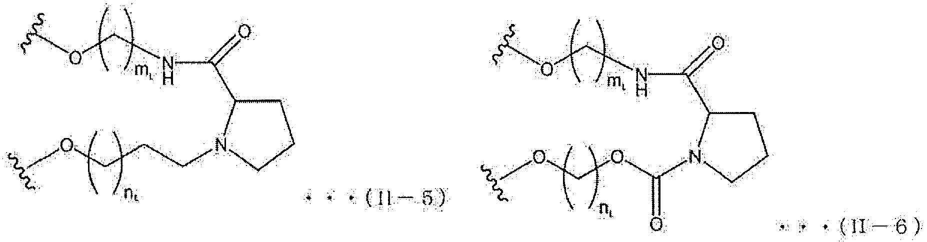

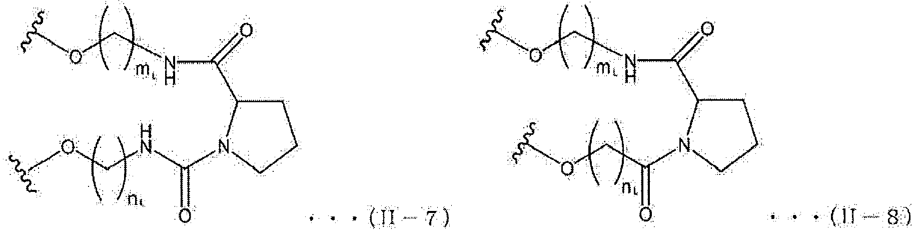

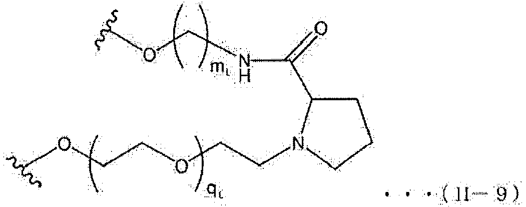

In the aforementioned formulas (II-1) to (II-9), n_{L}, m_{L} and q_{L} are not particularly limited, and are as described above. Specific examples thereof include n_{L}=8 in the aforementioned formula (II-1), n_{L}=3 in the aforementioned (II-2), n_{L}=4 or 8 in the aforementioned formula (II-3), n_{L}=7 or 8 in the aforementioned (II-4), n_{L}=3 and m_{L}=4 in the aforementioned formula (II-5), n_{L}=8 and m_{L}=4 in the aforementioned (II-6), n_{L}=8 and m_{L}=4 in the aforementioned formula (II-7), n_{L}=5 and m_{L}=4, n_{L}=7 and m_{L}=6, n_{L}=9 and m_{L}=8 or n_{L}=11 and m_{L}=10 in the aforementioned (II-8), and q_{L}=1 and m_{L}=4 in the aforementioned formula (II-9). One embodiment (n_{L}=8) of the aforementioned formula (II-4) is shown in the following formula (II-4a), and one embodiment (n_{L}=5, m_{L}=4) of the aforementioned formula (II-8) is shown in the following formula (II-8a). Among these, the formula (II-8a) is preferable.

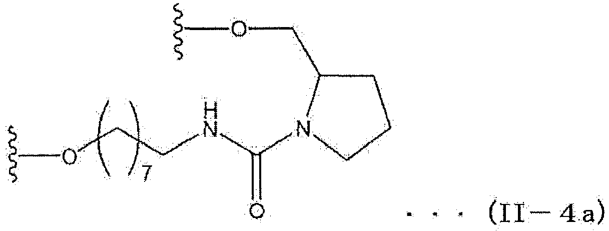

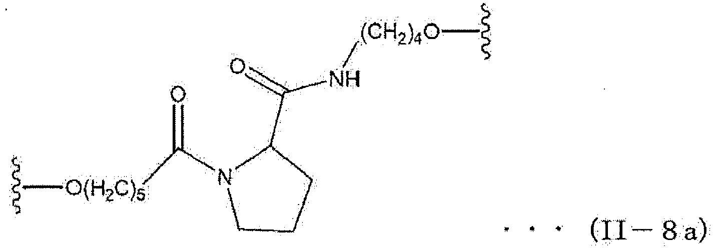

In the present invention, the "alkyl" encompasses, for example, straight-chain and branched alkyl groups. The number of carbon atoms in the aforementioned alkyl is not particularly limited, and is, for example, 1 to 30, preferably 1 to 6 or 1 to 4. Examples of the aforementioned alkyl group include: methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, n-nonyl, and n-decyl and the like. Among them, for example, methyl, ethyl, n-propyl, isopropyl, n-butyl, isobutyl, sec-butyl, tert-butyl, n-pentyl, isopentyl, neopentyl, n-hexyl, isohexyl, and the like are preferable.

In the present invention, the "alkenyl" encompasses, for example, straight-chain and branched alkenyls. Examples of the aforementioned alkenyl include the aforementioned alkyls having one or more double bonds and the like. The number of carbon atoms in the aforementioned alkenyl is not particularly limited, and is, for example, the same as that in the aforementioned alkyl, preferably 2 to 8. Examples of the aforementioned alkenyl include vinyl, 1-propenyl, 2-propenyl, 1-butenyl, 2-butenyl, 3-butenyl, 1,3-butadienyl, 3-methyl-2-butenyl and the like.

In the present invention, the "alkynyl" encompasses, for example, straight-chain and branched alkynyls. Examples of the aforementioned alkynyl include the aforementioned alkyls having one or more triple bonds and the like. The number of carbon atoms in the aforementioned alkynyl is not particularly limited, and is, for example, the same as that in the aforementioned alkyl, preferably 2 to 8. Examples of the aforementioned alkynyl include ethynyl, propynyl, butynyl and the like. The aforementioned alkynyl may further include, for example, one or more double bonds.

In the present invention, the "aryl" encompasses, for example, monocyclic aromatic hydrocarbon groups and polycyclic aromatic hydrocarbon groups. Examples of the aforementioned monocyclic aromatic hydrocarbon group include phenyl and the like. Examples of the aforementioned polycyclic aromatic hydrocarbon group include 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl and the like. Among them, for example, phenyl, naphthyls such as 1-naphthyl and 2-naphthyl, and the like are preferable.

In the present invention, the "heteroaryl" encompasses, for example, monocyclic aromatic heterocyclic groups and condensed aromatic heterocyclic groups. Examples of the aforementioned heteroaryl include furyls (e.g., 2-furyl, 3-furyl), thienyls (e.g., 2-thienyl, 3-thienyl), pyrrolyls (e.g., 1-pyrrolyl, 2-pyrrolyl, 3-pyrrolyl), imidazolyls (e.g., 1-imidazolyl, 2-imidazolyl, 4-imidazolyl), pyrazolyls (e.g., 1-pyrazolyl, 3-pyrazolyl, 4-pyrazolyl), triazolyls (e.g., 1,2,4-triazol-1-yl, 1,2,4-triazol-3-yl, 1,2,4-triazol-4-yl), tetrazolyls (e.g., 1-tetrazolyl, 2-tetrazolyl, 5-tetrazolyl), oxazolyls (e.g., 2-oxazolyl, 4-oxazolyl, 5-oxazolyl), isoxazolyls (e.g., 3-isoxazolyl, 4-isoxazolyl, 5-isoxazolyl), thiazolyls (e.g., 2-thiazolyl, 4-thiazolyl, 5-thiazolyl), thiadiazolyls, isothiazolyls (e.g., 3-isothiazolyl, 4-isothiazolyl, 5-isothiazolyl), pyridyls (e.g., 2-pyridyl, 3-pyridyl, 4-pyridyl), pyridazinyls (e.g., 3-pyridazinyl, 4-pyridazinyl), pyrimidinyls (e.g., 2-pyrimidinyl, 4-pyrimidinyl, 5-pyrimidinyl), furazanyls (e.g., 3-furazanyl), pyrazinyls (e.g., 2-pyrazinyl), oxadiazolyls (e.g., 1,3,4-oxadiazol-2-yl), benzofuryls (e.g., 2-benzo[b]furyl, 3-benzo[b]furyl, 4-benzo[b]furyl, 5-benzo[b]furyl, 6-benzo[b]furyl, 7-benzo[b]furyl), benzothienyls (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzimidazolyls (e.g., 1-benzimidazolyl, 2-benzimidazolyl, 4-benzimidazolyl, 5-benzimidazolyl), dibenzofuryls, benzoxazolyls, benzothiazolyls, quinoxalinyls (e.g., 2-quinoxalinyl, 5-quinoxalinyl, 6-quinoxalinyl), cinnolinyls (e.g., 3-cinnolinyl, 4-cinnolinyl, 5-cinnolinyl, 6-cinnolinyl, 7-cinnolinyl, 8-cinnolinyl), quinazolinyls (e.g., 2-quinazolinyl, 4-quinazolinyl, 5-quinazolinyl, 6-quinazolinyl, 7-quinazolinyl, 8-quinazolinyl), quinolyls (e.g., 2-quinolyl, 3-quinolyl, 4-quinolyl, 5-quinolyl, 6-quinolyl, 7-quinolyl, 8-quinolyl), phthalazinyls (e.g., 1-phthalazinyl, 5-phthalazinyl, 6-phthalazinyl), isoquinolyls (e.g., 1-isoquinolyl, 3-isoquinolyl, 4-isoquinolyl, 5-isoquinolyl, 6-isoquinolyl, 7-isoquinolyl, 8-isoquinolyl), puryls, pteridinyls (e.g., 2-pteridinyl, 4-pteridinyl, 6-pteridinyl, 7-pteridinyl), carbazolyls, phenanthridinyls, acridinyls (e.g., 1-acridinyl, 2-acridinyl, 3-acridinyl, 4-acridinyl, 9-acridinyl), indolyls (e.g., 1-indolyl, 2-indolyl, 3-indolyl, 4-indolyl, 5-indolyl, 6-indolyl, 7-indolyl), isoindolyls, phenazinyls (e.g., 1-phenazinyl, 2-phenazinyl), and phenothiazinyls (e.g., 1-phenothiazinyl, 2-phenothiazinyl, 3-phenothiazinyl, 4-phenothiazinyl) and the like.

In the present invention, for example, the term "cycloalkyl" refers to cyclic saturated hydrocarbon groups and the number of carbon atoms in the cycloalkyl is, for example, 3 to 15. Examples of the aforementioned cycloalkyl include cyclopropyl, cyclobutyl, cyclopentyl, cyclohexyl, cycloheptyl, cyclooctyl, bridged cyclic hydrocarbon groups, spiro hydrocarbon groups and the like. Among them, cyclopropyl, cyclobutyl, cyclopentyl, cyclohexyl, bridged cyclic hydrocarbon groups, and the like are preferable.

In the present invention, examples of the "bridged cyclic hydrocarbon groups" include bicyclo[2.1.0]pentyl, bicyclo[2.2.1]heptyl, bicyclo[2.2.2]octyl, and bicyclo[3.2.1]octyl, tricyclo[2.2.1.0]heptyl, bicyclo[3.3.1]nonane, 1-adamantyl, 2-adamantyl and the like.

In the present invention, examples of the "spiro hydrocarbon groups" include spiro[3.4]octyl and the like.

In the present invention, the term "cycloalkenyl" encompasses, for example, unsaturated cyclic aliphatic hydrocarbon groups and the number of carbon atoms in the cycloalkenyl is, for example, 3 to 7. Examples of the aforementioned group include cyclopropenyl, cyclobutenyl, cyclopentenyl, cyclohexenyl, cycloheptenyl and the like. Among them, cyclopropenyl, cyclobutenyl, cyclopentenyl, cyclohexenyl, and the like are preferable. The aforementioned term "cycloalkenyl" also encompasses, for example, bridged cyclic hydrocarbon groups and spiro hydrocarbon groups having an unsaturated bond in their rings.

In the present invention, examples of the "arylalkyl" include benzyl, 2-phenethyl, naphthalenylmethyl and the like. Examples of the "cycloalkylalkyl" and "cyclylalkyl" include cyclohexylmethyl adamantylmethyl and the like. Examples of the "hydroxyalkyl" include hydroxymethyl 2-hydroxyethyl and the like.

In the present invention, the "alkoxy" encompasses, for example, the aforementioned alkyl-O-group and examples thereof include methoxy, ethoxy, n-propoxy, isopropoxy, n-butoxy and the like. Examples of the "alkoxyalkyl" include methoxymethyl and the like. Examples of the "aminoalkyl" include 2-aminoethyl and the like.

In the present invention, examples of the "heterocyclyl" include 1-pyrrolinyl, 2-pyrrolinyl, 3-pyrrolinyl, 1-pyrrolidinyl, 2-pyrrolidinyl, 3-pyrrolidinyl, pyrrolidinone, 1-imidazolinyl, 2-imidazolinyl, 4-imidazolinyl, 1-imidazolidinyl, 2-imidazolidinyl, 4-imidazolidinyl, imidazolidinone, 1-pyrazolinyl, 3-pyrazolinyl, 4-pyrazolinyl, 1-pyrazolidinyl, 3-pyrazolidinyl, 4-pyrazolidinyl, piperidinone, piperidino, 2-piperidinyl, 3-piperidinyl, 4-piperidinyl, 1-piperazinyl, 2-piperazinyl, piperazinone, 2-morpholinyl, 3-morpholinyl, morpholino, tetrahydropyranyl, tetrahydrofuranyl and the like.

In the present invention, examples of the "heterocyclylalkyl" include piperidinylmethyl, piperazinylmethyl and the like. Examples of the "heterocyclylalkenyl" include 2-piperidinylethenyl and the like. Examples of the "heteroarylalkyl" include pyridylmethyl, quinolin-3-ylmethyl and the like.

In the present invention, the "silyl" encompasses groups represented by the formula R₃Si-, where R independently can be selected from the aforementioned alkyls, aryls, and cycloalkyls. Examples of the silyl include a trimethylsilyl group, a tert-butyldimethylsilyl group and the like. Examples of the "silyloxy" include a trimethylsilyloxy group and the like. Examples of the "silyloxyalkyl" include trimethylsilyloxymethyl and the like.

In the present invention, examples of the "alkylene" include methylene, ethylene, propylene and the like.

In the present invention, the above-described various groups may be substituted. Examples of the aforementioned substituent include hydroxy, carboxy, sulfo, halogen, alkyl halide (haloalkyl, e.g., CF₃, CH₂CF₃, CH₂CCl₃), nitro, nitroso, cyano, alkyl (e.g., methyl, ethyl, isopropyl, tert-butyl), alkenyl (e.g., vinyl), alkynyl (e.g., ethynyl), cycloalkyl (e.g., cyclopropyl, adamantyl), cycloalkylalkyl (e.g., cyclohexylmethyl, adamantylmethyl), cycloalkenyl (e.g., cyclopropenyl), cyclylalkyl, hydroxyalkyl (e.g., hydroxymethyl, hydroxyethyl), alkoxyalkyl (e.g., methoxymethyl, ethoxymethyl, ethoxyethyl), aryl (e.g., phenyl, naphthyl), arylalkyl (e.g., benzyl, phenethyl), alkylaryl (e.g., p-methylphenyl), heteroaryl (e.g., pyridyl, furyl), heteroarylalkyl (e.g., pyridylmethyl), heterocyclyl (e.g., piperidyl), heterocyclylalkenyl, heterocyclylalkyl (e.g., morpholylmethyl), alkoxy (e.g., methoxy, ethoxy, propoxy, butoxy), halogenated alkoxy (e.g., OCF₃), alkenyloxy (e.g., vinyloxy, allyloxy), aryloxy (e.g., phenyloxy), alkyloxycarbonyl (e.g., methoxycarbonyl, ethoxycarbonyl, tert-butoxycarbonyl), arylalkyloxy (e.g., benzyloxy), amino [alkylamino (e.g., methylamino, ethylamino, dimethylamino), acylamino (e.g., acetylamino, benzoylamino), arylalkylamino (e.g., benzylamino, tritylamino), hydroxyamino], aminoalkyl (e.g., aminomethyl), alkylaminoalkyl (e.g., diethylaminomethyl), carbamoyl, sulfamoyl, oxo, silyl, silyloxyalkyl and the like.

Alternatively, in the cyclic nucleic acid molecule of the present invention, the aforementioned linker region (L) is shown by any of the following formulas (III-1) - (III-3). In each formula, n_{L} and m_{L} are the same as those in the aforementioned chemical formula (I).

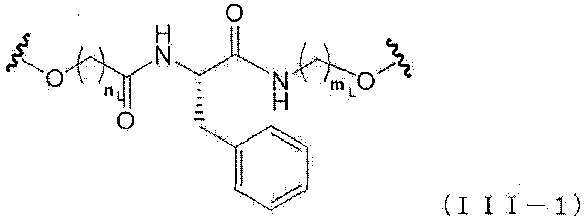

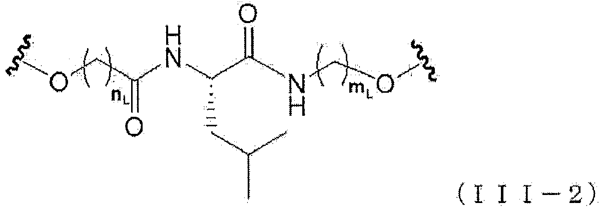

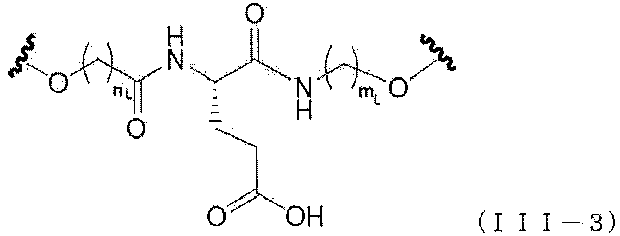

In the aforementioned chemical formulas (III-1) - (III-3), n_{L} and m_{L} are not particularly limited, and are as described above. Specific examples thereof include n_{L}=5 and m_{L}=4 in the aforementioned chemical formula (III-1), n_{L}=5 and m_{L}=4 in the aforementioned chemical formula (III-2), and n_{L}=5 and m_{L}=4 in the aforementioned chemical formula (III-3). The structures thereof are shown by the following chemical formulas (III-1a), (III-2a) and (III-3a). Preferred are the formulas (III-1a), (III-2a) and (III-3a).

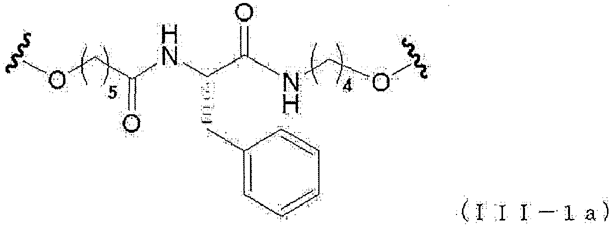

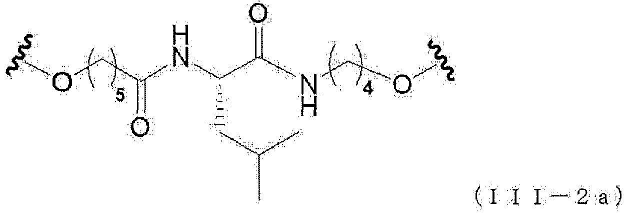

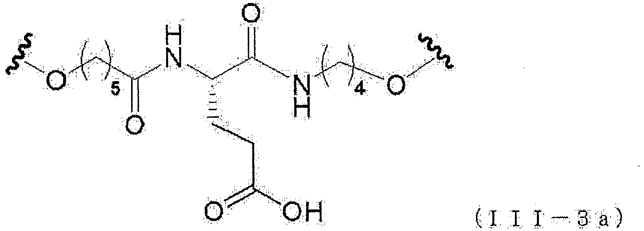

The cyclic nucleic acid molecule of the present invention may include, for example, a labeling substance, and may be labeled with the aforementioned labeling substance. The aforementioned labeling substance is not particularly limited, and may be, for example, a fluorescent substance, a dye, an isotope or the like. Examples of the aforementioned labeling substance include: fluorophores such as pyrene, TAMRA, fluorescein, a Cy3 dye, a Cy5 dye and the like. Examples of the aforementioned dye include Alexa dyes such as Alexa 488 and the like. Examples of the aforementioned isotope include stable isotopes and radioisotopes. Among them, stable isotopes are preferable. Moreover, for example, the aforementioned stable isotope does not change the physical properties of a compound labeled therewith and thus has an excellent property as a tracer. The aforementioned stable isotope is not particularly limited, and examples thereof include ²H, ¹³C, ¹⁵N, ¹⁷O, ¹⁸O, ³³S, ³⁴S and ³⁶S.

As described above, the cyclic nucleic acid molecule of the present invention can inhibit the aforementioned expression of a target gene. Thus, the cyclic nucleic acid molecule of the present invention can be used, for example, as a therapeutic agent for treating a disease caused by a gene. When the cyclic nucleic acid molecule of the present invention has a guide strand sequence of a gene that inhibits expression of a gene relating the aforementioned disease, for example, it is possible to treat the aforementioned disease by inhibiting the expression of the aforementioned target gene. In the present invention, the "treatment" encompasses prevention of the aforementioned diseases; improvement of the diseases; and improvement in prognosis, for example, and it can mean any of them. The aforementioned disease is not particularly limited and, for example, the aforementioned sequence that inhibits expression can be set appropriately according to the object disease. Examples of the aforementioned disease include cancer such as breast cancer, lung cancer, stomach cancer, colorectal cancer, liver cancer, pancreatic cancer, esophageal cancer, prostate cancer, gall bladder cancer, uterine body cancer, uterus cervix cancer, ovarian cancer, osteosarcoma, leukemia and the like, and diseases such as lung fibrosis, hepatic fibrosis and the like.

The method of using the cyclic nucleic acid molecule of the present invention is not particularly limited. For example, the aforementioned cyclic nucleic acid molecule may be administered to a subject having the aforementioned target gene.

Examples of the aforementioned subject include cells, tissues and organs. Examples of the aforementioned subject also include humans, nonhuman animals such as nonhuman mammals excluding humans. The aforementioned administration may be performed, for example, in vivo or in vitro. The aforementioned cells are not particularly limited, and examples thereof include: various cultured cells such as HeLa cells, 293 cells, NIH3T3 cells, COS cells and the like; stem cells such as ES cells, hematopoietic stem cells and the like; and cells isolated from living organisms, such as primary cultured cells and the like.

In the present invention, the aforementioned target gene whose expression is to be inhibited is not particularly limited, and any desired gene can be set to the target gene. As mentioned above, the aforementioned cyclic nucleic acid molecule can be selected according to the kind of the aforementioned target gene.

As to the use of the cyclic nucleic acid molecule of the present invention, the following description regarding the composition, the expression inhibitory method, the treatment method, and the like according to the present invention to be describe below can be referred to.

Since the cyclic nucleic acid molecule of the present invention can inhibit the expression of a target gene as described above, for example, it is useful as a pharmaceutical product, a diagnostic agent, an agricultural chemical, and a tool for conducting research on agriculture, medical science, life science, and the like.

As the synthesis method of the cyclic nucleic acid molecule of the present invention, for example, a production method conventionally known in the protein chemistry can be utilized. Examples of the production method of the straight, single stranded nucleic acid molecule necessary for the aforementioned synthesis include synthesis methods according to genetic engineering procedures, chemical synthesis methods and the like. Examples of the genetic engineering procedures include: synthesis methods utilizing in vitro transcription; methods using a vector; methods carried out using a PCR cassette and the like. The aforementioned vector is not particularly limited, and examples thereof include non-virus vectors such as plasmid and the like, and virus vectors and the like. The aforementioned chemical synthesis methods are not particularly limited, and examples thereof include a phosphoramidite method, an H-phosphonate method and the like. The aforementioned chemical synthesis methods can be carried out, for example, using a commercially available automated nucleic acid synthesizer. In the aforementioned chemical synthesis methods, an amidite is generally used. The aforementioned amidite is not particularly limited. Examples of commercially available amidites include RNA Phosphoramidites (2'-O-TBDMSi, trade name, Samchully Pharm. Co., Ltd.), ACE amidite, TOM amidite, CEE amidite, CEM amidite, TEM amidite and the like.

The thus-obtained single-stranded nucleic acid molecule can be subjected to a cyclization reaction using, for example, a compound having a disulfide bond to link the 5'-terminal and 3'-terminal to obtain a cyclic nucleic acid molecule. As the cyclization reaction, various reactions generally performed in the pertinent field can be utilized. For example, a method using a homobifunctional crosslinking agent such as dithiobis(succinimidylpropionate) (DSP), dithiobis(succinimidyloctanoate) (DSO) can be mentioned.

Specifically, a single-stranded nucleic acid molecule obtained by the solid phase synthesis method (containing amino group at 5', 3'-terminals) is dissolved in, for example, phosphate buffer, and a homobifunctional crosslinking agent (about 10 equivalents) is added and the mixture is reacted for several hours at 25°C. The single-stranded nucleic acid molecule as the starting material almost disappears and the reaction mixture is purified by reversed-phase chromatography, whereby the object compound can be obtained in a high yield.

### (2) Composition

The expression inhibitory composition according to the present invention is, as described above, a composition for inhibiting the expression of a target gene, characteristically containing the aforementioned cyclic nucleic acid molecule of the present invention. The composition of the present invention is characterized in that it contains the aforementioned cyclic nucleic acid molecule of the present invention, and other configurations are by no means limited. The expression inhibitory composition of the present invention can also be referred to, for example, as an expression inhibitory reagent.

According to the present invention, for example, by administering to a subject in which the aforementioned target gene is present, it is possible to inhibit the expression of the aforementioned target gene.

Furthermore, as described above, the pharmaceutical composition according to the present invention characteristically contains the aforementioned cyclic nucleic acid molecule of the present invention. The composition of the present invention is characterized in that it contains the aforementioned cyclic nucleic acid molecule of the present invention, and other configurations are by no means limited. The pharmaceutical composition of the present invention can also be referred to, for example, as a pharmaceutical product.

According to the present invention, for example, administration to a patient with a disease caused by a gene can inhibit the expression of the aforementioned gene, thereby treating the aforementioned disease. In the present invention, the "treatment" encompasses, as mentioned above, prevention of the aforementioned diseases; improvement of the diseases; and improvement in prognosis, for example, and it can mean any of them.

In the present invention, a disease to be treated is not particularly limited, and examples thereof include diseases caused by the expression of genes. Depending on the kind of the aforementioned disease, a gene that causes the disease may be set as the aforementioned target gene, and further, depending on the aforementioned target gene, a guide strand sequence of the aforementioned cyclic nucleic acid molecule may be selected.

The method of using the expression inhibitory composition and the pharmaceutical composition according to the present invention (hereinafter, both the compositions simply are referred to as "the compositions") are not particularly limited, and examples thereof include administering the aforementioned cyclic nucleic acid molecule to a subject having the aforementioned target gene.

Examples of the aforementioned subject include cells, tissues, and organs. Examples of the aforementioned subject also include humans, nonhuman animals such as nonhuman mammals excluding humans. The aforementioned administration may be performed, for example, in vivo or in vitro. The aforementioned cells are not particularly limited, and examples thereof include: various cultured cells such as HeLa cells, 293 cells, NIH3T3 cells, COS cells and the like; stem cells such as ES cells, hematopoietic stem cells and the like; and cells isolated from living organisms, such as primary cultured cells and the like.

The aforementioned administration method is not particularly limited, and can be determined, for example, as appropriate depending on the subject. When the aforementioned subject is a cultured cell, the administration method may be, for example, a method using a transfection reagent, an electroporation method or the like.

For example, each of the compositions of the present invention may contain only the cyclic nucleic acid molecule of the present invention or further may contain an additive(s). The aforementioned additive is not particularly limited, and is preferably, for example, a pharmaceutically acceptable additive. The kind of the aforementioned additive is not particularly limited, and can be selected as appropriate depending on, for example, the kind of the subject.

In the composition of the present invention, for example, the aforementioned cyclic nucleic acid molecule may form a complex with the aforementioned additive. The aforementioned additive can also be referred to, for example, as a complexing agent. The aforementioned complex formation allows, for example, the aforementioned cyclic nucleic acid molecule to be delivered efficiently.

The aforementioned complexing agent is not particularly limited, and examples thereof include polymers, cyclodextrins, adamantine and the like. Examples of the aforementioned cyclodextrins include linear cyclodextrin copolymers, linear oxidized cyclodextrin copolymers and the like.

Other examples of the aforementioned additive include a carrier, a binding substance that binds to a target cell, a condensing agent, a fusogenic agent, an excipient and the like.

### (3) Expression inhibitory method

The expression inhibitory method according to the present invention is, as described above, a method for inhibiting the expression of a target gene, in which the aforementioned cyclic nucleic acid molecule of the present invention is characteristically used. The expression inhibitory method of the present invention is characterized in that the aforementioned cyclic nucleic acid molecule of the present invention is used therein, and other steps and conditions are by no means limited.

In the expression inhibitory method of the present invention, the mechanism by which the aforementioned target gene expression is inhibited is not particularly limited, and examples thereof include inhibition of the expression by cyclic nucleic acid molecule.

The expression inhibitory method of the present invention includes, for example, the step of administering the aforementioned cyclic nucleic acid molecule to a subject in which the aforementioned target gene is present. By the aforementioned administration step, for example, the aforementioned cyclic nucleic acid molecule is brought into contact with the aforementioned subject. Examples of the aforementioned subject include cells, tissues, and organs. Examples of the aforementioned subject also include humans, nonhuman animals such as nonhuman mammals excluding humans. The aforementioned administration may be performed, for example, in vivo or in vitro.

In the expression inhibitory method of the present invention, for example, the aforementioned cyclic nucleic acid molecule alone may be administered or the aforementioned composition of the present invention containing the aforementioned cyclic nucleic acid molecule may be administered. The aforementioned administration method is not particularly limited and, for example, can be selected as appropriate depending on the kind of the subject.

### (4) Treatment method

As described above, the method for treating a disease according to the present invention includes the step of administering the aforementioned cyclic nucleic acid molecule of the present invention to a patient, and is characterized in that the aforementioned guide strand sequence in the aforementioned cyclic nucleic acid molecule is the guide strand sequence of a cyclic nucleic acid molecule that inhibits expression of a gene relating to the aforementioned disease. The treatment method of the present invention is characterized by the use of the aforementioned cyclic nucleic acid molecule of the present invention, and other steps and conditions are by no means limited.

The aforementioned expression inhibitory method of the present invention also applies to, for example, the treatment method of the present invention. The aforementioned administration method is not particularly limited and may be, for example, any of oral administration and parenteral administration.

### (5) Use of cyclic nucleic acid molecule

The use according to the present invention is the use of the aforementioned cyclic nucleic acid molecule of the present invention for the aforementioned inhibition of the expression of a target gene.

The cyclic nucleic acid molecule according to the present invention is a cyclic nucleic acid for use in the treatment of a disease, and is characterized in that the aforementioned guide strand sequence in the aforementioned cyclic nucleic acid molecule is the guide strand sequence of a cyclic nucleic acid molecule that inhibits expression of a gene relating to the aforementioned disease.

In the following, the present invention will be described in detail with reference to examples and the like. It is to be noted, however, the present invention is by no means limited thereto.

### [Examples]

### Production Example: Synthesis of cyclic nucleic acid molecule

### Synthesis of single-stranded nucleic acid molecule

The single-stranded nucleic acid molecule shown below was synthesized based on the phosphoramidite method by nucleic acid synthesizers (trade name ABI Expedite (trademark registration) 8909 Nucleic Acid Synthesis System, Applied Biosystems and trade name ABI 3900 DNA Synthesizer, Applied Biosystems). Solid phase synthesis was performed using EMM amidite (WO 2013/027843) as RNA amidite from the 3'-side. L-proline amidite (Japanese Patent 4965745), glycine amidite (Japanese Patent 5876890) and L-lysine amidite (Japanese Patent 5876890) were used for the linker region. The deprotection of the aforementioned amidite was performed by a conventional method.

The following Phthalamido C-6 Linker lcaa CPG (ChemGenes) was used for the 3'-terminal of the single-stranded nucleic acid molecule of the present invention.

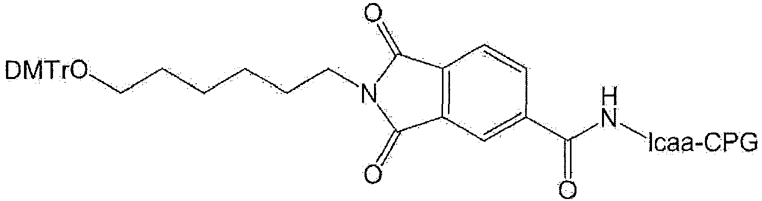

The following Tfa-NH-C6 amidite was used for the 5'-terminal of the single-stranded nucleic acid molecule of the present invention. Tfa-NH-C6 amidite (2.6 g) was synthesized by reacting 6-amino-1-hexanol (1 g) and ethyl trifluoroacetate (1.6 g) in ethanol, drying by evaporating the solvent and phosphoramiditing by a conventional method. Commercially available Tfa-NH-C6 amidite may also be used.

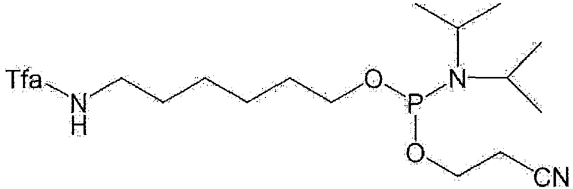

L-proline linker (hereinafter P or PA), glycine linker (hereinafter Gly) and L-lysine linker (hereinafter K or KA) of the following structures were used for the linker region (L) of the single-stranded nucleic acid molecule of the present invention.

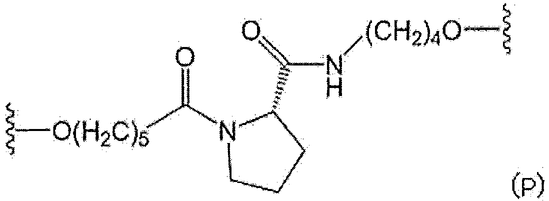

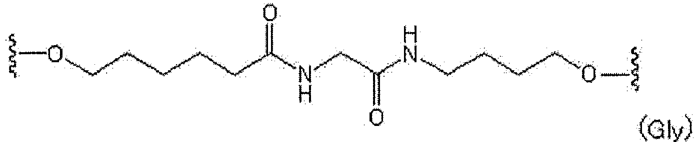

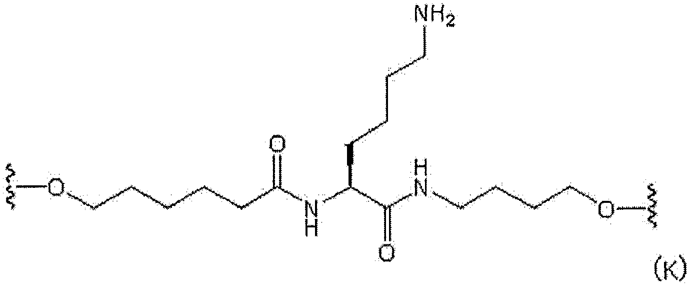

The obtained single-stranded nucleic acid molecule was purified by HPLC and used for the cyclization reaction in the next step. PA-0028 to PA-0033 were used unpurified for the next step. Table 1 shows the names and base sequences of the prepared single-stranded nucleic acid molecules. In the sequences, uppercase letters show RNA and lowercase letters show DNA (ChemGenes). The underlined bold letters show nucleotide containing 2'-O-methylribose (SamchullyPharm).

**[Table 1-1]**

| name | base sequence | SEQ ID NO: | Example |
|---|---|---|---|
| PA-0001 | 5′-GCAGAGUACACACAGCAUAUACC-P- GGUAUAUGCUGUGUGUACUCUGCUU-3′ | No.5 | 1,2 |
| PA-0002 | 5′-AAGCAGAGUACACACAGCAUAUACC-P- GGUAUAUGCUGUGUGUACUCUGCUU-3′ | No.6 | 3 |
| PA-0003 | 5′-AAGCAGAGUACACACAGCAtAtACC-P- GGtAUAUGCUGUGUGUACUCUGCUU-3′ | No.7 | 4 |
| PA-0004 | 5′-AAGCAGAGUACACACAGCAtatacc-P- ggtAUAUGCUGUGUGUACUCUGCUU-3′ | No.8 | 5 |
| PA-0005 | 5'-AAGCAGAGUACACACAGCAtatacc-P- gGUAUAUGCUGUGUGUACUCUGCUU-3' | No.9 | 6 |
| PA-0006 | 5′-AAGCAGAGUACACACAGCAUAtacc-P- gGUAUAUGCUGUGUGUACUCUGCUU-3′ | No.10 | 7 |
| PA-0007 | 5′-AAGCAGAGUACACACAGCAUatacc-P- ggtatAUGCUGUGUGUACUCUGCUU-3′ | No.11 | 8 |
| PA-0008 | 5′-AAGCAGAGUACACACAGCAUAUACC-P- GGUAUAUGCUGUGUGUACUCUGCUU-3′ | No.12 | 9 |
| PA-0009 | 5′-AAGCAGAGUACACACAGCAUAUACC-P- GGUAUAUGCUGUGUGUACUCUGCUU-3′ | No.13 | 10 |
| PA-0010 | 5′-AAGCAGAGUACACACAGCAUAUACC-P- GGUAUAUGCUGUGUGUACUCUGCUU-3′ | No.14 | 11 |
| PA-0011 | 5′-AACCAUGAGAAGUAUGACAACAGCC-P- GGCUGUUGUCAUACUUCUCAUGGUU-3′ | No.15 | 12 |
| PA-0012 | 5'-AACCAUGAGAAGUAUGACAacagcc-P- ggcUGUUGUCAUACUUCUCAUGGUU-3' | No.16 | 13 |
| PA-0013 | 5′-AAGCAGAGUACACACAGCAUAUACC-P- GGUAUAUGCUGUGUGUACUCUGCUU-3′ | No.17 | 14 |
| PA-0014 | 5'-AAGCAGAGUACACACAGCAUAUACC-P- GGUAUAUGCUGUGUGUACUCUGCUU-3' | No.18 | 15 |
| PA-0015 | 5′-AAUAGCACCAUUUGAAAUCAGUGUUCC- P-GGAACACUGAUUUCAAAUGGUGCUAUU-3′ | No.19 | 16 |
| PA-0016 | 5′-AAUAGCACCAUUUGAAAUCAGtgttcc- P-ggaACACUGAUUUCAAAUGGUGCUAUU-3′ | No.20 | 17 |
| PA-0017 | 5′-AAGCAGAGUACACACAGCAUAUacc-P- ggtAUAUGCUGUGUGUACUCUGCUU-3′ | No.21 | 18 |

**[Table 1-2]**

| | | | |
|---|---|---|---|
| PA-0018 | 5′-AAGCAGAGUACACACAGCAtatacc-P- ggtAUAUGCUGUGUGUACUCUGCtt-3′ | No.22 | 19 |
| PA-0019 | 5′-ggtAUAUGCUGUGUGUACUCUGCUU-P- AAGCAGAGUACACACAGCAtatacc-3′ | No.23 | 20 |
| PA-0020 | 5′-AAGCAGAGUACACACAgcatatacc-P- ggtAUAUGCUGUGUGUACUCUGCUU-3′ | No.24 | 21 |
| PA-0021 | 5′-AAGCAGAGUACACACAGCAtatacc-P- ggtaUAUGCUGUGUGUACUCUGCUU-3′ | No.25 | 22 |
| PA-0024 | 5'-AAGCAGAGUACACACAGCAUAUACC-P- GGUAUAUGCUGUGUGUACUCUGCUU-3' | No.26 | 23 |
| PA-0025 | 5′-aaGCAGAGUACACACAGCAtatacc-P- ggtAUAUGCUGUGUGUACUCUGCtt-3′ | No.27 | 24 |
| PA-0026 | 5′-AAGCAGAGUACACACAGCAUAUACC-P- GGUAUAUGCUGUGUGUACUCUGCUU-3′ | No.28 | 25 |
| PA-0027 | 5′-AAGCAGAGUACACACAGCAtatacc-P- ggtAUaUGCUGUGUGUACUCUGCtt-3′ | No.29 | 26 |
| PA-0028 | 5′-AAGCAGAGUACACACAGCAtatacc-P- ggtAUAUGCUGUGUGUACUCUGCUU-3′ | No.30 | 27 |
| PA-0029 | 5′-AAGCAGAGUACACACAGCAtatacc-P- ggtAUAUGCUGUGUGUACUCUGCUU-3′ | No.31 | 28 |
| PA-0030 | 5′-AAGCAGAGUACACACAGCAUAUACC-P- ggtAUAUGCUGUGUGUACUCUGCtt-3′ | No.32 | 29 |
| GA-0001 | 5′-AAGCAGAGUACACACAGCAUAUACC- Gly-GGUAUAUGCUGUGUGUACUCUGCUU-3′ | No.33 | 30 |
| KA-0001 | 5′-AAGCAGAGUACACACAGCAUAUACC-K- GGUAUAUGCUGUGUGUACUCUGCUU-3′ | No.34 | 31 |
| PA-0031 | 5′-AAUAGCACCAUUUGAAAUCAGUGUtcc- P-ggaacaCUGAUUUCAAAUGGUGCUAUU-3′ | No.35 | 32 |
| PA-0032 | 5′-AACACUGAUUUCAAAUGGUGCUAGACC- P-GGUCUAGCACCAUUUGAAAUCAGUGUU-3′ | No.36 | 33 |
| PA-0033 | 5′-AACACUGAUUUCAAAUGGUGCtagacc- P-ggtCUAGCACCAUUUGAAAUCAGUGUU-3′ | No.37 | 34 |

Table 2 shows the names and base sequences of siRNA as double-stranded nucleic acid molecule and single-stranded nucleic acid molecule, which were used as Reference Example.

Each nucleic acid molecule was synthesized by a nucleic acid synthesizer (trade name: ABI Expedite (registered trademark) 8909 Nucleic Acid Synthesis System, Applied Biosystems) based on the phosphoramidite method. For the aforementioned synthesis, RNA Phosphoramidites (2'-O-TBDMSi, trade name, Samchully Pharm. Co., Ltd.) and EMM amidite (WO 2013/027843) were used as RNA amidite. The aforementioned amidite was deprotected by a conventional method, and the synthesized RNA was purified by HPLC.

**[Table 2]**

| name | base sequence | SEQ ID NO: | Reference Example |
|---|---|---|---|
| NI-0053 (TGF-β) | ^{5'}GCAGAGUACACACAGCAUAUA^{3'} | No.38 | 1 |
| | ^{3'}UUCGUCUCAUGUGUGUCGUAU^{5'} | No.39 | |
| NI-0011 (GAPDH) | ^{5'}CCAUGAGAAGUAUGACAACAG^{3'} | No.40 | 2 |
| | ^{3'}UUGGUACUCUUCAUACUGUUG^{5'} | No.41 | |
| NI-0211 (miR-29b) | ^{5'}CACUGAUUUCAAAUGGUGCUAGA^{3'} | No.42 | 3 |
| | ^{3'}UUGUGACUAAAGUUUACCACGAU^{5'} | No.43 | |
| PH-0009 | 5′-GCAGAGUACACACAGCAUAUACC-P- GGUAUAUGCUGUGUGUACUCUGCUU-3′ | No.44 | 4 |

### Example 1: Synthesis of PA-0001-DSP

The structure of the cyclic nucleic acid molecule of the present invention, PA-0001-DSP, is shown below.

PA-0001-DSP is a cyclic nucleic acid molecule in which Dithiobis (succinimidyl propionate) (hereinafter DSP-ONSu, DOJINDO) containing a disulfide bond is bonded to the amino group at the 5'-terminal and 3'-terminal of the aforementioned PA-0001 by the below-mentioned method.

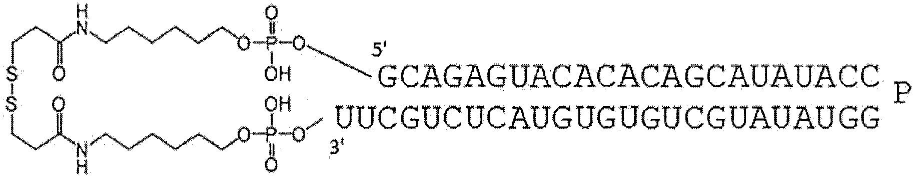

### Synthesis of PA-0001-DSP

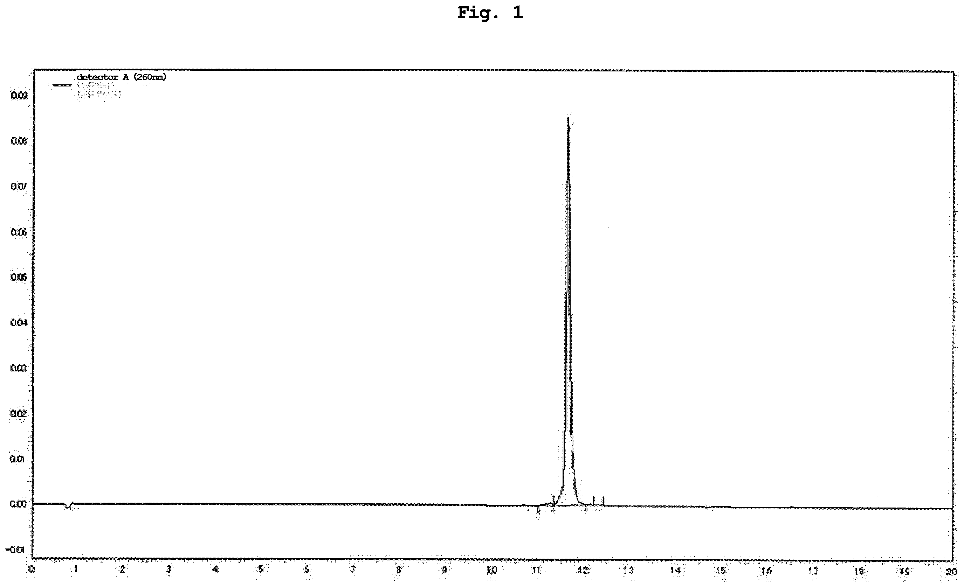

114 µmol/L PA-0001 (350 µL), 10 mg/mL DSP-ONSu/DMF solution (65 µL), ethanol (250 µL) and 1% triethylamine aqueous solution (35 µL) were mixed and stirred at 40°C for 6 hr. The reaction mixture was purified by HPLC (column: Develosil C8-UG-5, 5 µm, 10×50 mm; flow rate: 4.7 mL/min; detection: UV260 nm; column oven: 40°C; Buffer A: 50 mmol/L TEAA (pH 7.0), 5% CH₃CN; Buffer B: CH₃CN) and the peak of the desired product was collected. The collected fraction was subjected to ethanol precipitation and the resulting precipitate was dissolved in distilled water for injection (Otsuka Pharmaceutical, Co., Ltd.). PA-0001-DSP with purity 98.69% was obtained. Mass spectrometry: 16224.00 (Calculated: 16224.08). The HPLC chart after purification is shown in Fig. 1.

### Example 2: Synthesis of PA-0001-DSO

The structure of the cyclic nucleic acid molecule of the present invention, PA-0001-DSO, is shown below.

PA-0001-DSO is a cyclic nucleic acid molecule in which Dithiobis (succinimidyl octanoate) (hereinafter DSO-ONSu, DOJINDO) containing a disulfide bond is bonded to the amino group at the 5'-terminal and 3'-terminal of the aforementioned PA-0001 by the below-mentioned method.

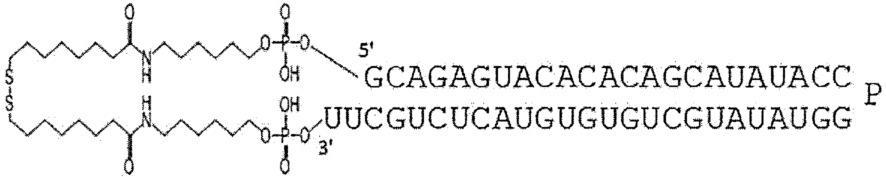

### Synthesis of PA-0001-DSO

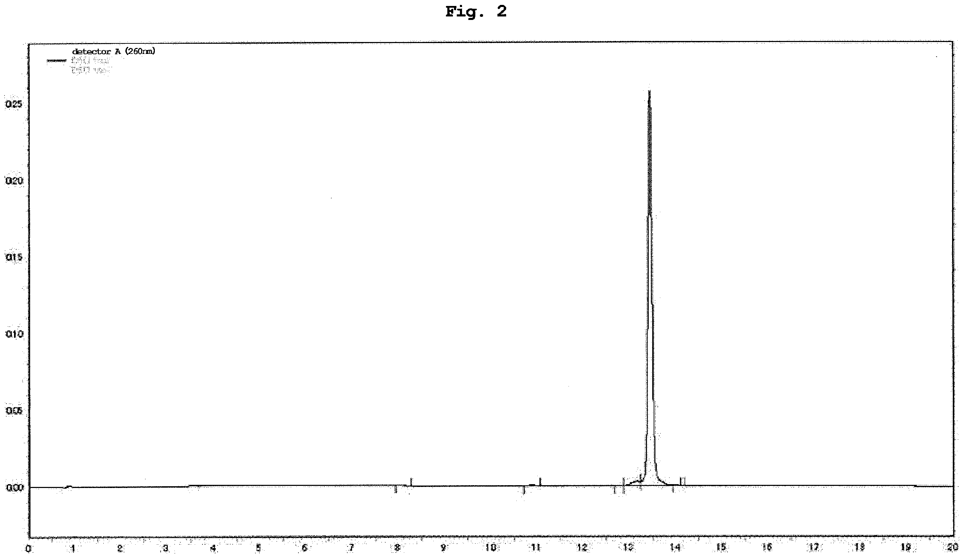

160 µmol/L PA-0001 (250 µL), 10 mmol/L DSO-ONSu/DMSO solution (160 µL), isopropanol (255 µL), 1% triethylamine aqueous solution (35 µL) were mixed and stirred at 40°C for 4 hr. The reaction mixture was purified by HPLC (column: Develosil C8-UG-5, 5 µm, 10×50 mm; flow rate: 4.7 mL/min; detection: UV260 nm; column oven: 40°C; Buffer A: 50 mmol/L TEAA (pH 7.0), 5% CH₃CN; Buffer B: CH₃CN) and the peak of the desired product was collected. The collected fraction was subjected to ethanol precipitation and the resulting precipitate was dissolved in distilled water for injection (Otsuka Pharmaceutical, Co., Ltd.). PA-0001-DSO with purity 97.39% was obtained. Mass spectrometry: 16364.20 (Calculated: 16364.35). The HPLC chart after purification is shown in Fig. 2.

### Example 3: Synthesis of PA-0002-DSP

The structure of the cyclic nucleic acid molecule of the present invention, PA-0002-DSP, is shown below.

PA-0002-DSP is a cyclic nucleic acid molecule in which DSP-ONSu containing a disulfide bond is bonded to the amino group at the 5'-terminal and 3'-terminal of the aforementioned PA-0002 by the below-mentioned method.

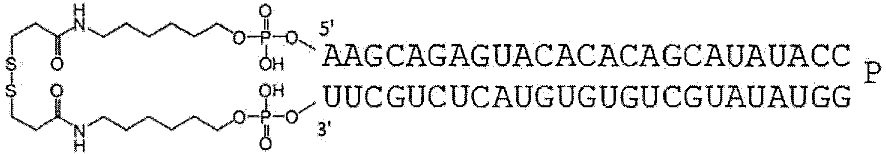

### Synthesis of PA-0002-DSP

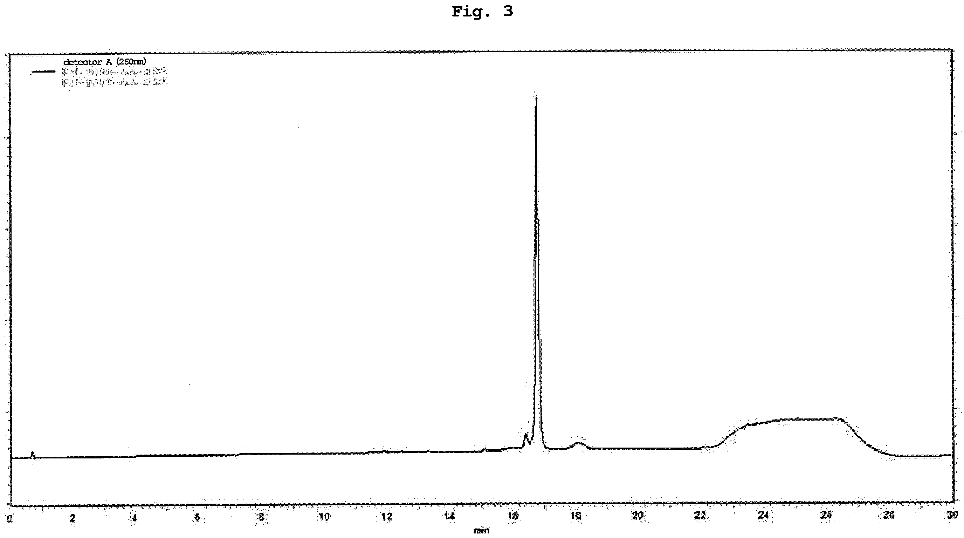

120 µmol/L PA-0002 (350 µL), 10 mg/mL DSP-ONSu/DMF solution (65 µL), isopropanol (250 µL) and 1% triethylamine aqueous solution (35 µL) were mixed and stirred at 40°C for 4 hr. The reaction mixture was purified by HPLC (column: Develosil C8-UG-5, 5 µm, 10×50 mm; flow rate: 4.7 mL/min; detection: UV260 nm; column oven: 40°C; Buffer A: 50 mmol/L TEAA (pH 7.0), 5% CH₃CN; Buffer and the peak of the desired product was collected. The collected fraction was subjected to ethanol precipitation and the resulting precipitate was dissolved in distilled water for injection (Otsuka Pharmaceutical, Co., Ltd.). Absorbance at UV260 nm was measured and the yield was calculated. PA-0002-DSP (0.25 mg) with purity 89.32% was obtained. Mass spectrometry: 16881.90 (Calculated: 16882.50). The HPLC chart after purification is shown in Fig. 3.

### Example 4: Synthesis of PA-0003-DSP

The structure of the cyclic nucleic acid molecule of the present invention, PA-0003-DSP, is shown below.

PA-0003-DSP is a cyclic nucleic acid molecule in which DSP-ONSu containing a disulfide bond is bonded to the amino group at the 5'-terminal and 3'-terminal of the aforementioned PA-0003 by the below-mentioned method.

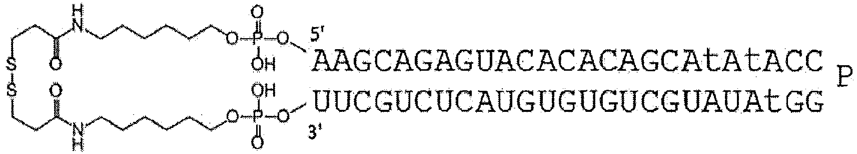

### Synthesis of PA-0003-DSP

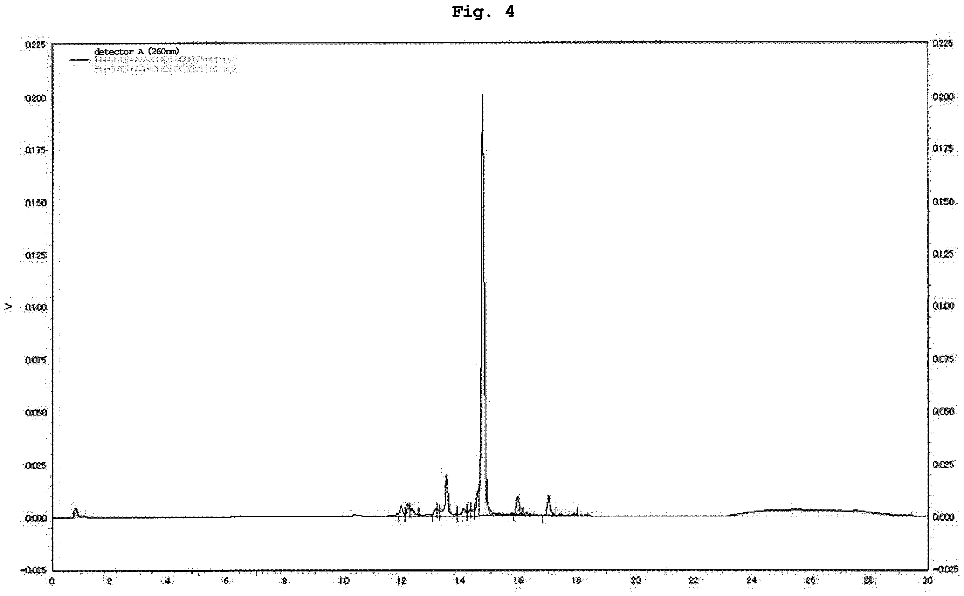

286 µmol/L PA-0003 (110 µL), 10 mg/mL DSP-ONSu/DMF solution (50 µL) and phosphate buffer (pH 8.5, 40 µL, final concentration 200 mmol/L) were mixed and stirred at 35°C for 35 min. The reaction mixture was purified by gel filtration (illustra MicroSpin G-25 Columns, GE Healthcare). Absorbance of the collected fraction at UV260 nm was measured and the yield was calculated. PA-0003-DSP (0.16 mg) with purity 70.99% was obtained. Mass spectrometry: 16876.50 (Calculated: 16876.58). The HPLC chart after purification is shown in Fig. 4.

### Example 5: Synthesis of PA-0004-DSP

The structure of the cyclic nucleic acid molecule of the present invention, PA-0004-DSP, is shown below.

PA-0004-DSP is a cyclic nucleic acid molecule in which DSP-ONSu containing a disulfide bond is bonded to the amino group at the 5'-terminal and 3'-terminal of the aforementioned PA-0004 by the below-mentioned method.

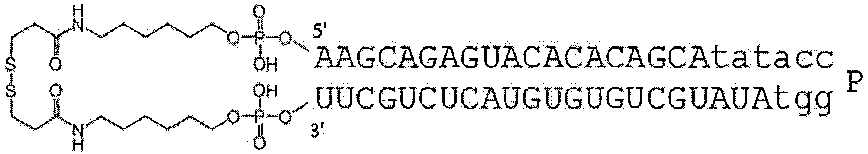

### Synthesis of PA-0004-DSP

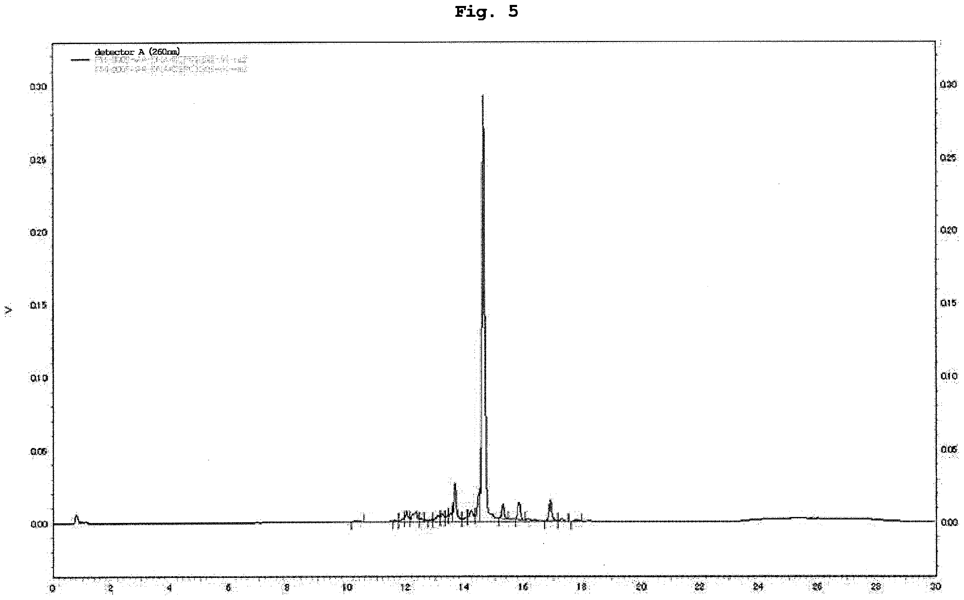

234 µmol/L PA-0004 (116 µL), 10 mg/mL DSP-ONSu/DMF solution (44 µL) and phosphate buffer (pH 8.5, 40 µL, final concentration 200 mmol/L) were mixed and stirred at 35°C for 35 min. The reaction mixture was purified by gel filtration (illustra MicroSpin G-25 Columns, GE Healthcare). Absorbance of the collected fraction at UV260 nm was measured and the yield was calculated. PA-0004-DSP (0.18 mg) with purity 67.13% was obtained. Mass spectrometry: 16780.19 (Calculated: 16780.59). The HPLC chart after purification is shown in Fig. 5.

### Example 6: Synthesis of PA-0005-DSP

The structure of the cyclic nucleic acid molecule of the present invention, PA-0005-DSP, is shown below.

PA-0005-DSP is a cyclic nucleic acid molecule in which DSP-ONSu containing a disulfide bond is bonded to the amino group at the 5'-terminal and 3'-terminal of the aforementioned PA-0005 by the below-mentioned method.

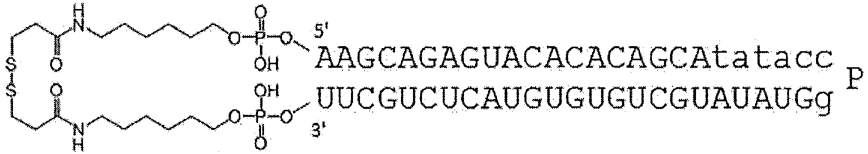

### Synthesis of PA-0005-DSP

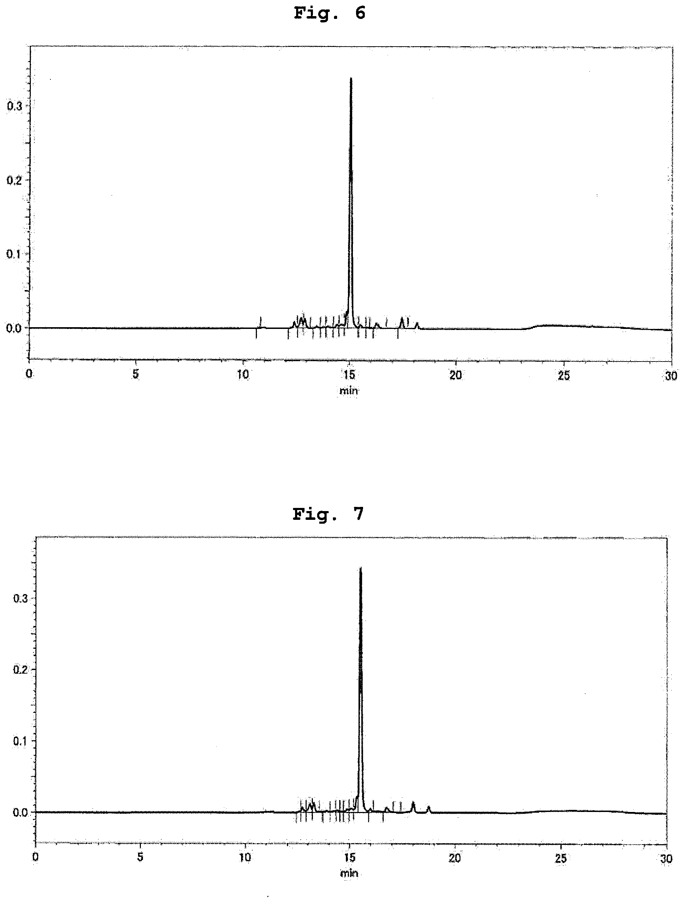

572 µmol/L PA-0005 (70 µL, 40 nmol), distilled water for injection (137.3 µL), phosphate buffer (pH 8.5, 60 µL, final concentration 200 mmol/L) and 10 mg/mL DSP-ONSu/DMF solution (32.7 µL) were mixed and stirred at 35°C for 35 min. To the reaction mixture was added 10 mg/mL dithiothreitol (hereinafter DTT) solution (30 µL) and the mixture was stirred at 25°C for 45 min. Ethanol precipitation was performed and the resulting precipitate was dissolved in distilled water for injection. Absorbance at UV260 nm was measured and the yield was calculated. PA-0005-DSP (0.33 mg) with purity 74.04% was obtained. Mass spectrometry: 16797.10 (Calculated: 16798.56). The HPLC chart after purification is shown in Fig. 6.

### Example 7: Synthesis of PA-0006-DSP

The structure of the cyclic nucleic acid molecule of the present invention, PA-0006-DSP, is shown below.

PA-0006-DSP is a cyclic nucleic acid molecule in which DSP-ONSu containing a disulfide bond is bonded to the amino group at the 5'-terminal and 3'-terminal of the aforementioned PA-0006 by the below-mentioned method.

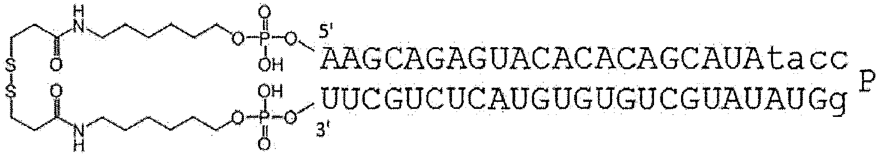

PA-0006 (40 nmol) was subjected to a reaction similar to that in PA-0005. Ethanol precipitation was performed and the resulting precipitate was dissolved in distilled water for injection. Absorbance at UV260 nm was measured and the yield was calculated. PA-0006-DSP (0.36 mg) with purity 77.59% was obtained. Mass spectrometry: 16815.70 (Calculated: 16816.53). The HPLC chart after purification is shown in Fig. 7.

### Example 8: Synthesis of PA-0007-DSP

The structure of the cyclic nucleic acid molecule of the present invention, PA-0007-DSP, is shown below.

PA-0007-DSP is a cyclic nucleic acid molecule in which DSP-ONSu containing a disulfide bond is bonded to the amino group at the 5'-terminal and 3'-terminal of the aforementioned PA-0007 by the below-mentioned method.

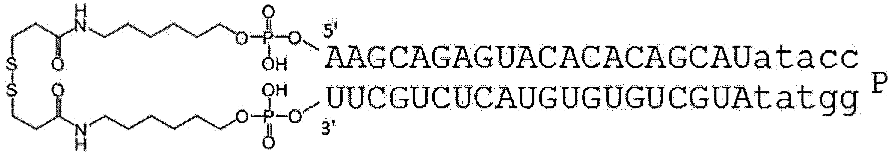

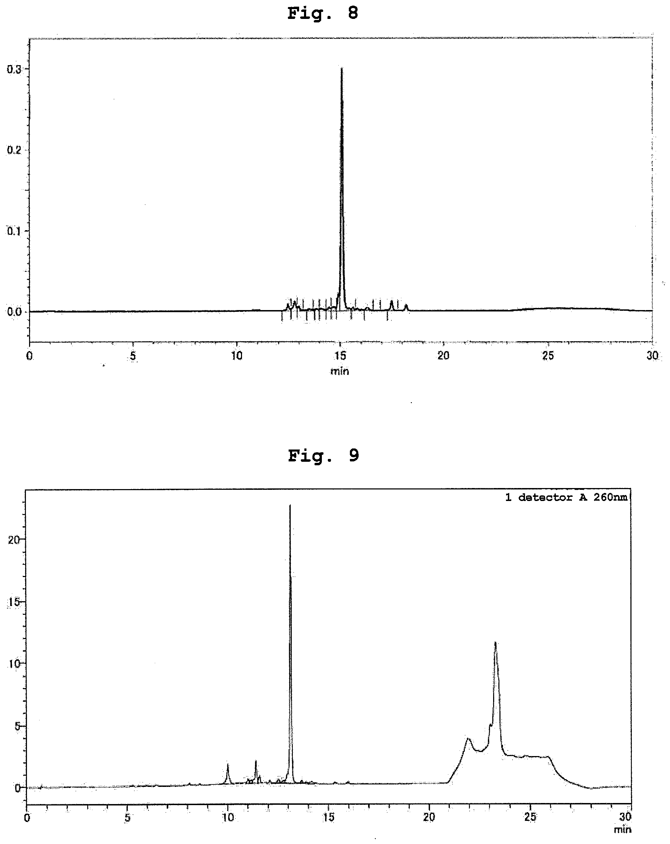

PA-0007 (40 nmol) was subjected to a reaction similar to that in PA-0005. Ethanol precipitation was performed and the resulting precipitate was dissolved in distilled water for injection. Absorbance at UV260 nm was measured and the yield was calculated. PA-0007-DSP (0.35 mg) with purity 75.32% was obtained. Mass spectrometry: 16763.80 (Calculated: 16764.59). The HPLC chart after purification is shown in Fig. 8.

### Example 9: Synthesis of PA-0008-DSP

The structure of the cyclic nucleic acid molecule of the present invention, PA-0008-DSP, is shown below.

PA-0008-DSP is a cyclic nucleic acid molecule in which DSP-ONSu containing a disulfide bond is bonded to the amino group at the 5'-terminal and 3'-terminal of the aforementioned PA-0008 by the below-mentioned method.

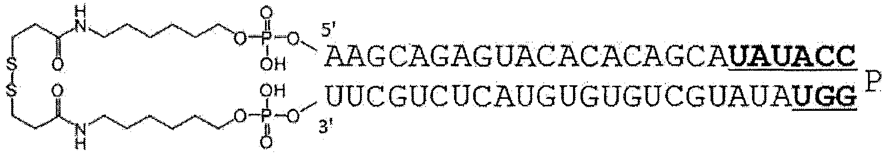

### Synthesis of PA-0008-DSP

218 µmol/L PA-0008 (137.6 µL, 30 nmol), distilled water for injection (16.3 µL), phosphate buffer (pH 8.5, 40 µL, final concentration 200 mmol/L) and 10 mg/mL DSP-ONSu/DMF solution (6.07 µL) were mixed and stirred at 35°C for 4 hr. To the reaction mixture was added 10 mg/mL DTT solution (10 µL) and the mixture was stirred at 25°C for 3 hr.

Ethanol precipitation was performed and the resulting precipitate was dissolved in distilled water for injection and purified by gel filtration (illustra MicroSpin G-25 Columns, GE Healthcare). Absorbance at UV260 nm was measured and the yield was calculated. PA-0008-DSP (0.04 mg) with purity 75.28% was obtained. Mass spectrometry: 17008.40 (Calculated: 17009.55). The HPLC chart after purification is shown in Fig. 9.

### Example 10: Synthesis of PA-0009-DSP

The structure of the cyclic nucleic acid molecule of the present invention, PA-0009-DSP, is shown below.

PA-0009-DSP is a cyclic nucleic acid molecule in which DSP-ONSu containing a disulfide bond is bonded to the amino group at the 5'-terminal and 3'-terminal of the aforementioned PA-0009 by the below-mentioned method.

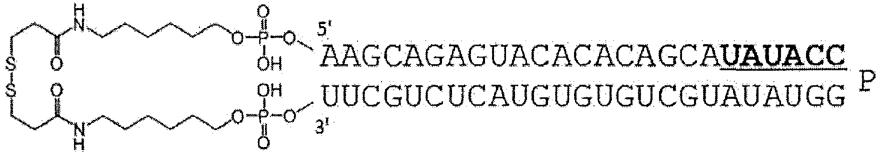

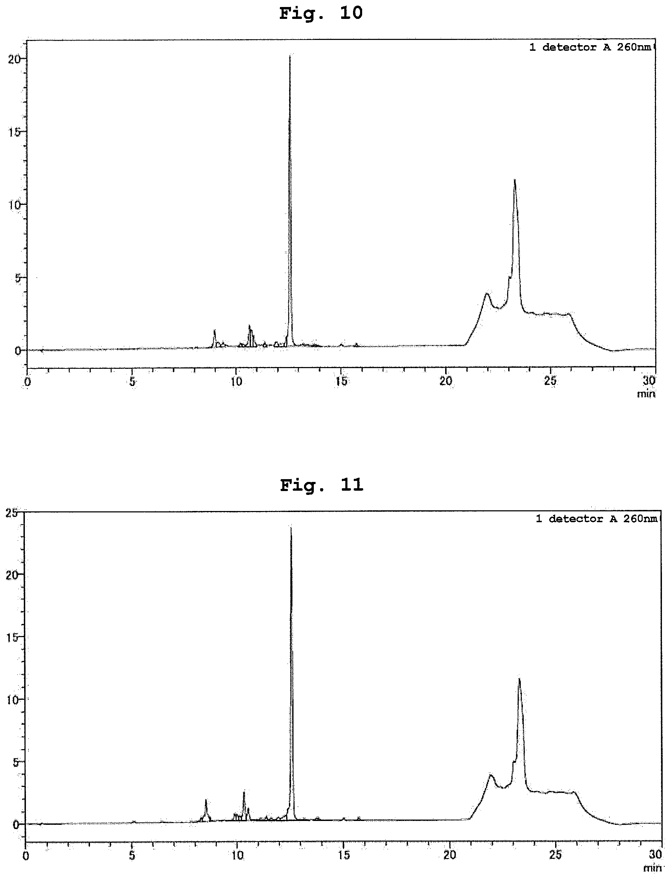

PA-0009 (30 nmol) was subjected to a reaction similar to that for PA-0008, ethanol precipitation and purification by gel filtration. Absorbance at UV260 nm was measured and the yield was calculated. PA-0009-DSP (0.04 mg) with purity 70.17% was obtained. Mass spectrometry: 16966.40 (Calculated: 16967.20). The HPLC chart after purification is shown in Fig. 10.

### Example 11: Synthesis of PA-0010-DSP

The structure of the cyclic nucleic acid molecule of the present invention, PA-0010-DSP, is shown below.

PA-0010-DSP is a cyclic nucleic acid molecule in which DSP-ONSu containing a disulfide bond is bonded to the amino group at the 5'-terminal and 3'-terminal of the aforementioned PA-0010 by the below-mentioned method.

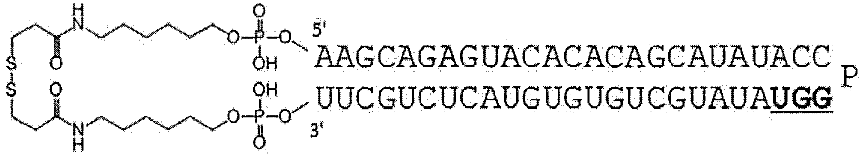

PA-0010 (30 nmol) was subjected to a reaction similar to that for PA-0008, ethanol precipitation and purification by gel filtration. Absorbance at UV260 nm was measured and the yield was calculated. PA-0010-DSP (0.04 mg) with purity 66.61% was obtained. Mass spectrometry: 16924.10 (Calculated: 16924.85). The HPLC chart after purification is shown in Fig. 11.

### Example 12: Synthesis of PA-0011-DSP

The structure of the cyclic nucleic acid molecule of the present invention, PA-0011-DSP, is shown below.

PA-0011-DSP is a cyclic nucleic acid molecule in which DSP-ONSu containing a disulfide bond is bonded to the amino group at the 5'-terminal and 3'-terminal of the aforementioned PA-0011 by the below-mentioned method.

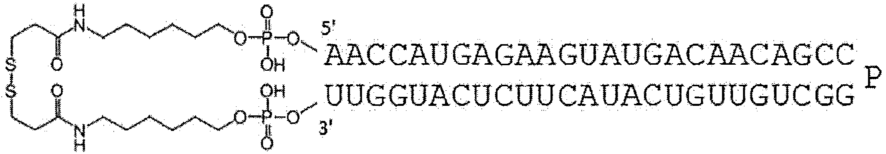

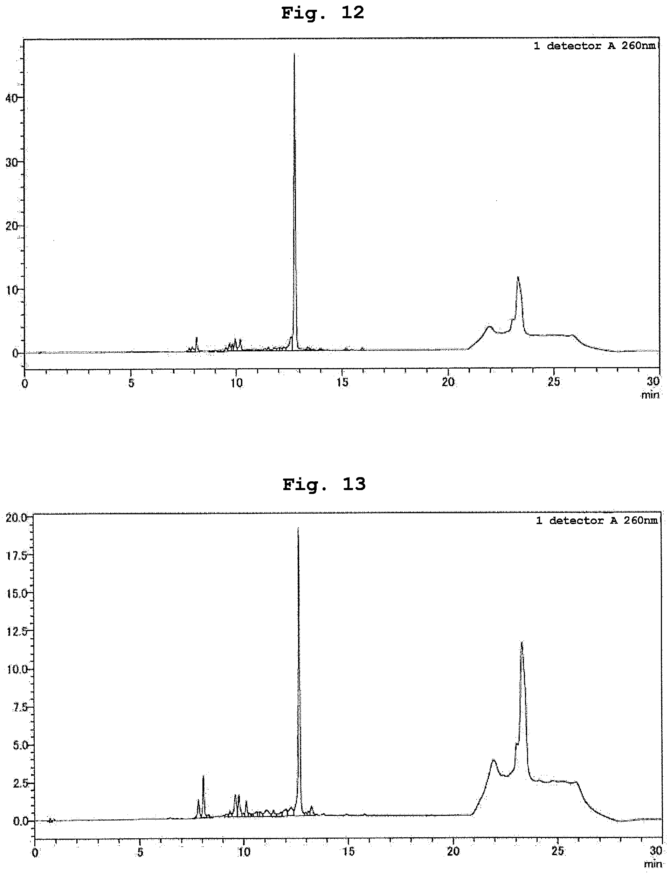

PA-0011 (30 nmol) was subjected to a reaction similar to that for PA-0008, ethanol precipitation and purification by gel filtration. Absorbance at UV260 nm was measured and the yield was calculated. PA-0011-DSP (0.07 mg) with purity 66.43% was obtained. Mass spectrometry: 16882.30 (Calculated: 16882.50). The HPLC chart after purification is shown in Fig. 12.

### Example 13: Synthesis of PA-0012-DSP

The structure of the cyclic nucleic acid molecule of the present invention, PA-0012-DSP, is shown below.

PA-0012-DSP is a cyclic nucleic acid molecule in which DSP-ONSu containing a disulfide bond is bonded to the amino group at the 5'-terminal and 3'-terminal of the aforementioned PA-0012 by the below-mentioned method.

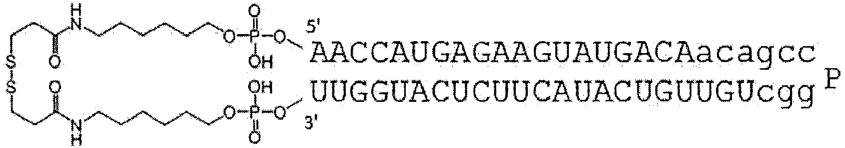

PA-0012 (30 nmol) was subjected to a reaction similar to that for PA-0008, ethanol precipitation and purification by gel filtration. Absorbance at UV260 nm was measured and the yield was calculated. PA-0012-DSP (0.04 mg) with purity 51.99% was obtained. Mass spectrometry: 16738.00 (Calculated: 16738.50). The HPLC chart after purification is shown in Fig. 13.

### Example 14: Synthesis of PA-0013-DSP

The structure of the cyclic nucleic acid molecule of the present invention, PA-0013-DSP, is shown below.

PA-0013-DSP is a cyclic nucleic acid molecule in which DSP-ONSu containing a disulfide bond is bonded to the amino group at the 5'-terminal and 3'-terminal of the aforementioned PA-0013 by the below-mentioned method.

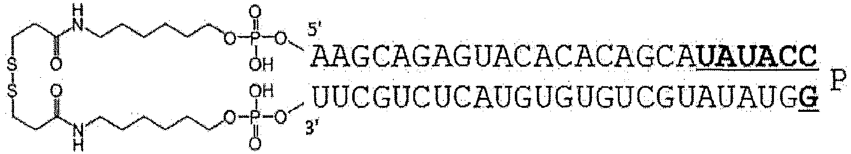

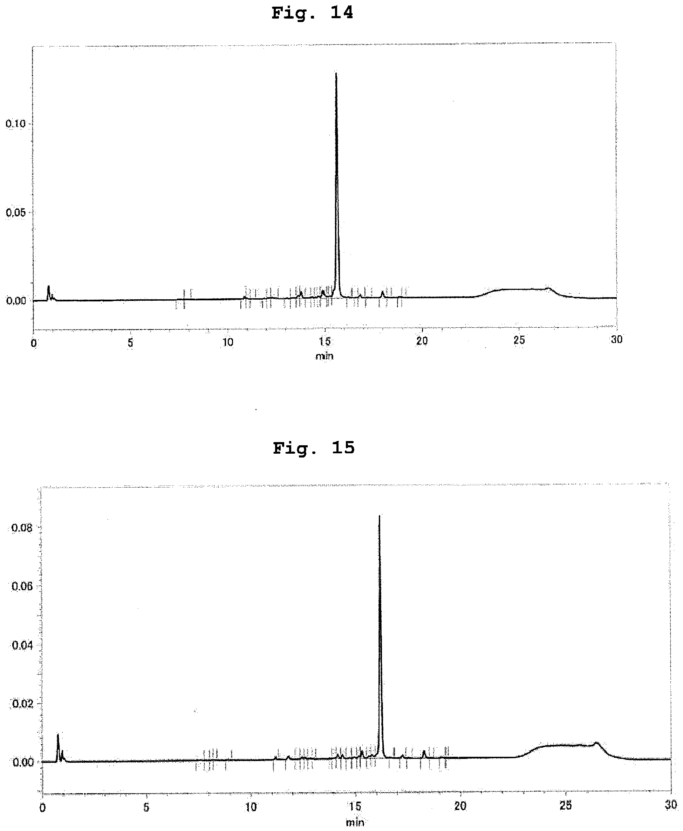

394 µmol/L PA-0013 (25.4 µL, 10 nmol), distilled water for injection (50.5 µL), phosphate buffer (pH 8.5, 20 µL, final concentration 200 mmol/L) and 10 mg/mL DSP-ONSu/DMF solution (4.1 µL) were mixed and stirred at 25°C for 5 hr. The reaction mixture was purified by gel filtration (illustra MicroSpin G-25 Columns, GE Healthcare). After concentration by centrifugal concentrator, the mixture was dissolved in distilled water for injection. Absorbance at UV260 nm was measured and the yield was calculated. PA-0013-DSP (0.14 mg) with purity 82.57% was obtained. Mass spectrometry: 16981.20 (Calculated: 16981.31). The HPLC chart after purification is shown in Fig. 14.

### Example 15: Synthesis of PA-0014-DSP

The structure of the cyclic nucleic acid molecule of the present invention, PA-0014-DSP, is shown below.

PA-0014-DSP is a cyclic nucleic acid molecule in which DSP-ONSu containing a disulfide bond is bonded to the amino group at the 5'-terminal and 3'-terminal of the aforementioned PA-0014 by the below-mentioned method.

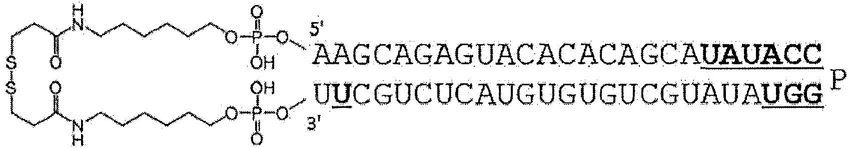

PA-0014 (10 nmol) was subjected to a reaction and purification similar to those for PA-0013. Absorbance at UV260 nm was measured and the yield was calculated. PA-0014-DSP (0.12 mg) with purity 80.14% was obtained. Mass spectrometry: 17023.30 (Calculated: 17023.67). The HPLC chart after purification is shown in Fig. 15.

### Example 16: Synthesis of PA-0015-DSP

The structure of the cyclic nucleic acid molecule of the present invention, PA-0015-DSP, is shown below.

PA-0015-DSP is a cyclic nucleic acid molecule in which DSP-ONSu containing a disulfide bond is bonded to the amino group at the 5'-terminal and 3'-terminal of the aforementioned PA-0015 by the below-mentioned method.

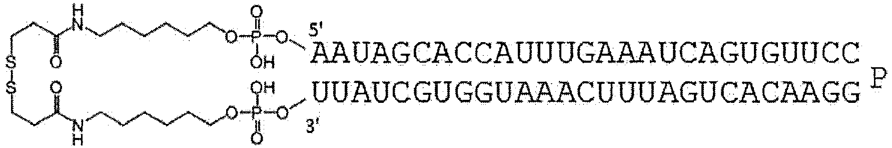

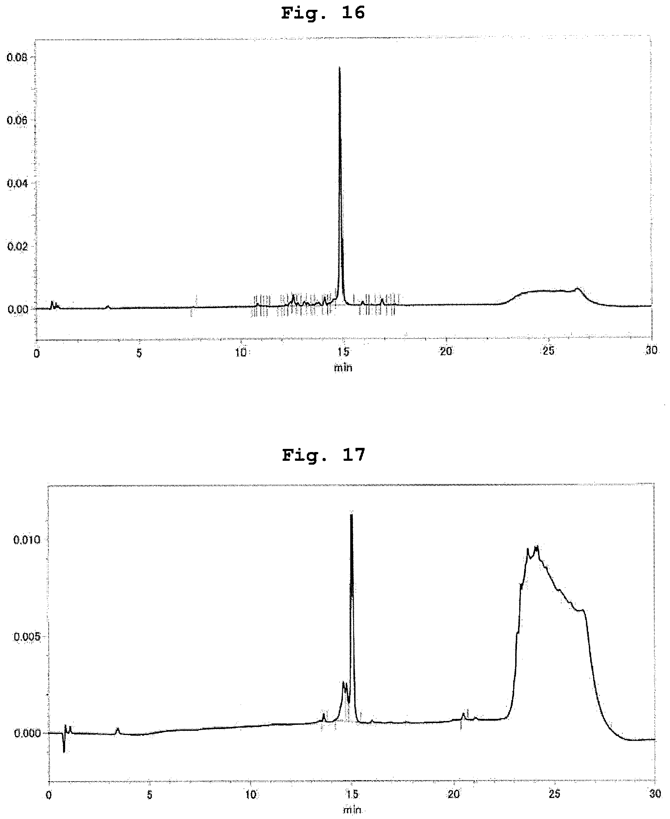

PA-0015 (10 nmol) was subjected to a reaction and purification similar to those for PA-0013. Absorbance at UV260 nm was measured and the yield was calculated. PA-0015-DSP (0.09 mg) with purity 76.21% was obtained. Mass spectrometry: 18138.80 (Calculated: 18138.23). The HPLC chart after purification is shown in Fig. 16.

### Example 17: Synthesis of PA-0016-DSP

The structure of the cyclic nucleic acid molecule of the present invention, PA-0016-DSP, is shown below.

PA-0016-DSP is a cyclic nucleic acid molecule in which DSP-ONSu containing a disulfide bond is bonded to the amino group at the 5'-terminal and 3'-terminal of the aforementioned PA-0016 by the below-mentioned method.

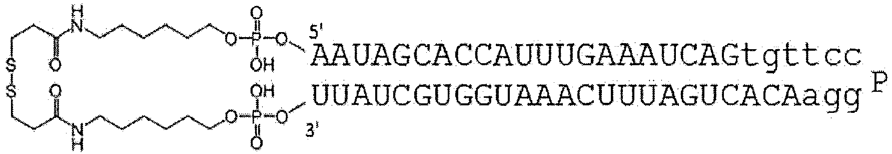

PA-0016 (10 nmol) was subjected to a reaction similar to that for PA-0013. The reaction mixture was purified by HPLC (column: XBridge Oligonucleotide BEH C18, 2.5 µm, 10×50 mm; flow rate: 4.0 mL/min; detection: UV260 nm; column oven: 60°C; Buffer A: 50 mmol/L TEAA (pH 7.0), 5% CH₃CN; Buffer B: 50 mmol/L TEAA (pH 7.0), 50% CH₃CN) and the peak of the desired product was collected. The collected fraction was concentrated by centrifugal concentrator and dissolved in distilled water for injection. Absorbance at UV260 nm was measured and the yield was calculated. PA-0016-DSP (0.05 mg) with purity 62.48% was obtained. Mass spectrometry: 18037.40 (Calculated: 18036.32). The HPLC chart after purification is shown in Fig. 17.

### Example 18: Synthesis of PA-0017-DSP

The structure of the cyclic nucleic acid molecule of the present invention, PA-0017-DSP, is shown below.

PA-0017-DSP is a cyclic nucleic acid molecule in which DSP-ONSu containing a disulfide bond is bonded to the amino group at the 5'-terminal and 3'-terminal of the aforementioned PA-0017 by the below-mentioned method.

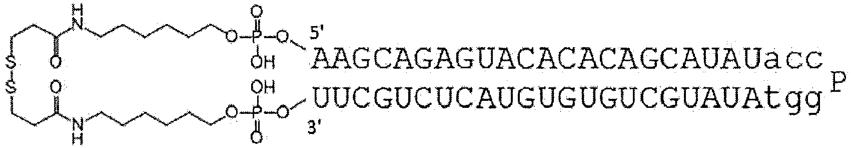

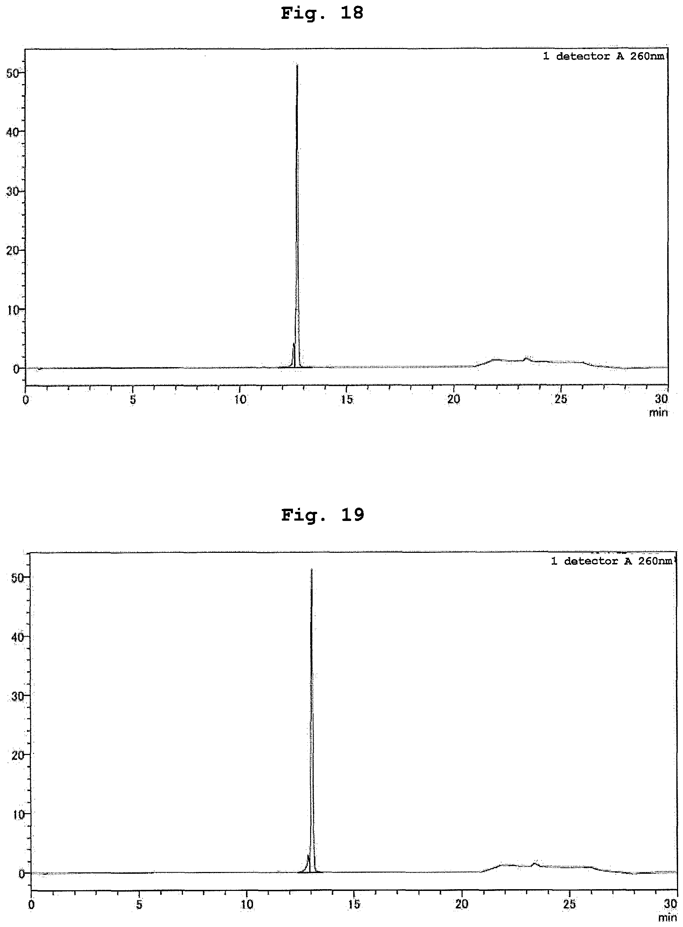

469 µmol/L PA-0017 (42.6 µL, 20 nmol), distilled water for injection (29.3 µL), phosphate buffer (pH 8.5, 20 µL, final concentration 200 mmol/L) and 10 mg/mL DSP-ONSu/DMF solution (8.1 µL) were mixed and stirred at 25°C for 3.5 hr. The reaction mixture was purified by HPLC (column: Develosil C8-UG-5, 5 µm, 10×50 mm; flow rate: 4.0 mL/min; detection: UV260 nm; column oven: 40°C; Buffer A: 50 mmol/L TEAA (pH 7.0), 5% CH₃CN; Buffer B: 50 mmol/L TEAA (pH 7.0), 50% CH₃CN) and the peak of the desired product was collected. The collected fraction was subjected to ethanol precipitation and the resulting precipitate was dissolved in distilled water for injection. Absorbance at UV260 nm was measured and the yield was calculated. PA-0017-DSP (0.13 mg) with purity 90.76% was obtained. Mass spectrometry: 16800.50 (Calculated: 16800.53). The HPLC chart after purification is shown in Fig. 18.

### Example 19: Synthesis of PA-0018-DSP

The structure of the cyclic nucleic acid molecule of the present invention, PA-0018-DSP, is shown below.

PA-0018-DSP is a cyclic nucleic acid molecule in which DSP-ONSu containing a disulfide bond is bonded to the amino group at the 5'-terminal and 3'-terminal of the aforementioned PA-0018 by the below-mentioned method.

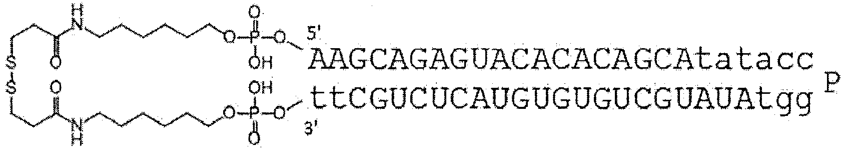

PA-0018 (20 nmol) was subjected to a reaction and purification similar to those for PA-0017. Absorbance at UV260 nm was measured and the yield was calculated. PA-0018-DSP (0.13 mg) with purity 92.87% was obtained. Mass spectrometry: 16776.00 (Calculated: 16776.64). The HPLC chart after purification is shown in Fig. 19.

### Example 20: Synthesis of PA-0019-DSP

The structure of the cyclic nucleic acid molecule of the present invention, PA-0019-DSP, is shown below.

PA-0019-DSP is a cyclic nucleic acid molecule in which DSP-ONSu containing a disulfide bond is bonded to the amino group at the 5'-terminal and 3'-terminal of the aforementioned PA-0019 by the below-mentioned method.

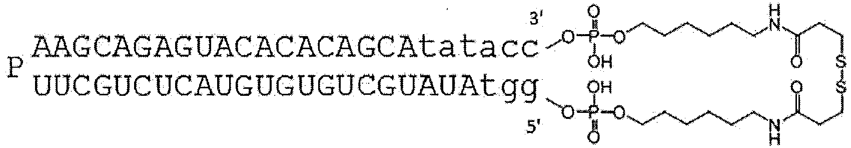

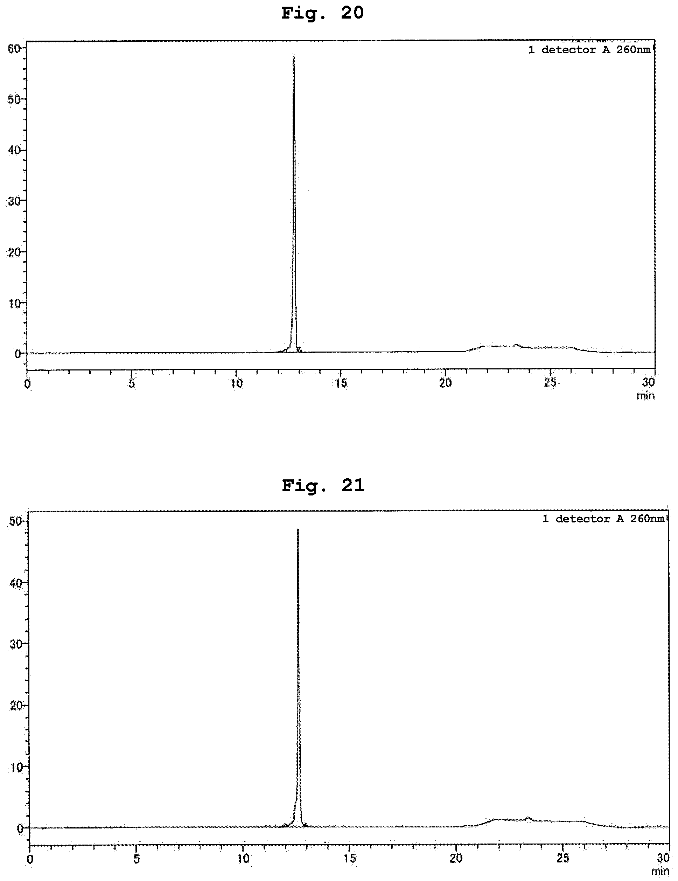

PA-0019 (20 nmol) was subjected to a reaction and purification similar to those for PA-0017. Absorbance at UV260 nm was measured and the yield was calculated. PA-0019-DSP (0.15 mg) with purity 96.37% was obtained. Mass spectrometry: 16779.90 (Calculated: 16780.59). The HPLC chart after purification is shown in Fig. 20.

### Example 21: Synthesis of PA-0020-DSP

The structure of the cyclic nucleic acid molecule of the present invention, PA-0020-DSP, is shown below.

PA-0020-DSP is a cyclic nucleic acid molecule in which DSP-ONSu containing a disulfide bond is bonded to the amino group at the 5'-terminal and 3'-terminal of the aforementioned PA-0020 by the below-mentioned method.

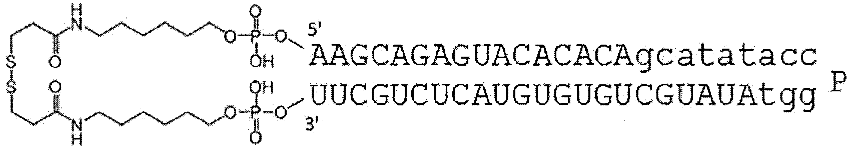

### Synthesis of PA-0020-DSP

PA-0020 (20 nmol) was subjected to a reaction and purification similar to those for PA-0017. Absorbance at UV260 nm was measured and the yield was calculated. PA-0020-DSP (0.13 mg) with purity 98.13% was obtained. Mass spectrometry: 16731.70 (Calculated: 16732.59). The HPLC chart after purification is shown in Fig. 21.

### Example 22: Synthesis of PA-0021-DSP

The structure of the cyclic nucleic acid molecule of the present invention, PA-0021-DSP, is shown below.

PA-0021-DSP is a cyclic nucleic acid molecule in which DSP-ONSu containing a disulfide bond is bonded to the amino group at the 5'-terminal and 3'-terminal of the aforementioned PA-0021 by the below-mentioned method.

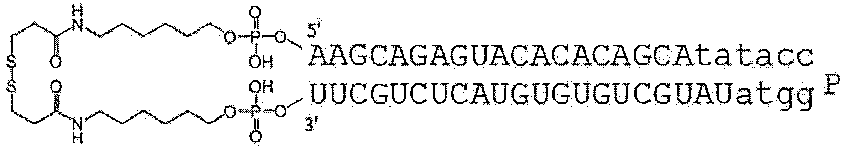

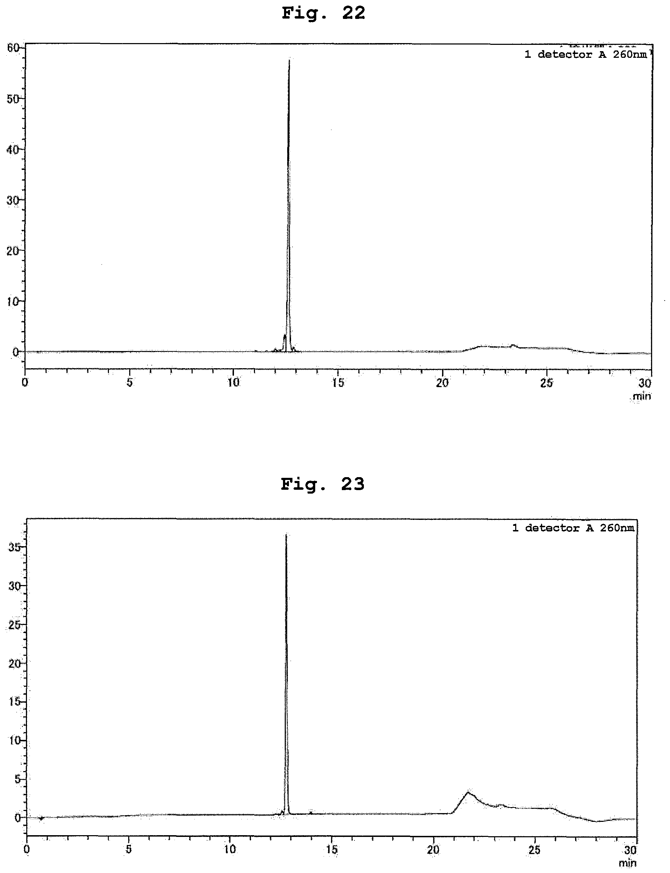

PA-0021 (20 nmol) was subjected to a reaction and purification similar to those for PA-0017. Absorbance at UV260 nm was measured and the yield was calculated. PA-0021-DSP (0.14 mg) with purity 91.16% was obtained. Mass spectrometry: 16763.80 (Calculated: 16764.59). The HPLC chart after purification is shown in Fig. 22.

### Example 23: Synthesis of PA-0024-DSP

The structure of the cyclic nucleic acid molecule of the present invention, PA-0024-DSP, is shown below.

PA-0024-DSP is a cyclic nucleic acid molecule in which DSP-ONSu containing a disulfide bond is bonded to the amino group at the 5'-terminal and 3'-terminal of the aforementioned PA-0024 by the below-mentioned method.

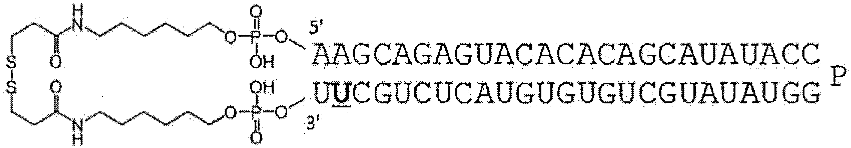

337 µmol/L PA-0024 (149 µL, 50 nmol), distilled water for injection (31 µL), phosphate buffer (pH 8.5, 50 µL, final concentration 200 mmol/L) and 10 mg/mL DSP-ONSu/DMF solution (20 µL) were mixed and stirred at 25°C for 1.5 hr. The reaction mixture was purified by anion exchange chromatography (column: DNAPacPA200, 9×250 mm; flow rate: 2.5 mL/min; detection: UV260 nm; Buffer A: 25 mmol/L Tris-HCl (pH 8.0), 10% CH₃CN; Buffer B: 25 mmol/L Tris-HCl (pH 8.0), 10% CH₃CN, 700 mmol/L NaClO₄) and the peak of the desired product was collected. The collected fraction was subjected to ethanol precipitation and the resulting precipitate was dissolved in distilled water for injection. Absorbance at UV260 nm was measured and the yield was calculated. PA-0024-DSP (0.35 mg) with purity 96.89% was obtained. Mass spectrometry: 16897.20 (Calculated: 16896.61). The HPLC chart after purification is shown in Fig. 23.

### Example 24: Synthesis of PA-0025-DSP

The structure of the cyclic nucleic acid molecule of the present invention, PA-0025-DSP, is shown below.

PA-0025-DSP is a cyclic nucleic acid molecule in which DSP-ONSu containing a disulfide bond is bonded to the amino group at the 5'-terminal and 3'-terminal of the aforementioned PA-0025 by the below-mentioned method.

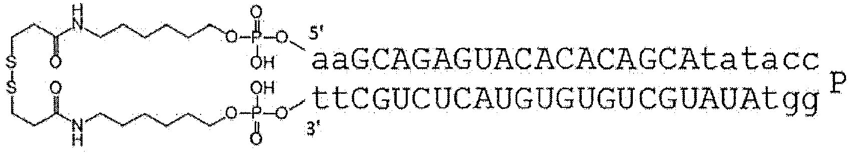

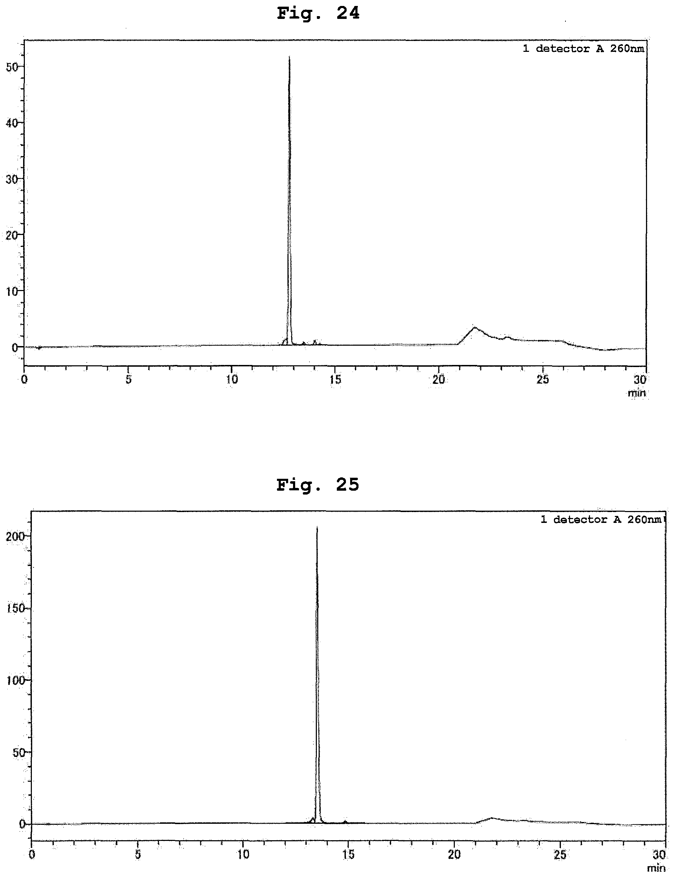

PA-0025 (50 nmol) was subjected to a reaction and purification similar to those for PA-0024. Absorbance at UV260 nm was measured and the yield was calculated. PA-0025-DSP (0.39 mg) with purity 93.33% was obtained. Mass spectrometry: 16745.20 (Calculated: 16744.64). The HPLC chart after purification is shown in Fig. 24.

### Example 25: Synthesis of PA-0026-DSP

The structure of the cyclic nucleic acid molecule of the present invention, PA-0026-DSP, is shown below.

PA-0026-DSP is a cyclic nucleic acid molecule in which DSP-ONSu containing a disulfide bond is bonded to the amino group at the 5'-terminal and 3'-terminal of the aforementioned PA-0026 by the below-mentioned method.

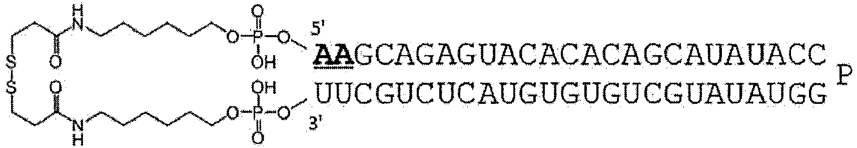

PA-0026 (50 nmol) was subjected to a reaction and purification similar to those for PA-0024 with the reaction time as 30 min. Absorbance at UV260 nm was measured and the yield was calculated. PA-0026-DSP (0.33 mg) with purity 95.86% was obtained. Mass spectrometry: 16911.20 (Calculated: 16910.73). The HPLC chart after purification is shown in Fig. 25.

### Example 26: Synthesis of PA-0027-DSP

The structure of the cyclic nucleic acid molecule of the present invention, PA-0027-DSP, is shown below.

PA-0027-DSP is a cyclic nucleic acid molecule in which DSP-ONSu containing a disulfide bond is bonded to the amino group at the 5'-terminal and 3'-terminal of the aforementioned PA-0027 by the below-mentioned method.

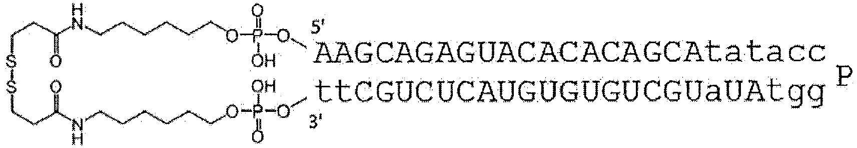

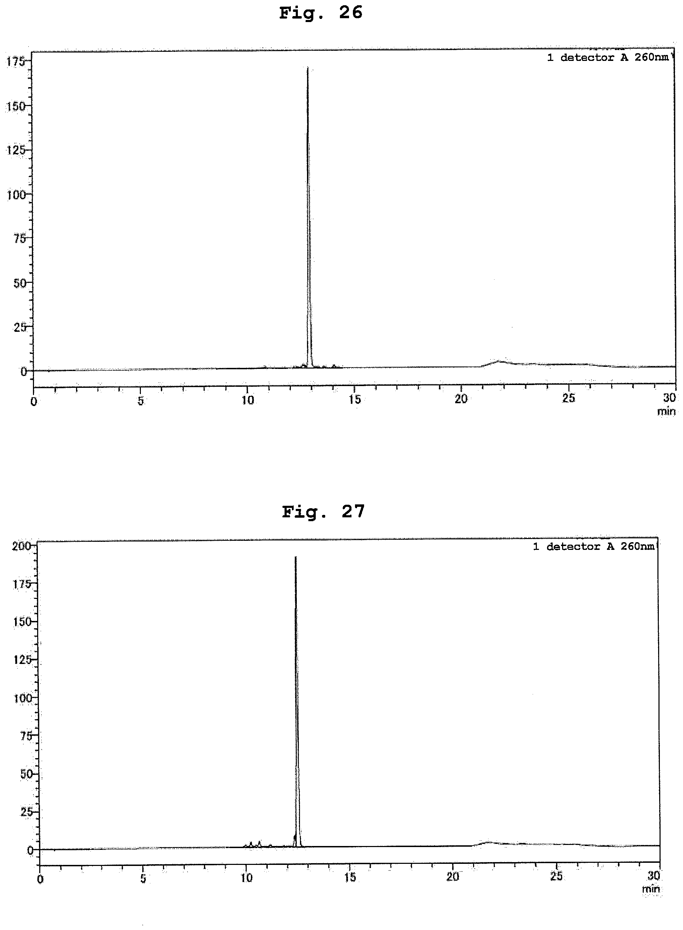

PA-0027 (50 nmol) was subjected to a reaction and purification similar to those for PA-0024 with the reaction time as 40 min. Absorbance at UV260 nm was measured and the yield was calculated. PA-0027-DSP (0.39 mg) with purity 92.83% was obtained. Mass spectrometry: 16761.10 (Calculated: 16760.64). The HPLC chart after purification is shown in Fig. 26.

### Example 27: Synthesis of PA-0028-DSP

The structure of the cyclic nucleic acid molecule of the present invention, PA-0028-DSP, is shown below.

PA-0028-DSP is a cyclic nucleic acid molecule in which DSP-ONSu containing a disulfide bond is bonded to the amino group at the 5'-terminal and 3'-terminal of the aforementioned PA-0028 by the below-mentioned method.

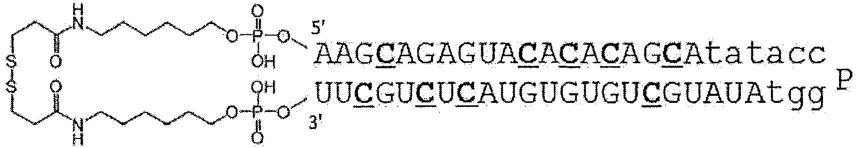

343 µmol/L PA-0028 (583 µL) as unpurified product (200 nmol as unpurified product), distilled water for injection (136 µL), phosphate buffer (pH 8.5, 200 µL, final concentration 200 mmol/L) and 10 mg/mL DSP-ONSu/DMF solution (81 µL) were mixed and stirred at 25°C for 1 hr. The reaction mixture was purified by HPLC (column: XBridge Oligonucleotide BEH C18, 2.5 µm, 10×50 mm; flow rate: 4.0 mL/min; detection: UV260 nm; column oven: 60°C; Buffer A: 50 mmol/L TEAA (pH 7.0), 5% CH₃CN; Buffer B: 50 mmol/L TEAA (pH 7.0), 50% CH₃CN). The collected fraction was further purified by anion exchange chromatography (column: DNAPacPA200, 9×250 mm; flow rate: 2.5 mL/min; detection: UV260 nm; Buffer A: 25 mmol/L Tris-HCl (pH 8.0), 10% CH₃CN; Buffer B: 25 mmol/L Tris-HCl (pH 8.0), 10% CH₃CN, 700 mmol/L NaClO₄) and the peak of the desired product was collected. The collected fraction was subjected to ethanol precipitation and the resulting precipitate was dissolved in distilled water for injection. Absorbance at UV260 nm was measured and the yield was calculated. PA-0028-DSP (0.22 mg) with purity 86.04% was obtained. Mass spectrometry: 16907.40 (Calculated: 16907.64). The HPLC chart after purification is shown in Fig. 27.

### Example 28: Synthesis of PA-0029-DSP

The structure of the cyclic nucleic acid molecule of the present invention, PA-0029-DSP, is shown below.

PA-0029-DSP is a cyclic nucleic acid molecule in which DSP-ONSu containing a disulfide bond is bonded to the amino group at the 5'-terminal and 3'-terminal of the aforementioned PA-0029 by the below-mentioned method.

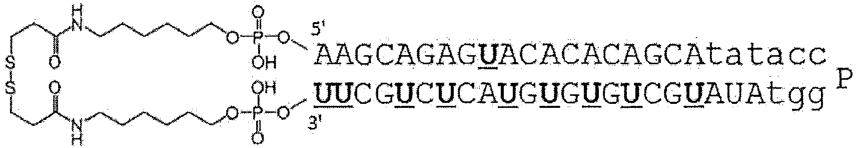

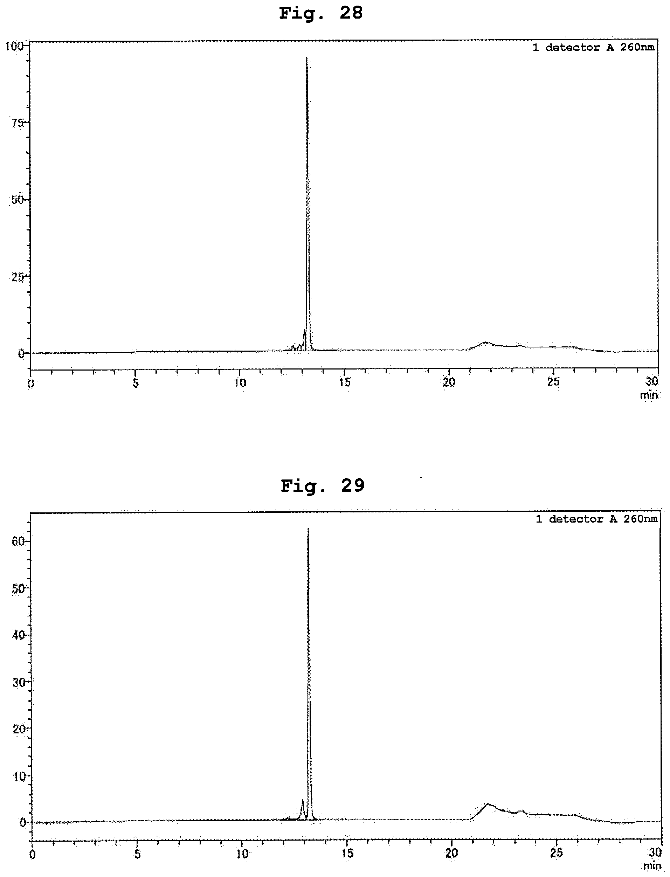

PA-0029 (200 nmol) (as unpurified product) was subjected to a reaction and purification similar to those for PA-0028. Absorbance at UV260 nm was measured and the yield was calculated. PA-0029-DSP (0.10 mg) with purity 86.88% was obtained. Mass spectrometry: 16922.10 (Calculated: 16921.76). The HPLC chart after purification is shown in Fig. 28.

### Example 29: Synthesis of PA-0030-DSP

The structure of the cyclic nucleic acid molecule of the present invention, PA-0030-DSP, is shown below.

PA-0030-DSP is a cyclic nucleic acid molecule in which DSP-ONSu containing a disulfide bond is bonded to the amino group at the 5'-terminal and 3'-terminal of the aforementioned PA-0030 by the below-mentioned method.

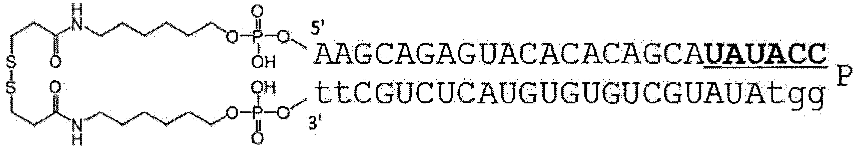

PA-0030 (200 nmol) (as unpurified product) was subjected to a reaction and purification similar to those for PA-0028. Absorbance at UV260 nm was measured and the yield was calculated. PA-0030-DSP (0.11 mg) with purity 88.77% was obtained. Mass spectrometry: 16929.10 (Calculated: 16929.28). The HPLC chart after purification is shown in Fig. 29.

### Example 30: Synthesis of GA-0001-DSP

The structure of the cyclic nucleic acid molecule of the present invention, GA-0001-DSP, is shown below.

GA-0001-DSP is a cyclic nucleic acid molecule in which DSP-ONSu containing a disulfide bond is bonded to the amino group at the 5'-terminal and 3'-terminal of the aforementioned GA-0001 by the below-mentioned method.

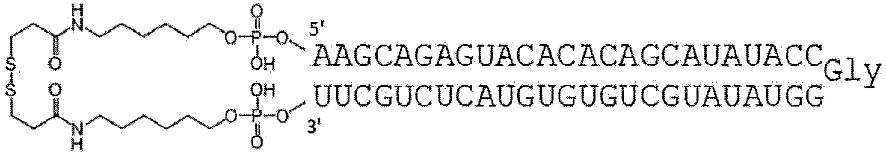

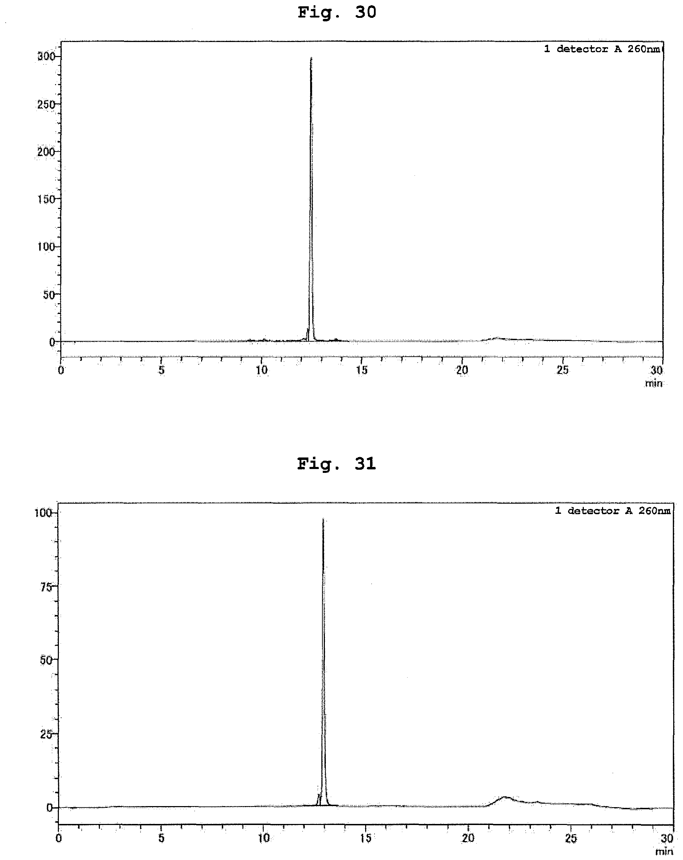

GA-0001 (50 nmol) was subjected to a reaction and purification similar to those for PA-0024 with the reaction time as 1 hr. Absorbance at UV260 nm was measured and the yield was calculated. GA-0001-DSP (0.42 mg) with purity 90.89% was obtained. Mass spectrometry: 16843.40 (Calculated: 16842.43). The HPLC chart after purification is shown in Fig. 30.

### Example 31: Synthesis of KA-0001-DSP

The structure of the cyclic nucleic acid molecule of the present invention, KA-0001-DSP, is shown below.

KA-0001-DSP is a cyclic nucleic acid molecule in which DSP-ONSu containing a disulfide bond is bonded to the amino group at the 5'-terminal and 3'-terminal of the aforementioned KA-0001 by the below-mentioned method.

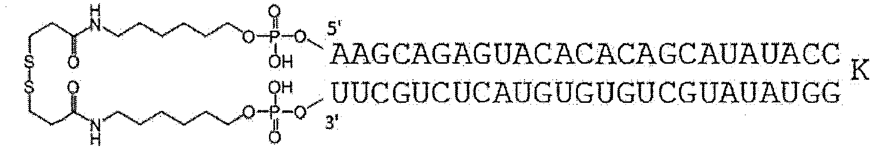

608 µmοl/L KA-0001 (132 µL, 80 nmol), distilled water for injection (139 µL), phosphate buffer (pH 8.5, 80 µL, final concentration 200 mmol/L) and 1 mg/mL DSP-ONSu/DMF solution (49 µL) were mixed and stirred at 25°C for 1 hr. The reaction mixture was purified by HPLC (column: Develosil C8-UG-5, 5 µm, 10×50 mm; flow rate: 4.0 mL/min; detection: UV260 nm; column oven: 40°C; Buffer A: 50 mmol/L TEAA (pH 7.0), 5% CH₃CN; Buffer B: 50 mmol/L TEAA (pH 7.0), 50% CH₃CN) and the peak of the desired product was collected. The collected fraction was subjected to ethanol precipitation and the resulting precipitate was dissolved in distilled water for injection. Absorbance at UV260 nm was measured and the yield was calculated. KA-0001-DSP (0.24 mg) with purity 94.90% was obtained. Mass spectrometry: 16914.00 (Calculated: 16913.55). The HPLC chart after purification is shown in Fig. 31.

### Example 32: Synthesis of PA-0031-DSP

The structure of the cyclic nucleic acid molecule of the present invention, PA-0031-DSP, is shown below.

PA-0031-DSP is a cyclic nucleic acid molecule in which DSP-ONSu containing a disulfide bond is bonded to the amino group at the 5'-terminal and 3'-terminal of the aforementioned PA-0031 by the below-mentioned method.

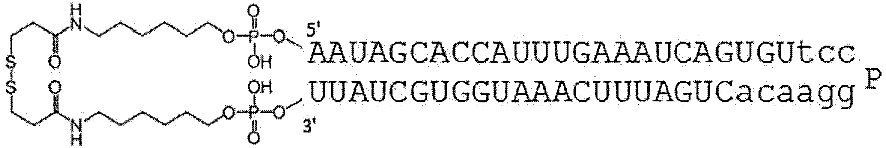

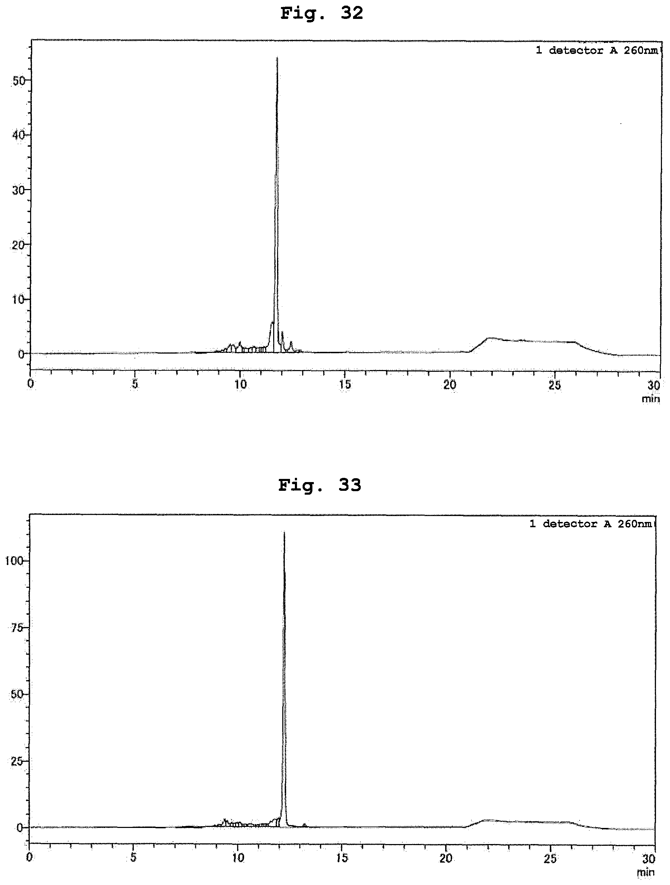

812 µmol/L PA-0031 (369 µL) as an unpurified product (300 nmol as unpurified product), distilled water for injection (50 µL), phosphate buffer (pH 8.5, 120 µL, final concentration 200 mmol/L) and 10 mg/mL DSP-ONSu/DMF solution (61 µL) were mixed and stirred at 25°C for 1 hr. The reaction mixture was subjected to ethanol precipitation and purified by anion exchange chromatography (column: DNAPacPA100, 9x250 mm; flow rate: 2.5 mL/min; detection: UV260 nm; Buffer A: 25 mmol/L Tris-HCl (pH 8.0), 10% CH₃CN; Buffer B: 25 mmol/L Tris-HCl (pH 8.0), 10% CH₃CN, 700 mmol/L NaClO₄) and the peak of the desired product was collected. The collected fraction was subjected to ethanol precipitation and the resulting precipitate was dissolved in distilled water for injection. Absorbance at UV260 nm was measured and the yield was calculated. PA-0031-DSP (0.41 mg) with purity 60.06% was obtained. Mass spectrometry: 18008.80 (Calculated: 18008.27). The HPLC chart after purification is shown in Fig. 32.

### Example 33: Synthesis of PA-0032-DSP

The structure of the cyclic nucleic acid molecule of the present invention, PA-0032-DSP, is shown below.

PA-0032-DSP is a cyclic nucleic acid molecule in which DSP-ONSu containing a disulfide bond is bonded to the amino group at the 5'-terminal and 3'-terminal of the aforementioned PA-0032 by the below-mentioned method.

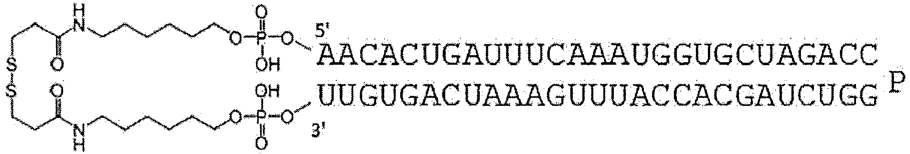

PA-0032 (300 nmol) (as unpurified product) was subjected to a reaction and purification similar to those for PA-0031. Absorbance at UV260 nm was measured and the yield was calculated. PA-0032-DSP (0.43 mg) with purity 69.74% was obtained. Mass spectrometry: 18153.80 (Calculated: 18153.24). The HPLC chart after purification is shown in Fig. 33.

### Example 34: Synthesis of PA-0033-DSP

The structure of the cyclic nucleic acid molecule of the present invention, PA-0033-DSP, is shown below.

PA-0033-DSP is a cyclic nucleic acid molecule in which DSP-ONSu containing a disulfide bond is bonded to the amino group at the 5'-terminal and 3'-terminal of the aforementioned PA-0033 by the below-mentioned method.

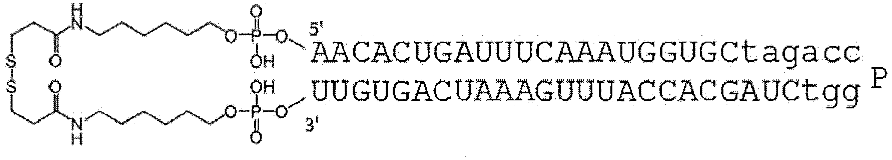

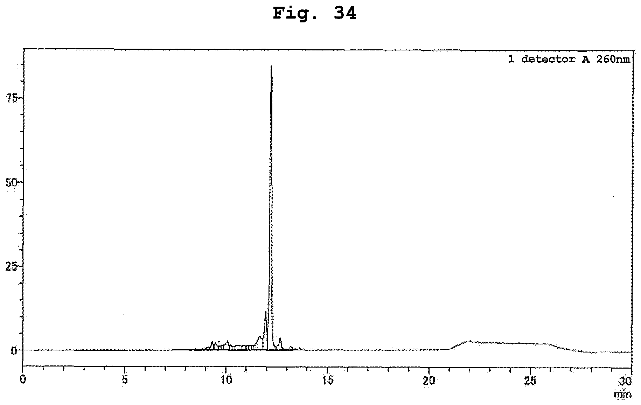

PA-0033 (300 nmol) (as unpurified product) was subjected to a reaction and purification similar to those for PA-0031. Absorbance at UV260 nm was measured and the yield was calculated. PA-0033-DSP (0.48 mg) with purity 57.27% was obtained. Mass spectrometry: 18037.80 (Calculated: 18037.31). The HPLC chart after purification is shown in Fig. 34.

### Experimental Example 1 (Experimental method for evaluation of stability in serum)

Each of the aforementioned nucleic acid molecules was diluted with distilled water for injection (Otsuka Pharmaceutical Co., Ltd.), HUMAN SERUM STERILE (MP Biomedicals) was added and the mixture was reacted for a given time. To be specific, the final concentration of the aforementioned nucleic acid molecule in the reaction mixture in a 1.5 mL micro tube was 2.5 µmol/L and the final concentration of HUMAN SERUM STERILE was 50%.

The solution after the reaction was treated with TE saturated phenol (Nacalai Tesque) to remove proteins. The aqueous layer was recovered and ethanol precipitation was performed to collect serum-treated nucleic acid molecules. The aforementioned serum-treated nucleic acid molecules were dissolved in distilled water for injection and 5 pmol per lane was electrophoresed on a 15% polyacrylamide gel (Bio Craft).

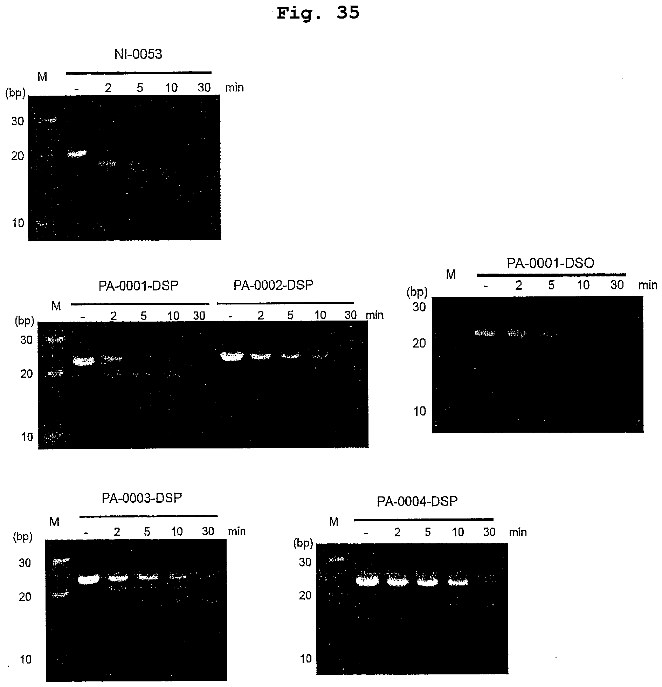

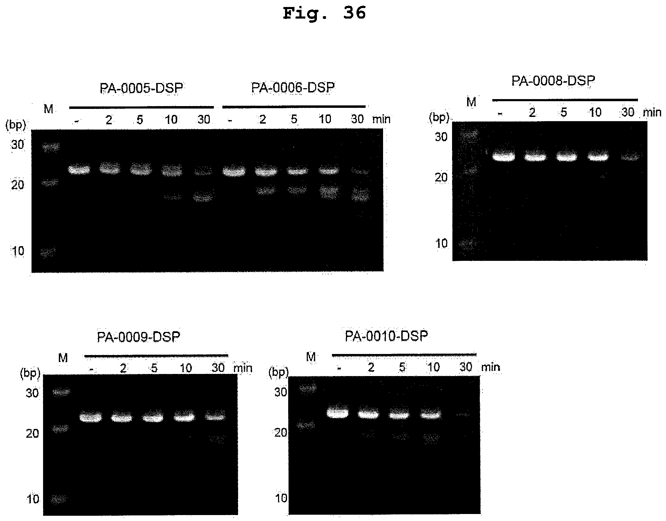

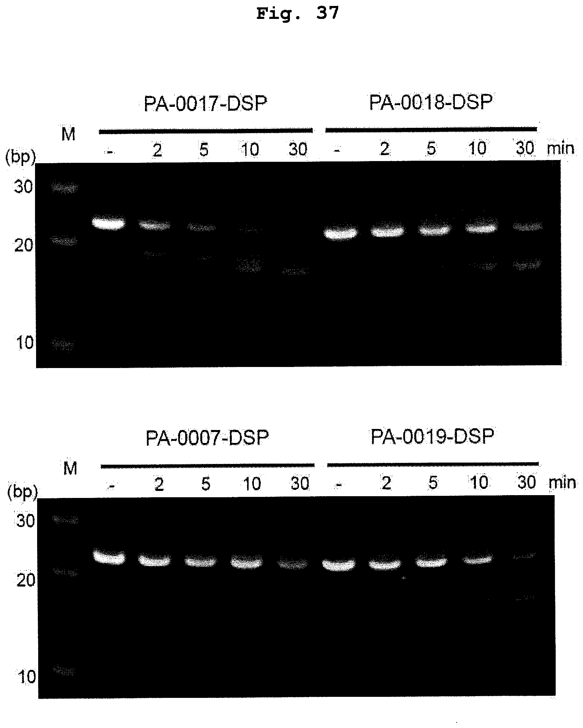

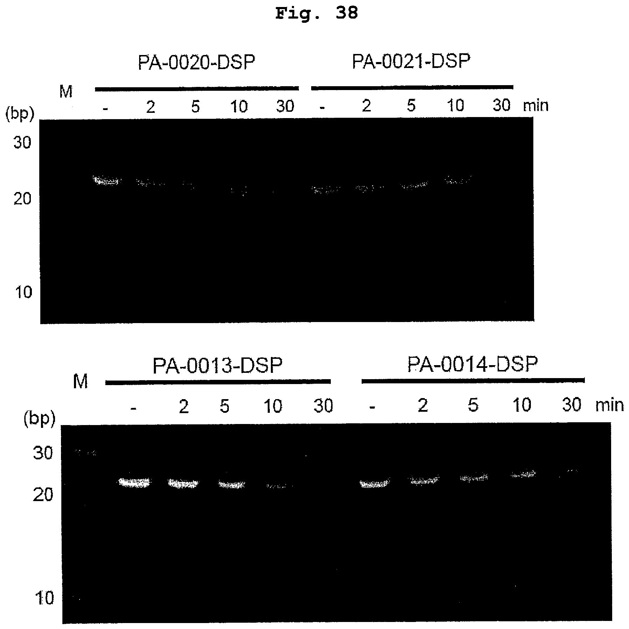

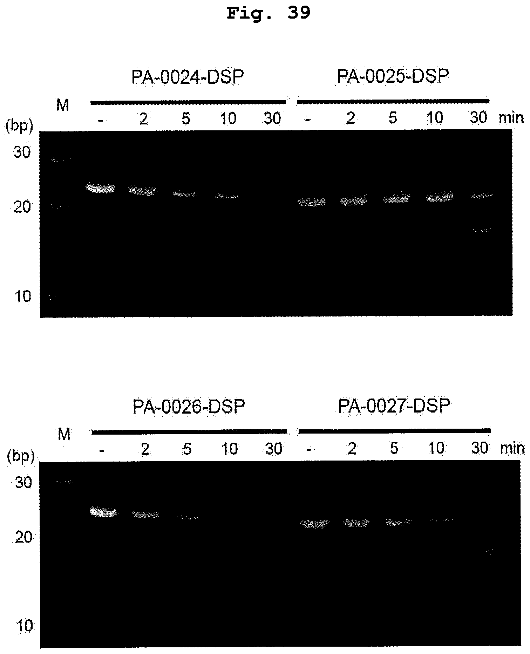

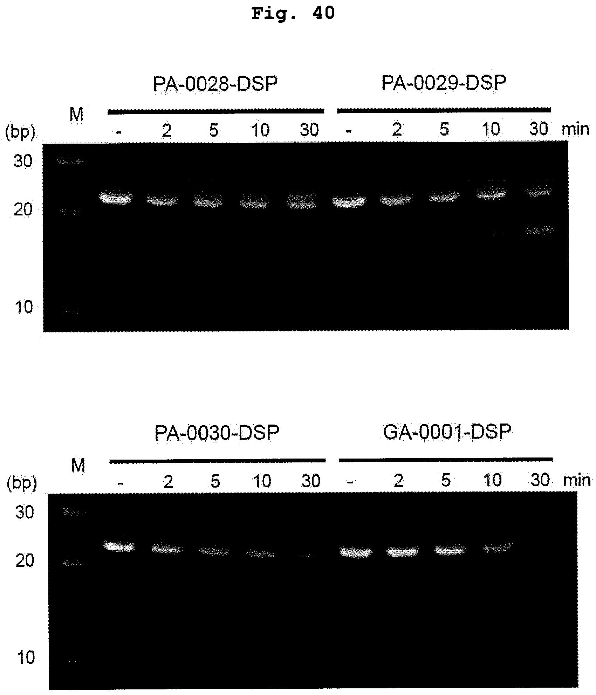

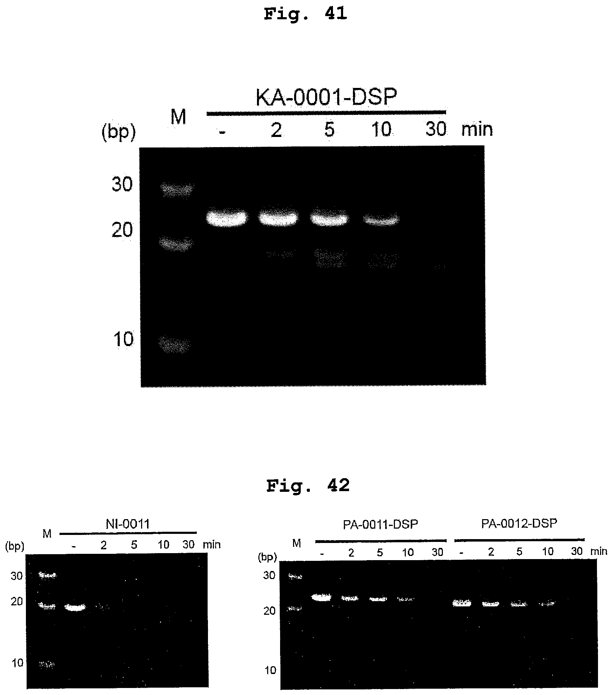

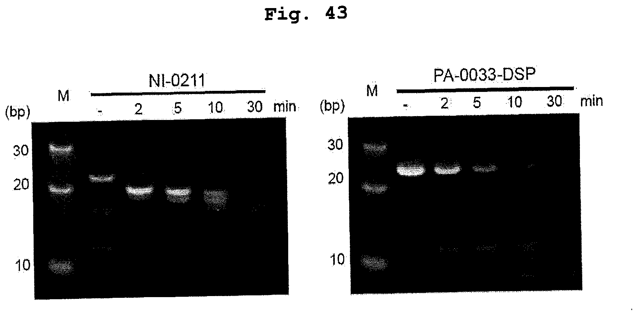

In Reference Example 1, the band before the treatment disappeared within 2 minutes. Using this as the standard, the stability of each nucleic acid molecule carrying a TGF-β1 expression inhibitory sequence in the serum was evaluated. The results are shown in Fig. 35 - Fig. 41. In Reference Example 2, the band before the treatment disappeared within 30 minutes. Using this as the standard, the stability of each nucleic acid molecule carrying a GAPDH expression inhibitory sequence in the serum was evaluated. The results are shown in Fig. 42. In Reference Example 3, the band before the treatment disappeared within 2 minutes. Using this as the standard, the stability of each nucleic acid molecule carrying a miR-29b guide strand sequence in the serum was evaluated. The results are shown in Fig. 43. As shown in each Figure, it was found that the nucleic acid molecule of the present invention shows high stability in the serum.

### Experimental Example 2 (gene expression inhibitory activity measurement method)

A549 cells (DS Pharma Biomedical Co., Ltd.) were used as the cells. A 10% FBS-containing DMEM (Invitrogen) was used as the medium. The culture conditions were set to 37°C and 5% CO₂.

First, the cells were cultured in the medium, and the cell suspension was dispensed to a 24-well plate so that each well contained 400 µL at 4 × 10⁴ cells/well. The cells were transfected with the aforementioned nucleic acid molecule by using a transfection reagent RNAiMAX (Invitrogen) according to the attached protocol. Specifically, the transfection was carried out by setting the composition per well as follows. In the following composition, (B) is Opti-MEM (Invitrogen) and (C) is a nucleic acid molecule solution, and they were added in a total of 98.5 µL. The final concentration of the aforementioned nucleic acid molecule in the aforementioned well was set to 1 nmol/L or 1, 10 nmol/L.

**[Table 3]**

| (Composition per well: µL) | |
|---|---|
| culture medium | 400 |
| transfection reagent | 1.5 |
| (B) + (C) | 98.5 |
| Total | 500 |

After the transfection, the cells were cultured for 24 hours, and RNA was collected using NucleoSpin RNA Plus (Takara Bio Inc.) according to the attached protocol. Subsequently, cDNA was synthesized from the aforementioned RNA by using a reverse transcriptase (Transcriptor First Strand cDNA Synthesis Kit, Roche) and according to the attached protocol. Then, as shown below, PCR was carried out using the aforementioned synthesized cDNA as a template, and the expression level of the TGF-β1 gene, GAPDH gene and COL1A1 gene and the expression level of the internal standard β-actin gene were measured. The expression level of the aforementioned TGF-β1 gene, GAPDH gene and COL1A1 gene was normalized with reference to that of the β-actin gene.

The aforementioned PCR was carried out using LightCycler480 SYBR Green I Master (Roche) as a reagent and Light Cycler480 Instrument II (Roche) as an instrument. The TGF-β1 gene, COL1A1 gene, β-actin gene and GAPDH gene were respectively amplified using the following primer sets.
PCR primer set for TGF-β1 gene
   5'-ttgtgcggcagtggttgagccg-3' (SEQ ID NO: 45)
   5'-gaagcaggaaaggccggttcatgc-3' (SEQ ID NO: 46)
PCR primer set for COL1A1 gene
   5'- cccaaggacaagaggcatgt-3' (SEQ ID NO: 47)
   5'- ccgccatactcgaactggaa-3' (SEQ ID NO: 48)
PCR primer set for β-actin gene
   5'-gccacggctgcttccagctcctc-3' (SEQ ID NO: 49)
   5'-aggtctttgcggatgtccacgtcac-3' (SEQ ID NO: 50)
PCR primer set for GAPDH gene
   5'- atggggaaggtgaaggtcg-3' (SEQ ID NO: 51)
   5'- gggtcattgatggcaacaatatc-3' (SEQ ID NO: 52)

As a control, regarding the cells subjected to the same transfection procedures as in the above except that the aforementioned RNA solution was not added and that the aforementioned (B) and 1.5 µL of the aforementioned transfection reagent were added so that the total amount would be 100 µL, the expression level of the gene also was measured (mock) .

As for the normalized expression levels of TGF-β1 gene, COL1A1 gene and GAPDH gene, the relative value in the cell introduced with each nucleic acid molecule was determined based on the expression level in the cells of the control (mock) set as 1.

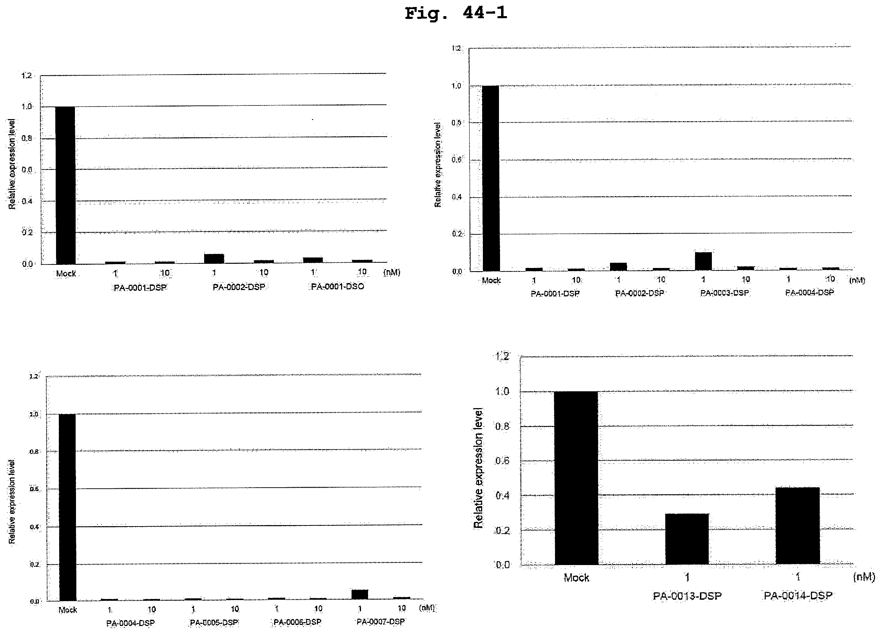

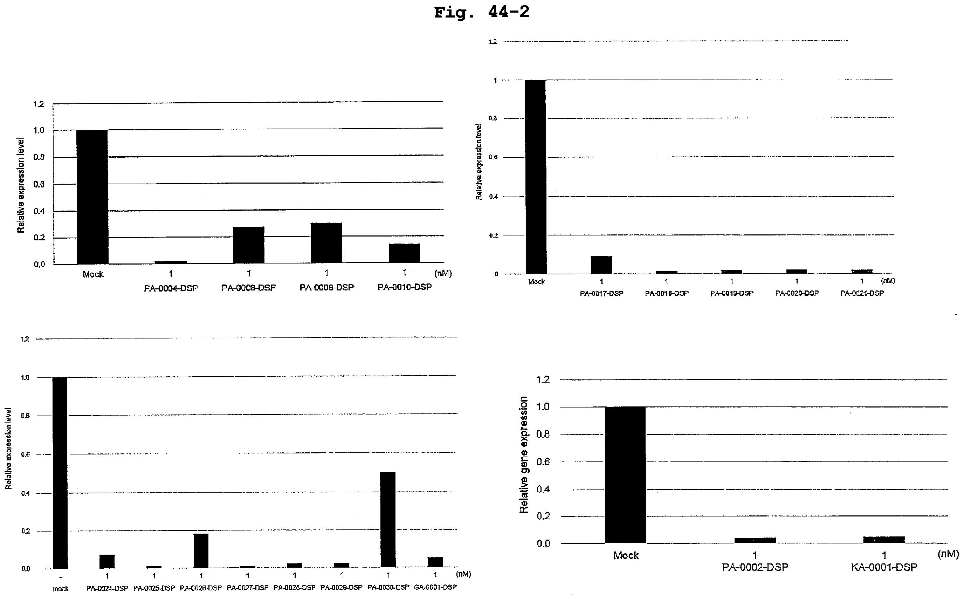

These results are shown in Figs. 44 (TGF-β1 gene), 45 (COL1A1 gene) and 46 (GAPDH gene). The vertical axis indicates the relative gene expression level. As shown in each Figure, it was found that the nucleic acid molecule of the present invention shows a strong gene expression inhibitory activity.

### Experimental Example 3 (stability in mouse living body (in blood))

### (Experimental method)

Saline was added to each test substance, and the mixture was stirred with a vortex mixer and rehydrated to prepare a 1 mg/mL nucleic acid solution. After rehydration, the mixture was filtered using a 0.2 µm filter to give an administration solution. It was administered at a dose of 5 mg nucleic acid/5 mL/kg into the tail vein of male mice (C57BL/6Jcl, 8-week-old, manufactured by CLEA Japan). The mice were divided into a group for blood collection at 0 hr after administration, a group for blood collection after 0.5 hr, a group for blood collection after 1 hr, a group for blood collection after 3 hr, and a group for blood collection after 6 hr, and 3 mice per group were used. At 0, 0.5, 1, 3, and 6 hr after administration, the mice were bled to death by blood collection using an EDTA-treated syringe from the heart under isoflurane inhalation anesthesia. After adding 3 volumes of Trizol LS Reagent (manufactured by Ambion) to 1 volume of blood, the mixture was stirred by a vortex mixer and total RNA was extracted according to the reagent instructions.

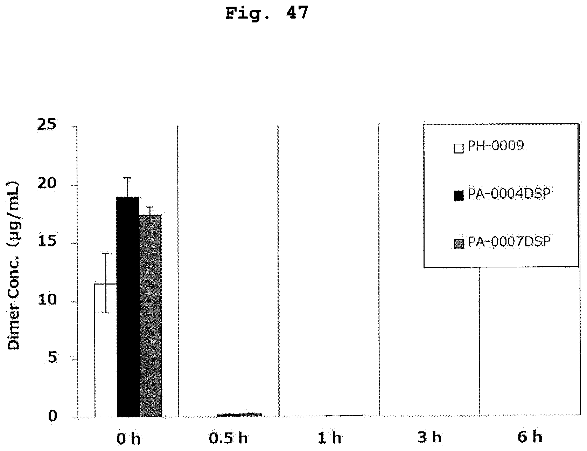

Nuclease P1 (manufactured by Sigma Aldrich) was added to the RNA solution and heated at 60°C for 2 hr. Thereafter, a prescribed amount of 10 × Alkaline Phosphatase Buffer was added and stirred, then Alkaline Phosphatase (manufactured by Promega) was added, and the mixture was heated at 56°C for 2 hr. After allowing to cool, the amount of the Dimer produced was quantified by a high-speed liquid chromatograph-mass spectrometer
The results are shown in Fig. 47. PH-0009 was not detected at all in 0.5 hr but PA-0004-DSP and PA-0007-DSP of the present invention were detected in 0.5 hr. That is, it was shown that the nucleic acid molecule of the present invention is more stable in blood than known single-stranded nucleic acid molecules.

It was found that PA-0004-DSP, PA-0018-DSP and PA-0025-DSP are particularly superior in terms of both stability and gene expression inhibitory effect.

From the above results, it was shown that cyclization of a single-stranded nucleic acid molecule having a gene expression regulation function (preferably 5'-terminal and 3'-terminal are blunted) by a disulfide bond drastically improves stability in human serum compared to double stranded RNA (siRNA), without impairing the expression inhibitory effect in vitro.

While the present invention has been described above with reference to illustrative embodiments, the present invention is by no means limited thereto. Various changes that may become apparent to those skilled in the art may be made in the configuration and specifics of the present invention without departing from the scope of the present invention.

The contents disclosed in any publication cited herein, including patents and patent applications, are hereby incorporated in their entireties by reference, to the extent that they have been disclosed herein.

### [Industrial Applicability]

Since the cyclic nucleic acid molecule of the present invention can be easily synthesized at a low cost, and can inhibit the translation of a protein encoded by the aforementioned gene. Therefore, the cyclic nucleic acid molecule of the present invention is useful as, for example, a pharmaceutical product, a diagnostic agent, an agricultural chemical, and a tool for conducting research on agriculture, medical science, life science, and the like.

This application is based on a patent application No. 2017-070695 filed in Japan (filing date: March 31, 2017), the contents of which are incorporated in full herein.

### [Sequence Listing free text]

SEQ ID NO: 1: expression inhibitory sequence
SEQ ID NO: 2: sequence complementary to expression inhibitory sequence
SEQ ID NO: 3: expression inhibitory sequence
SEQ ID NO: 4: sequence complementary to expression inhibitory sequence
SEQ ID NO: 5: single-stranded nucleic acid molecule_PA-0001
SEQ ID NO: 6: single-stranded nucleic acid molecule_PA-0002
SEQ ID NO: 7: single-stranded nucleic acid molecule_PA-0003
SEQ ID NO: 8: single-stranded nucleic acid molecule_PA-0004
SEQ ID NO: 9: single-stranded nucleic acid molecule_PA-0005
SEQ ID NO: 10: single-stranded nucleic acid molecule_PA-0006
SEQ ID NO: 11: single-stranded nucleic acid molecule_PA-0007
SEQ ID NO: 12: single-stranded nucleic acid molecule_PA-0008
SEQ ID NO: 13: single-stranded nucleic acid molecule_PA-0009
SEQ ID NO: 14: single-stranded nucleic acid molecule_PA-0010
SEQ ID NO: 15: single-stranded nucleic acid molecule_PA-0011
SEQ ID NO: 16: single-stranded nucleic acid molecule_PA-0012
SEQ ID NO: 17: single-stranded nucleic acid molecule_PA-0013
SEQ ID NO: 18: single-stranded nucleic acid molecule_PA-0014
SEQ ID NO: 19: single-stranded nucleic acid molecule_PA-0015
SEQ ID NO: 20: single-stranded nucleic acid molecule_PA-0016
SEQ ID NO: 21: single-stranded nucleic acid molecule_PA-0017
SEQ ID NO: 22: single-stranded nucleic acid molecule_PA-0018
SEQ ID NO: 23: single-stranded nucleic acid molecule_PA-0019
SEQ ID NO: 24: single-stranded nucleic acid molecule_PA-0020
SEQ ID NO: 25: single-stranded nucleic acid molecule_PA-0021
SEQ ID NO: 26: single-stranded nucleic acid molecule_PA-0024
SEQ ID NO: 27: single-stranded nucleic acid molecule_PA-0025
SEQ ID NO: 28: single-stranded nucleic acid molecule_PA-0026
SEQ ID NO: 29: single-stranded nucleic acid molecule_PA-0027
SEQ ID NO: 30: single-stranded nucleic acid molecule_PA-0028
SEQ ID NO: 31: single-stranded nucleic acid molecule_PA-0029
SEQ ID NO: 32: single-stranded nucleic acid molecule_PA-0030
SEQ ID NO: 33: single-stranded nucleic acid molecule_GA-0001
SEQ ID NO: 34: single-stranded nucleic acid molecule_KA-0001
SEQ ID NO: 35: single-stranded nucleic acid molecule_PA-0031
SEQ ID NO: 36: single-stranded nucleic acid molecule_PA-0032
SEQ ID NO: 37: single-stranded nucleic acid molecule_PA-0033
SEQ ID NO: 38: siRNA_NI-0053(sense strand)
SEQ ID NO: 39: siRNA_NI-0053(antisense strand)
SEQ ID NO: 40: siRNA_NI-0011 (sense strand)
SEQ ID NO: 41: siRNA_NI-0011 (antisense strand)
SEQ ID NO: 42: siRNA_NI-0211 (sense strand)
SEQ ID NO: 43: siRNA_NI-0211 (antisense strand)
SEQ ID NO: 44: single-stranded nucleic acid molecule _PH-0009
SEQ ID NO: 45: TGF-β1 primer
SEQ ID NO: 46: TGF-β1 primer
SEQ ID NO: 47: COL1A1 primer
SEQ ID NO: 48: COL1A1 primer
SEQ ID NO: 49: β actin primer
SEQ ID NO: 50: β actin primer
SEQ ID NO: 51: GAPDH primer
SEQ ID NO: 52: GAPDH primer

## Claims

1. A cyclic nucleic acid molecule represented by the following formula (A): 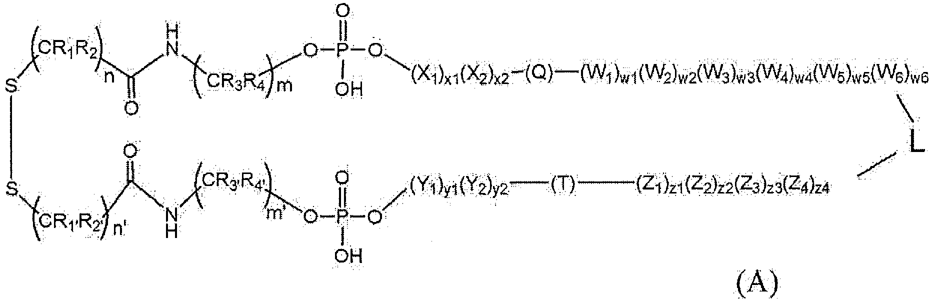 wherein
R₁, R₂, R_{1'} and R_{2'} are each independently a hydrogen atom, an alkyl group, an alkoxy group or hydroxy;
R₃, R₄, R_{3'} and R_{4'} are each independently a hydrogen atom, an alkyl group, an alkoxy group or hydroxy;
X₁ and X₂ are each independently an optionally modified ribonucleotide residue or deoxyribonucleotide residue;
Y₁ and Y₂ are each independently an optionally modified ribonucleotide residue or deoxyribonucleotide residue;
Z₁ - Z₄ are each independently an optionally modified ribonucleotide residue or deoxyribonucleotide residue;
W₁ - W₆ are each independently an optionally modified ribonucleotide residue or deoxyribonucleotide residue;
Q and T are each a guide strand or a passenger strand (when one is a guide strand, the other is a passenger strand);
L is a linker;
m and m' are each independently an integer of 1 - 12;
n and n' are each independently an integer of 1 - 10;
x1 and x2 are each independently 0 or 1;
y1 and y2 are each independently 0 or 1;
z1 - z4 are each independently 0, 1 or 2; and
w1 - w6 are each independently 0, 1 or 2.

2. The nucleic acid molecule according to claim 1 wherein X₁, X₂, Y₁, Y₂, Z₁ - Z₄ and W₁ - W₆ are each independently a ribonucleotide residue or deoxyribonucleotide residue optionally modified by a methyl group.

3. The nucleic acid molecule according to claim 1 or 2 wherein the linker is represented by any of the following formula:

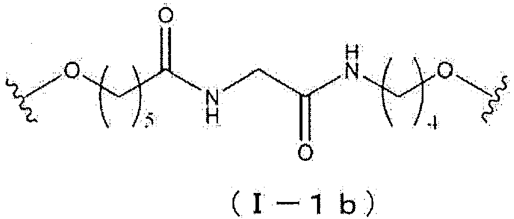

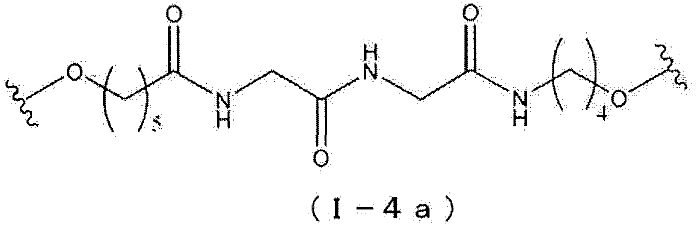

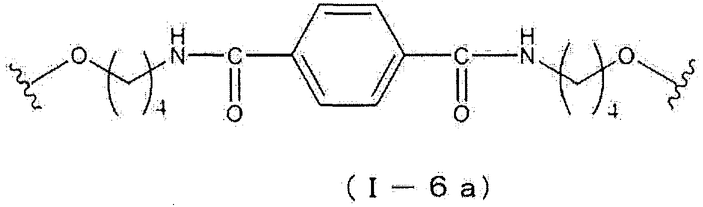

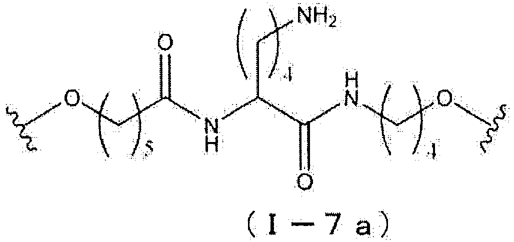

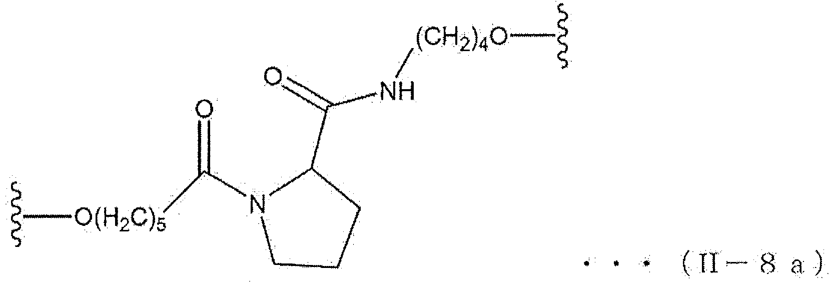

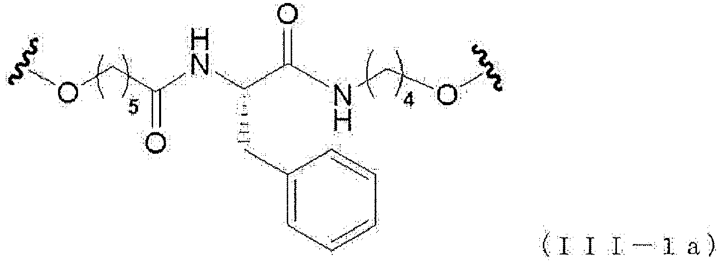

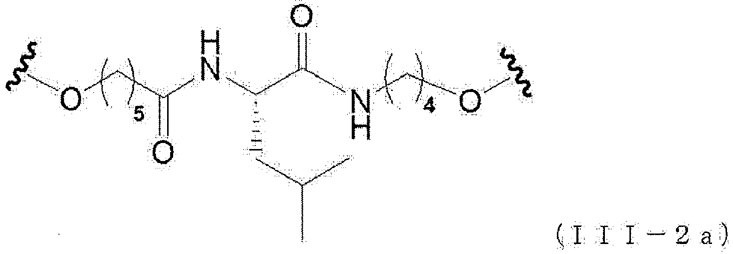

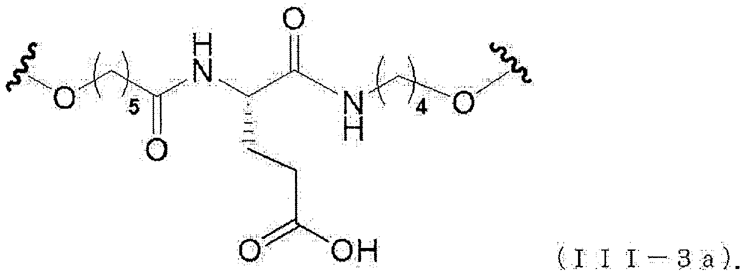

4. The nucleic acid molecule according to any one of claims 1 to 3 wherein at least one of X₁, X₂, Y₁, Y₂, Z₁ - Z₄ and W₁ - W₆ is independently a ribonucleotide residue or deoxyribonucleotide residue optionally modified by a methyl group.

5. The nucleic acid molecule according to any one of claims 1 to 4 wherein at least five of X₁, X₂, Y₁, Y₂, Z₁ - Z₄ and W₁ - W₆ are each independently a ribonucleotide residue or deoxyribonucleotide residue optionally modified by a methyl group.

6. The nucleic acid molecule according to any one of claims 1 to 5 comprising a polynucleotide strand comprising a guide strand, a passenger strand and a linker, and a double stranded structure formed in a part thereof, wherein both ends of the double stranded structure are blunt ends.

7. A composition for inhibiting expression of a target gene, comprising the nucleic acid molecule according to any one of claims 1 to 6.

8. A pharmaceutical composition comprising the nucleic acid molecule according to any one of claims 1 to 6.

9. A method for inhibiting expression of a target gene comprising using the nucleic acid molecule according to any one of claims 1 to 6.

10. The method for inhibiting expression according to claim 9 comprising a step of administering the nucleic acid molecule to a cell, tissue or organ.

11. The method for inhibiting expression according to claim 9 or 10 wherein the nucleic acid molecule is administered in vivo or in vitro.

12. The method for inhibiting expression according to claim 9 or 10 wherein the nucleic acid molecule is administered to a non-human animal.
